# EUROPEAN PATENT APPLICATION

(11) **EP 2 067 787 A1**
(43) Date of publication of application: **10.06.2009**
(21) Application number: 07122553.6
(22) Date of filing: 06.12.2007
(51) Int. Cl.: C07K 14/72

(54) **Method for controlling insect populations**

(71) Applicant: Boehringer Ingelheim International GmbH, 55216 Ingelheim am Rhein (DE)
(72) Inventor: Kim, Young-Joon, Dr., 55216 Ingelheim am Rhein (DE); Yapici, Nilay, Dr., 55216 Ingelheim am Rhein (DE); Dickson, Barry, Dr., 55216 Ingelheim am Rhein (DE); Ribeiro, Carlos, Dr., 55216 Ingelheim am Rhein (DE)
(74) Representative: Hammann, Heinz

(57) **Abstract**

A method for controlling insect pest populations by affecting the switch of female insects to post-mating behavior. The method is based on the identification of insect sex peptide receptors and assay methods employing the sex peptide receptor.

## Description

The present invention relates to methods for controlling the population of insects, in particular, for controlling their reproduction.

Insects have a massive impact upon agriculture and human health. Damage and destruction due to insects is responsible for a significant loss of agricultural crops and stored agricultural produce worldwide. Furthermore, a number of significant and devastating infectious diseases, like malaria, are transmitted to humans by blood-feeding insects such as mosquitoes, flies and ticks.

To date, the spread and activity of agricultural insect pests has mostly been controlled by the widespread application of potent, broad-spectrum chemical pesticides over agricultural fields, greenhouses, and storage warehouses. Pests that effect humans are usually combated by widespread spraying of insecticides in or near residential areas.

Since the use of conventional pesticides is associated with significant hazards to the environment and human health, increasingly severe restrictions and bans have been placed on pesticides and their use. Furthermore, since insects develop resistance to pesticides after prolonged use, it becomes necessary to apply increasing amounts of pesticide and/or to the develop new pesticides that are more potent and, consequently, may be even more hazardous to health and environment. Therefore, the need for safer means to combat insects has been recognized.

WO 2006/008652 describes a method for biological control of insects through the introduction of cytoplasmic incompatibility (CI) by infection with an endosymbiotic bacteria (endosymbionts) such as Wolbachia.

US 2005/053933 describes a strategy for insect control that is based on insect behavior and the molecular neurobiology of insect olfaction. This strategy is based on recognition of specific odors by insects in the environment, specifically, the "ubiquitous" odorant receptor gene Or83b and compounds that interact with Or83b.

Another strategy to control insect populations is the Sterile Insect Technique SIT (Krafsur, 1998), which involves the generation. rearing and mass release of sterile insects into the environment. The released insects are normally male. The sterile males compete with the wild males for female insects. If a female mates with a sterile male then it will have no offspring, thus the next generation's population is reduced. Repeated release of insects can eventually wipe out a population. SIT has successfully been used to eradicate the Screwworm fly (Cochliomyia hominivorax) in areas of North America. There has also been success in controlling species of fruit flies, most particularly the Medfly (Ceratitis capitata), and the painted apple moth. For combating insects that spread pathogens like malaria (which are therefore also called "disease vectors"), similar strategies have been used as for agricultural pest control.

However, SIT has its limitations. Since the male insects are mostly sterilized with radiation, the newly sterilized males may be weakened and therefore not fit enough to compete with wildtype males. Also, the release of female mosquitoes, which is undesirable, can hardly be avoided. To overcome this problem, attempts have been made to rear and release only males, a process which is called "sexing". To this end, Catteruccia et al. 2005, suggest the use of transgenic sexing lines for the mosquito *Anopheles stephensi*, the principal vector of human malaria in Asia. Male mosquitoes, expressing enhanced green fluorescent protein (EGFP) under the control of the β2-tubulin promoter, can be identified by their fluorescent gonads and can be efficiently separated from females using both manual methods and high-throughput automated sorting machines.

In spite of the advances brought about by biotechnological approaches like the above-described ones, to date, the majority of the pest and disease vector management programs in Europe, the US and globally are still based on massive spraying of toxic insecticides.

Therefore, there still remains a need for methods to control insect populations, such as agricultural and other pests as well as disease vectors like mosquitoes, which can selectively destroy the target insects but which are environmentally more acceptable than the chemical methods involving mass spraying of toxic pesticides.

It has been the object of the invention to provide an alternate, innovative approach for control of insect populations. The solution of the problem underlying the present invention targets female insects and is based on their reproductive behavior.

At various stages in their lifespan, animals can undergo dramatic switches in their (potential) patterns of innate behavior. A striking example of such a behavioral switch occurs in the adult females of many species as a result of mating. For example, in most insect species, virgin females are receptive to courting males and retain their eggs; whereas those that have mated are unreceptive and lay eggs. These changes in female behavior and physiology are induced by factors produced in the male and transferred along with sperm during mating (Gillott, 2003). In *Drosophila,* the primary trigger of this behavioral switch is the sex peptide (SP), a 36 amino acid peptide produced in the male accessory gland (Chen, et al., 1988; Chapman, et al., 2003). How SP exerts its effects on female behavior and physiology is unknown.

To solve the problem underlying the invention, the inventors took the first essential step in unraveling the effects of SP on female insect behavior by identifying and localizing the SP receptor(s) in the female insect.

The present invention is based on the identification of the receptor for the *Drosophila melanogaster* sex peptide (SP), the primary trigger of the post-mating response in this species (Chen, et al., 1988; Chapman, et al., 2003). It has been found in the experiments of the invention that the sex peptide receptor (SPR) is a G protein coupled receptor (GPCR) that is encoded by the gene *CG16752* and specifically activated by low nanomolar concentrations of SP. The SPR has been found to be expressed in the female's reproductive tract, and in the brain and ventral nerve cord of both sexes. Females that lack *SPR* function, either entirely or only in the nervous system, fail to respond to SP. Such females continue to show virgin behaviors even after mating. The inventors have identified SPR orthologues in many insect species, among them several that are agricultural pests or disease vectors. The fact that SPR is highly conserved structurally and functionally across the insect order, the present invention provides a widely applicable novel strategy to control the reproductive and host-seeking behaviors of important agricultural pests and human disease vectors.

In a first aspect, the present invention relates to a method for controlling the population of an insect species of interest whose females naturally undergo a switch to post-mating behavior in response to a sex peptide, which is an analogue of the *Drosophila* sex peptide (SP) with the sequence shown in SEQ ID NO:18, and which is produced in the male and transferred to the female during mating, said method comprising applying to females of the species of interest one or more compounds that modulate the activity of the receptor for said sex peptide, thereby affecting said switch to said post-mating behavior, wherein said receptor, in that species of interest, is the orthologue of the *Drosophila* sex peptide receptor (SPR) with the amino acid sequence shown in SEQ ID NO:2.
"Sex peptide receptor" (SPR) designates the receptor for the sex peptide. "Sex peptide" (SP) designates a peptide that is the naturally occurring ligand of the SPR and an analogue of the *Drosophila* SP (Acp70A; GenBank Accession No. AAR04619; SEQ ID NO:18; encoded by SEQ ID NO:17), or a functional equivalent, fragment or derivative thereof.

An "analogue of the *Drosophila* SP" is a peptide that has, in another insect species, the same or an essentially similar function as the *Drosophila* SP in *Drosophila.* A functional fragment or derivative may, for example, be a fragment of the SP or a substitution, addition or deletion mutant thereof, that has the ability to bind to and activate the SPR.

"Switch to post-mating behavior" (in the following also simply referred to as "switch" or "switching") means the switch from the behavior of virgin females that are receptive to courting males and retain their eggs, to the behavior of females that, as a consequence of having mated, are unreceptive to courting and do lay eggs.

"Affecting the switch to post-mating behavior" means either a) preventing mated females from switching to post-mating behavior or b) inducing a post-mating behavior in unmated females or c) delaying mated females from switching to post-mating behavior.

Affecting said switch in female insects reduces a population of insects by generating females that do not produce progeny.

Since females of blood sucking insect species, as part of their post-mating behavior, take a blood meal only after egg laying to foster the development of the laid eggs, preventing disease vectors like mosquitoes from switching also prevents them from blood-feeding and thus from transferring the pathogen to the host.

"Control" in the context of population, refers to the limitation, prevention or reduction of population growth, i.e., by at least about 10% per generation, preferably at least about 50%, 80%, or even up to and including 100% of the insect population. Advantageously, the population of pests is eliminated.

In a further aspect, the present invention provides isolated SPRs from insect species other than *Drosophila,* functional equivalents or fragments of such SPRs, as well as nucleic acid molecues encoding such insect SPRs or functional equivalents or fragments thereof. (In the following, if not otherwise stated, "*Drosophila*" refers to *Drosophila melanogaster.)*

Specifically, the present invention provides an isolated SPR that is an orthologue of the *Drosophila* SPR with the amino acid sequence of SEQ ID NO:2, wherein activation of said SPR orthologue, when naturally occurring, in response to the sex peptide results in a switch to post-mating behavior in said female insect, or a functional equivalent or a biologically active fragment or derivative of said SPR.

In a first aspect, the present invention relates to an isolated SPR of *Drosophila melanogaster* with an amino acid sequence shown in SEQ ID NO:2.

In particular, the present invention provides a representative number of orthologs of the *Drosophila melanogaster* SPR as set forth hereinafter.

In one further aspect, said SPR orthologue is the SPR of *Aedes.*
In a further aspect, said SPR orthologue is the SPR of *Aedes aegypti* and has an amino acid as shown in SEQ ID NO:4.
In a further aspect, said SPR orthologue is the SPR of *Anopheles.*
In a further aspect, said SPR orthologue is the SPR of *Anopheles gambiae* and has an amino acid as shown in SEQ ID NO:6.
In a further aspect, said SPR orthologue is the SPR of *Bombyx mori* and has an amino acid as shown in SEQ ID NO:8.
In a further aspect, said SPR orthologue is the SPR of *Tribolium castaneum* and has an amino acid as shown in SEQ ID NO:10.
In a further aspect, said SPR orthologue is the SPR of *Pediculus humanus corporis* that contains the partial amino acid sequence shown in SEQ ID NO:12.
In a further aspect, said SPR orthologue is the SPR of *Culex pipiens quinquefasciatus* that contains the partial amino acid sequence shown in SEQ ID NO:14.
In a further aspect, said SPR orthologue is the SPR of *Drosophila pseudoobscura* and has amino acid sequence shown in SEQ ID NO:16.

It is well within the skills of one in the art to isolate additional orthologs of the *Drosophila* SPR based on the teachings described herein.

"Functional equivalent" refers to an SPR polypeptide that is capable of exhibiting a substantially similar biological activity as a naturally occurring SPR including, but not limited to, SPRs with one with the above-recited SPR sequences. Such activity need not necessarily be the same or substantially similar to the SPR's *in vivo* activity; such activity may also be an activity that the SPR exhibits under given non-natural conditions, e.g. in an assay, where, by way of example, a functionally equivalent SPR molecule may replace the naturally occurring SPR as long as the equivalent exhibits the function required in that assay, e.g. binding the SP and triggering a certain signal transduction pathway. Functionally equivalent SPRs also include naturally occurring SPRs in a different species, i.e. the orthologues. A biologically active fragment is a fragment that retains at least one of the biological activities of an SPR molecule described above.

SPR derivatives of the invention include, but are not limited to SPR variants containing, as a primary amino acid sequence, all or part of the amino acid sequence of an SPR protein including altered sequences in which functionally equivalent amino acid residues are substituted for residues within the sequence resulting in a silent change. For example, one or more amino acid residues within the sequence can be substituted by another amino acid of a similar polarity which acts as a functional equivalent, resulting in a silent alteration.
Substitutions for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Such substitutions are generally understood to be conservative substitutions.

In another aspect, the present invention relates to an isolated nucleic acid molecule which encodes the *Drosophila* SPR, wherein said nucleic acid molecule has a sequence shown in SEQ ID NO:1.

In a further aspect, the present invention relates to an isolated nucleic acid molecule which encodes an insect SPR that is an orthologue of the *Drosophilia* SPR, wherein said nucleic acid molecule
a) has a sequence selected from SEQ ID NO:3, 5, 7 or 9 or a partial sequence shown in SEQ ID NO:11 or 13;
b) encodes an SPR with an amino acid sequence selected from any of SEQ ID NO:4, 6, 8 or 10 or an SPR comprising a partial amino acid sequence shown in SEQ ID NO:12 or 14;
c) hybridizes to the complement of a nucleic acid molecule defined in a) or b) under stringent conditions;
d) hybridizes to the complement of a nucleic acid molecule defined in a) or b) under highly stringent conditions;
e) is any nucleic acid molecule that encodes an SPR polypeptide that
   (i) comprises a protein sequence or a partial protein sequence that is at least 65% identical overall to a sequence selected from any of SEQ ID NO:2, 4, 6, 8, 10, 12, 14, 16;
   (ii) is predicted, based on its amino acid sequence, to be a GPCR and has an amino acid sequence that is more closely related to *Drosophila* SPR of SEQ ID NO:2 than any other GPCR encoded in the *Drosophila* genome;
f) is the complement of any of the SPR sequences defined in a) to e).

Stringent conditions are well known in the art, e.g. hybridization to filter-bound DNA in 6x sodium chloride/sodium citrate (SSC) at about 45° C, followed by one or more washes in 0.2xSSC/0.1% SDS at about 50-65° C, or hybridization to filter-bound DNA in 0.5 M sodium pyrophosphate/7% SDS at about 6° C, followed by one or more washes in 0.2xSSC/1% SDS at about 42-55° C.

Highly stringent conditions are also well known in the art, e.g. hybridization to filter-bound nucleic acid in 6xSSC at about 45°C, followed by one or more washes in 0.1xSSC/0.2% SDS at about 68° C or hybridization to filter-bound DNA in 0.5 M sodium pyrophosphate/7% SDS at about 65°C, followed by one or more washes in 0.2xSSC/1% SDS at about 68° C.

"Isolated" when used to describe a nucleic acid molecule, means a nucleic acid molecule that is free from other nucleic acid(s) which are present in the natural source of the nucleic acid. Preferably, an "isolated" nucleic acid is free of nucleic acid(s) which naturally flank the nucleic acid in the genomic DNA of the insect from which the nucleic acid is derived. In the case of a recombinant nucleic acid molecule, such as a cDNA molecule, "isolated" also includes that the molecule is substantially free of cellular material, or culture medium. An isolated nucleic acid does not encompass a nucleic acid present in a library, such as a cDNA, genomic, or expression library.

"Isolated" when used herein to describe a polypeptide (or a biologically active portion thereof), refers to a protein that is substantially free of cellular material or proteins from the cell or tissue from which the polypeptide is derived.

The invention also relates to SPR nucleic acid molecules that encode portions of an SPR that correspond to one or more of its functional domains; nucleic acid molecules that encode one or more splice variants of an SPR and nucleic acid molecules that encode mutants of an SPR in which all or part of one of its domains is deleted or altered. The term "domain" refers to one or more extracellular domains (ECD), one or more transmembrane domains (TM), one or more cytoplasmic domains (CD) one or more intracellular domains (ID), and one or more SP-binding domains.

In one embodiment, SPR nucleic acids encoding dominant negative forms of SPRs can be tested as insect control agents in the form of recombinant viruses that direct the expression of a dominant negative SPR gene in the target pest. Suitable recombinant virus systems for expression of proteins in infected insect cells are known in the art, they include but are not limited to recombinant baculovirus, recombinant Semliki Forest virus, recombinant sindbis, recombinant pantropic retrovirus, and. Use of recombinant baculoviruses as a means to engineer expression of proteins in insects, and as insect control agents, is well known in the art. Alternatively, for testing the dominant negative activity of SPR genes, transgenic insects can be made as taught by Handler, 2001; or by Atkinson et al., 2001.

The SPR nucleic acid molecules of the present invention also include degenerate variants of the sequences described above.

The present invention also encompasses allelic variants of the SPR nucleic acid molecules of the present invention, as well as any and all nucleotide variations and/or amino acid polymorphisms or variations that are the result of natural allelic variation of the SPR sequences of he invention. Such allelic variants include, but are not limited to, ones that do not alter the functional activity of the SPRs of the invention. Variants also include, but are not limited to mutant alleles, which are allelic variants which do alter the functional activity of the SPR. A cDNA of a mutant SPR gene can be isolated, for example, by using PCR, or by screening a genomic or cDNA library prepared from a population of insects that have the mutant allele, as described below.

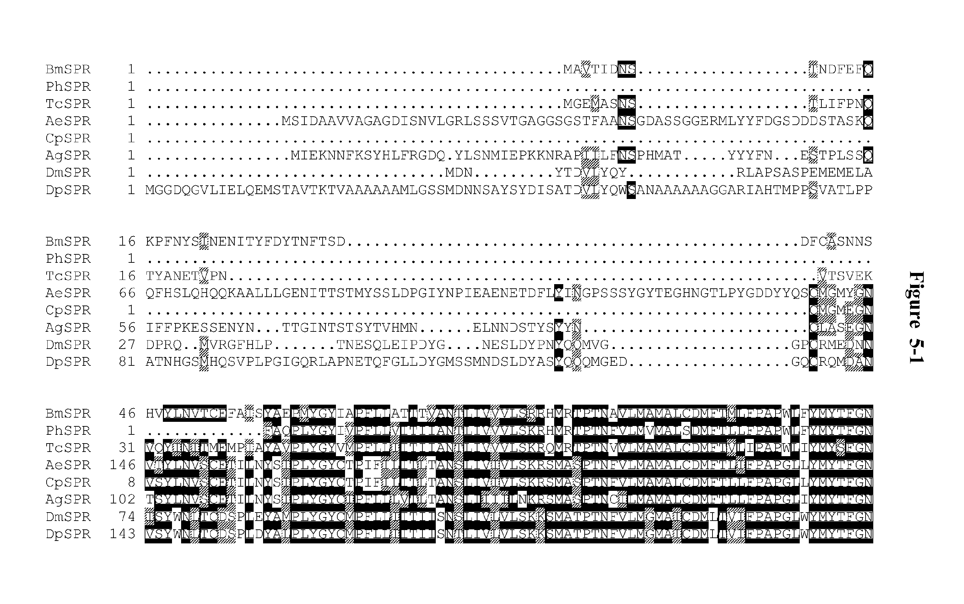

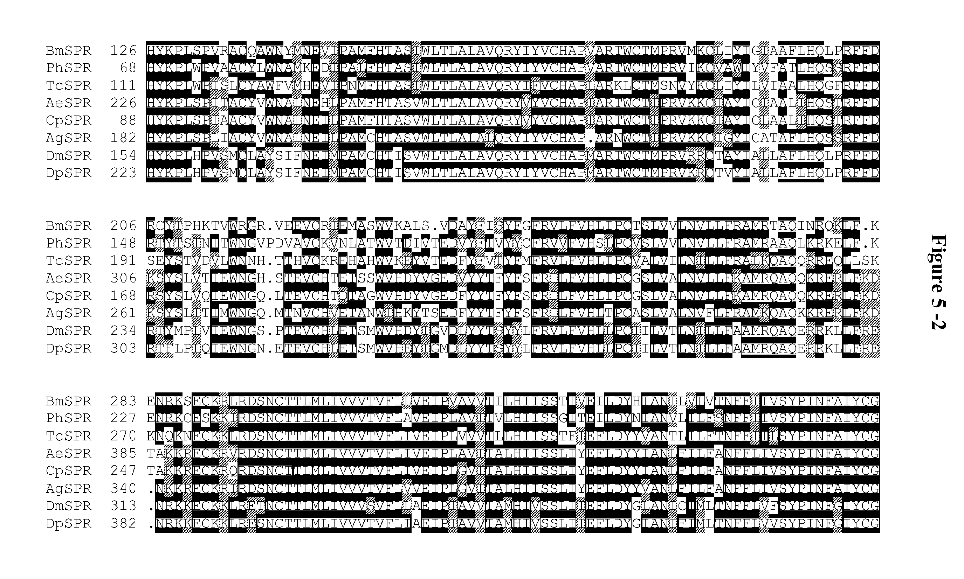

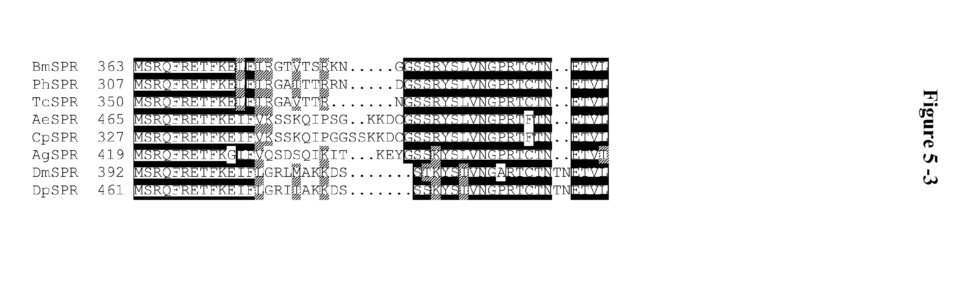

Fragments of SPR nucleic acids comprising regions conserved between (e.g., with homology to) other SPR nucleic acids of different insect species, are also provided. Sequence alignment of the amino acid sequences of SPRs and conserved regions are shown in Figure 5.

The SPR nucleic acid molecules of the invention still further include molecules that encode fusion proteins, such as fusion proteins containing all or a portion (preferably a biologically active one) of an SPR molecule, fused to another polypeptide, e.g. a natural or chimeric G protein alpha subunit as described below.

The present invention yet further provides vectors comprising a nucleic acid molecule encoding any of the foregoing SPR polypeptides. The present invention yet further provides a host cell transformed with a nucleic acid molecule encoding any of the foregoing polypeptides.

For expression, the nucleotide sequence coding for an SPR or a functional equivalent or fragment or other derivative thereof can be inserted into an appropriate expression vector, e.g., a vector which contains the elements required for transcription and translation of the inserted SPR-coding sequence. Thus, the nucleotide sequence is operatively linked to a promoter. A variety of host-vector systems may be utilized to express the SPR-coding sequence. These include but are not limited to mammalian cell systems infected with virus (e.g., vaccinia virus, adenovirus, etc.); insect cell systems infected with virus (e.g., baculovirus); microorganisms such as yeast containing yeast vectors, or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

For example, expression of an SPR protein may be controlled by any promoter/enhancer element known in the art. Promoters which may be used to control SPR expression include, but are not limited to, the SV40 early promoter, the promoter contained in the 3' long terminal repeat of Rous sarcoma virus, the herpes thymidine kinase promoter, the regulatory sequences of the metallothionein gene, the regulatory sequence of the human cytomegalovirus), prokaryotic expression vectors such as the β-lactamase promoter, or the lac promoter, plant expression vectors, promoter elements from yeast or other fungi. In a specific embodiment, a vector is used that comprises a promoter operably linked to an SPR gene nucleic acid, one or more origins of replication, and, optionally, one or more selectable markers (e.g., an antibiotic resistance gene).

The recombinant vectors comprising these the recombinant SPR vector can be introduced into host cells, e.g. bacterial cells or higher eukaryotic cells, e.g. mammalian cells, according to transfection methods well known in the art, including liposome-mediated transfection, polycation-mediated transfection, protoplast fusion, microinjections, calcium phosphate precipitation, electroporation or transfer by viral vectors.

Mammalian cell lines available as hosts for expression are well known in the art and include, inter alia, Chinese hamster ovary (CHO) cells, NSO, SP2/0 cells, HeLa cells, baby hamster kidney (BHK) cells, monkey kidney cells (COS), human carcinoma cells (e. g., Hep G2), A549 cells, 3T3 cells or the derivatives/progenies of any such cell line. Other mammalian cells, including but not limited to human, mice, rat, monkey and rodent cells lines, or other eukaryotic cells, including but not limited to yeast, insect and plant cells, or prokaryotic cells such as bacteria may be used. The SPRs of the invention are produced by culturing the host cells for a period of time sufficient to allow for expression of the antibody molecule in the host cells.

Once the SPR protein is expressed, it may be isolated and purified by standard methods including chromatography (e.g., ion exchange, affinity, and sizing column chromatography), centrifugation, differential solubility, or by any other standard technique for the purification of proteins.

In another aspect of the present invention, methods of identifying nucleic acids encoding SPR orthologues from further insect species are provided. This strategy has been successful in identifying several novel insect SPRs related to the SPR of *Drosophila melanogaster,* as shown in the Examples. These new SPR nucleic acid molecules, which encode highly conserved SPRs among various insect species, constitute useful targets for pest control agents.

In one embodiment, a method of identifying a nucleic acid molecule encoding an insect SPR polypeptide comprises PCR amplification of genomic DNA or cDNA from an insect species of interest with a forward primer comprising a degenerate oligonucleotide encoding at least six amino acids of a known SPR, e.g. taken from the *Drosophila* SPR encoding sequence shown in SEQ ID NO:1, or from any other of the SPR coding sequences identified in the present invention (SEQ ID NO:3, 5, 7, 9, 11, 13, 15), or its complement, and a reverse primer comprising a degenerate oligonucleotide encoding at least six amino acids of the known, e.g. *Drosophilia* SPR, taken from e.g. SEQ ID NOS:1 (or SEQ ID NO:3, 5, 7, 9, 11, 13, 15), or its complement; then detection of a PCR amplification product, thereby identifying a nucleic acid encoding the SPR polypeptide of the insect species of interest.

A "polymerase chain reaction" ("PCR") is a reaction well known in the art in which replicate copies are made of a target nucleic acid using one or more primers, and a catalyst of polymerization, such as a reverse transcriptase or a DNA polymerase, and particularly a thermally stable polymerase enzyme. Methods for PCR are taught in US 4,683,195 (Mullis) and US 4,683,202 (Mullis et al.).

The template for the reaction can be a cDNA obtained by reverse transcription of mRNA prepared from insect whole body or specific tissue, such as brains or reproductive organs, which are expected to express the SPR. Alternatively, if sequences from a given species are available in databases, primer design can be based on bioinformatics methods, as described in the Examples, e.g. by doing blast searches (e.g. TBLASTN; Altschul et al., 1997) on the relevant EST databases or genome assemblies and predicting gene structures (e.g. by Genscan).

The complete ORF of the *SPR* orthologue is amplified by RT-PCR. The PCR products are advantageously subcloned and sequenced to ensure that the amplified sequences are those encoding an SPR. The design of PCR primer pairs is well known in the art. Primers suitable in the present invention are generally capable of encoding at least five, more preferably six contiguous amino acids of the sequences found in highly conserved motifs of SPR. Thus, they are, at a minimum, preferably 15 to 18 nucleotides in length. The primer pair is chosen such that the reverse primer is downstream of a forward primer. Preferred oligonucleotides for amplification of a portion of an SPR gene or cDNA are pairs of degenerate oligonucleotide that serve as forward and reverse primers. Various programs for primer design are commercially available, for example, MacVector (Oxford Molecular Ltd.) and Vector NTI Suite (Informax, Inc.). Forward and reverse primers are preferably selected such that amplification of an SPR sequence results in a product of at least 150 nucleotides.

Suitable amplification conditions for amplification of an SPR nucleic acid from insect genomic DNA or cDNA include, but are not limited to, using 1 µg of cDNA or genomic DNA template and 80 pmol each primer in a 50 µl reaction, cycled between 94°C for 1 min, 51° C. for 1 min, 72° C for 1 min for a total of 40 cycles.

The PCR fragment can then be used to isolate a full length cDNA clone by a variety of well known methods, e.g. by screening a bacteriophage cDNA library. Alternatively, the labeled fragment can be used to screen a genomic library.

PCR technology can also be utilized to isolate full length SPR cDNA sequences. Following RNA isolation from the appropriate cellular or tissue source, e.g brain, reproductive organs , well known PCR amplification methods can be used to obtain the orthologue SPR cDNA, e.g. as described in US 2005/053933 or by Chenchik et al., 1996.

Alternately to using PCR technology, sequences of an orthologue SPR from an insect species of interest can be obtained by conventional methods, using labeled SPR nucleic acid from a known SPR encoding sequence, e.g. a probe from *Drosophila* SPR cDNA or genomic DNA for screening a cDNA library or a genomic DNA library of the insect species of interest.

A genomic DNA library is a clone library which contains representative nucleotide sequences (both transcribed DNA fragments as well as nontranscribed DNA fragments) from the genome of an insect of interest. In contrast, a "cDNA library", which can be established by various methods well known to the skilled person, contains only transcribed DNA sequences.

For screening DNA libraries, hybridization conditions should be of low to moderate stringency when the library has been derived from an insect that is evolutionarily distantly related to the insect from which the labeled SPR sequence has been derived. The suitable conditions may vary depending on the specific insects of interest; the skilled person will be able to choose the best conditions for a given situation; conditions are, for example, described in US 2005/053933 and in more detail in Sambrook et al., 1989; and Ausubel et al., 1989.

The above-described methods also allow to identify DNA sequence polymorphisms of an SPR gene, as described above, which may typically exist within a population of individual insects. Polymorphisms, including those that lead to changes in amino acid sequence, may be due to natural allelic variation. Natural allelic variations can typically result in 1-5% variance in the nucleotide sequence of a given gene. Certain allelic variations in the nucleotide sequence of a gene may be silent variations, i.e., do not encode a variant protein. Alternative alleles or allelic variants can be identified by sequencing the gene of interest in a number of different insects of the same species. This can be readily carried out by using PCR amplification of SPR gene products from genomic DNA from individual insects.

The normal SPR nucleic acid molecules or any suitable fragment thereof can then be labeled and used as a probed to identify the corresponding mutant allele in the library. The clone containing this mutant SPR gene can then be purified through methods routinely practiced in the art, and subjected to sequence analysis.

By the above methods, the SPR cDNA can be identified from an insect pest of interest, including disease vectors, whose population is to be controlle. Examples of insect species are selected, without limitation, from the following:
Hemiptera: planthoppers (Delphacidae) such as small brown planthopper (Laodelphax striatellus), brown rice planthopper (Nilaparvata lugens) and whitebacked rice planthopper (Sogatella furcifera); leafhoppers (Deltocephalidae) such as green rice leafhopper (Nephotettix cincticeps) and tea green leafhopper (Empoasca onukii); aphids (Aphididae) such as cotton aphid (Aphis gossypii), green peach aphid (Myzus persicae) and turnip aphid (Lipaphis pseudobrassicae); shield bugs (Pentatomidae); whiteflies (Aleyrodidae) such as greenhouse whitefly (Trialeurodes vaporariorum), sweetpotato whitefly (Bemisia tabaci) and silverleaf whitefly (Bemisia argentifolii); scales; lace bugs (Tingidae); psyllids (Psyllidae) and the like. Among the above, Diptera: Mosquitoes (Culicidae, vectors of filariasis or elephantiasis, West Nile Fever) such as Culex (e.g. common house mosquito (Culex pipiens pallens)), Aedes (the vector of yellow fever), Anopheles (the vectors of malaria) and Chironomus spp.; house flies (Muscidae) such as house fly (Musca domestica); blow flies (Calliphoridae); meat flies (Sarcophagidae); anthomyiid flies (Anthomyiidae); gall midges (Cecidomyiidae); leafminer flies (Agromyzidae); Fruit flies (Tephritidae); vinegar flies (Droophilidae); moth flies (Psychodidae); horse flies (Tabanidae); black flies (Simulidae) causing river blindness (Onchocerciasis); stable flies (Muscidae) and the like, sand flies (transmitting Leishmaniasis, sandfly fever), Tsetse fly (Glossina fuscipes fuscipes; the transmitter of Trypanosomiasis sleeping sickness).

Lepidoptera: pyralid moths (Pyralidae) such as rice stem borer (Chilo suppressalis), rice leaf roller (Cnaphalocrocis medinalis), European corn borer (Ostrinia nubilalis) and Parapediasia teterrella; owlet moths (Noctuidae) such as common cutworm (Spodoptera litura), beet armyworm (Spodoptera exigua), oriental armyworm (Pseudaletia separata), cabbage armyworm (Mamestra brassicae), black cutworm (Agrotis ipsilon), Trichoplusia spp., Heliothis spp., Helicoverpa spp. (e.g., corn earworm (Helicoverpa armigera)) Earias spp. and Autographa spp. (e.g., beet semi-looper (Autographa nigrisigna); whites (Pieridae) such as common cabbageworm (Pieris rapae crucivora); yponomeutids (Yponomeutidae) such as diamondback moth (Plutella xylostella); tussock moths (Lymantriidae) such as oriental tussockmoth (Euproctis taiwana), gypsy moth (Lymantria dispar), browntail moth (Euproctis similis); slug caterpillar moths (Limacodidae) such as Scopelodes contracus; tent caterpillar moths (Lasiocampidae) such as pine caterpillar (Dendrolimus spectabilis); leafroller moths (Tortricidae) such as summer fruit tortrix (Adoxophyesorana fasciata), oriental fruit moth (Grapholita molesta) and Codling moth (Cydia pomonella); Carposinidae such as peach fruit moth (Carposina niponensis); Lyonetiidae such as Apple leaf miner (Lyonetia clerkella); Gracillariidae such as apple leafminer (Phyllonorycter ringoniella) and horse chestnut leaf-miner (Cameraria ohridella); Phyllocnistidae such as citrus leafminer (Phyllocnistis citrella); Yponomeuta evonymella such as cabbage moth (Plutella xylostella); gelechiid moths (Gelechii) such as Pink Bollworm (Pectinophora gossypiella); tiger moths (Arctiidae); Tineidae and the like.

Coleoptera: leaf beetles (Chrysomelidae), chafers (Scarabaeidae), snout beetles (Curculionidae), leaf-rolling weevils (Attelabidae), Lady Beetles (Coccinelidae), Longhorn Beetles (Cerambycidae), Darkling Beetles (tenebrionidae like Tribolium castaneum) and the like.

Thysanoptera: thrips (Thripidae such as the genus Thrips (e.g., melon thrips (Thrips palmi)), the genus Frankliniella (e.g., Western Flower Thrips (Frankliniella occidentalis)) and the genus Sciltothrips (e.g., yellow tea thrips (Sciltothrips dorsalis)); Tube-tailed Thrips (Phlaeothripidae) and the like.

Blattaria: blattid cockroaches (Blattidae), blattellid cockroachs (blattelidiae), German cockroach (*Blattella germanica*), American cockroach (*Periplaneta americana*) and the like.

Orthoptera: grasshoppers (Acridiae), Desert locust (*Schistocerca gregaria*), mole crickets (Gryllotalpidae).

Human fleas (Pulex Irritans), and rodent fleas, the transmitters of Siphonaptera (Bubonic plague), cat fleas (Ctenocephalides felis) and the like.

Anoplura (sucking lice): body lice (Pediculus humanus, transmitting Rickettsiae causing typhus), and the like.

Isoptera: Japanese termite (Reticulitermes speratus), Formosan subterranean termite (Coptotermes formosanus) and the like.

Insects or insect families from the above list that are of special interest in view of population control due to their damage in agriculture or their role as disease vectors are Aedes, Anopheles, Culex, Nilaparvata lugens, Trialeurodes vaporariorum, various flies like Musca domestica and Glossina fuscipes fuscipes, Spodoptera litura, Agrotis ipsilon, Helicoverpa armigera, Plutella xylostella, Pectinophora gossypiella, Curculionidae, Thrips, Blattella germanica, Periplaneta Americana, Acridiae, Schistocerca gregaria, Pediculus humanus corporis, Reticulitermes speratus.

In a further aspect, the present invention provides a method for identifying a sex peptide (SP) from an insect species of interest, which is an analogue of the *Drosophila* SP (SEQ ID NO:18) and binds to the SPR from the species of interest.

By way of example, the SPR assay methods of the present invention, as described below, are useful for identifying new SPs that are not yet known to be capable of binding to an SPR polypeptide. Any of the assay methods described above may be used to identify new SP ligands based on their activation of SPR, as assessed for example using any of the read-outs described below.

The SPRs from insects of interest that have been identified with aforementioned methods can be utilized to screen peptide compounds isolated from tissues that are expected to produce its native ligand(s), such as male accessory glands or/and seminal fluid. Briefly, by way of example, the SPR is expressed in CHO cells together with Ca²⁺ sensing molecules such as aequorin, and a G-protein, e.g. a chimeric one. Cells expressing the transgenes are exposed to liquid chromatography fractions prepared from tissues of interest. Upon exposure to SP or SP like molecules, cells expressing SPR produce cellular responses, which are readily monitored by measuring luminescence. Compounds activating the SPR can then be further purified and characterized by biochemical methods such as PSD-MALDI, Q-TOF, or Edman-sequencing, if necessary, following enzymatic digestions.

The availability of SPs from various insect pest species of interest provides the basis for designing SP peptidomimetics, which are useful as SP agonists or antagonists to affect the switch to post-mating behavior in female insects.

The skilled person is familiar with methods for generating peptidomimetics and libraries containing collections of peptidomimetic compounds; e.g. the methods described in US 2002/016010, WO 00/18790, or WO 98/46631 for preparing and screening libraries of compounds useful to identify compounds that mimic the desired functional and/or structural aspects of a peptide of interest or portion thereof. Techniques for designing peptidomimetics that mimic SP function have also been described by Moore, 1994; Bursavich, et al., 2002; Gante, 1994; Golebiowski, et al., 2001; al-Obeidi, et al., 1998.

Peptidomimetics and peptidomimetic libraries can be assessed for their effect on the SPR of interest in one or more of the assay methods described below, e.g. a competition assay employing a labeled SP.

In a further aspect, the present invention provides molecules that bind to an SPR, in particular antibodies to an SPR, its derivatives and analogs. Such anti-SPR antibodies are in particular useful as research tools, e.g. to localize receptor expressing cells and thus e.g. further elucidate the biological function of SPR signaling. Anti-SPR antibodies are also useful to inhibit SPR activity.

An SPR as disclosed therein, its fragments or derivatives, or analogs thereof, may be used as an immunogen to generate antibodies which immunospecifically bind such SPR. Such antibodies include but are not limited to polyclonal, monoclonal, chimeric, single chain and Fab fragments. Various procedures known in the art may be used for the production of polyclonal or monoclonal antibodies to an SPR or derivative or analog. In a particular embodiment, rabbit polyclonal antibodies to an epitope of an SPR, can be obtained. Other antibodies can be obtained as e.g. described in US 2005/053933 for the insect Or83b odorant receptor.

The invention also relates to SPR antisense molecules. The present invention encompasses SPR antisense nucleic acid molecules, i.e. molecules that are complementary to a sense nucleic acid encoding an SPR, e.g. complementary to the coding strand of the double-stranded SPR cDNA molecule, or complementary to the SPR mRNA. The antisense nucleic acid can be complementary to an entire SPR coding strand, or, preferably, to only a portion thereof, e.g. all or part of the SPR coding region (or open reading frame).
Given the coding-strand sequence encoding the SPR protein, antisense nucleic acids of the invention can be designed according to the rules of Watson and Crick base pairing. For example, the antisense oligonucleotide can be complementary to the region surrounding the translation start site of the SPR mRNA. An antisense nucleic acid of the invention can be constructed using chemical synthesis and enzymatic ligation procedures known in the art.

Usually, an antisense oligonucleotide is chemically synthesized according to methods known in the art using naturally occurring nucleotides or nucleotides that have been modified to increase the biological stability of the molecules or to increase the physical stability of the duplex formed between the antisense and sense nucleic acids, e.g. phosphorothioate derivatives and acridine-substituted nucleotides. The antisense nucleic acid molecules of the invention are typically applied or generated in situ such that they hybridize with or bind to cellular mRNA and/or genomic DNA encoding the SPR protein to thereby inhibit expression of SPR, e.g. by inhibiting transcription and/or translation. With regard to SPR antisense molecules, their use as inhibitors of the function of SPR, in particular as active ingredients in pharmaceutical compositions for human therapy, the criteria described above for anti-SPR antibodies apply.

In a further aspect, the invention also encompasses short interfering RNA (siRNA) molecules that down regulate expression of an SPR gene by RNA interference ("SPR siRNA").

RNA interference refers to the process of sequence-specific post transcriptional gene silencing in animals mediated by short interfering RNAs (siRNA). siRNA molecules are short pieces of dsRNA obtained by processing of the dsRNA by Dicer, a ribonuclease III enzyme. Short interfering RNAs derived from Dicer activity are typically about 21-23 nucleotides in length and comprise about 19 base pair duplexes. Based on the RNA sequence of an SPR, SPR siRNA molecules with the ability to knock down SPR activity can be obtained by chemical synthesis.

In another embodiment, the present invention relates to transgenic plants comprising, integrated in their genome, an SPR inhibiting polynucleotide sequence. In one embodiment, said inhibiting polynucleotide encodes a oligoribonucleotide (SPR siRNA or SPR antisense oligonucleotide), wherein ingestion by females of the insect pest of interest of said plant that comprises said inhibiting oligonucleotide inhibits expression of the SPR.

The inhibiting polynucleotides may be introduced into a host plant cell by standard procedures known in the art for introducing recombinant sequences into a target plant host cell. Such procedures include, but are not limited to, transfection, infection, transformation and microorganism-mediated transformation, e.g. using Agrobacterium.

Methods based on transgenic plants for pest control according to the present invention and their generation have been described in WO 2007/074405.

In yet other aspect of the present invention, assay methods for identifying, assessing and optimizing molecules that bind to and/or modulate (i.e. agonize or antagonize) the activity of an insect SPR are provided. Since the SPR is a GPCR, the assay methods of the invention take advantage of the specific properties of the GPCRs, a family of receptors that transmit signals through the activation of heterotrimeric GTP-binding proteins (G proteins). The signals generated by the binding of the specific ligand to the GPCR are transmitted into the cell through the activation of heterotrimeric GTP-binding proteins (G proteins), protein complexes consisting of alpha, beta, and gamma subunits (Hepler and Gilman, 1992). Although one G protein can promiscuously bind to different GPCRs and transmit the generated signals, the interaction between the G protein and the GPCR is highly specific. The structural changes induced by the binding of an adequate ligand facilitate the exchange of GDP by GTP on the G alpha subunit of the heterotrimeric G protein. Because of these changes in the affinity for a nucleotide, the heterotrimeric G protein complex dissociates into the alpha and beta/gamma sub-complexes, which sequentially interact with their specific substrates in the cell. The G protein α subunits regulate the activity of several second messenger-generating systems; activated (GTP-bound) G-alpha proteins can regulate intracellular calcium, adenylyl cyclases, phospholipases, Rho-family members, protein kinase C, ion channels and phosphodiesterases.

In a further aspect, the present invention relates to a method for determining whether a test compound
a) binds to and activates an SPR polypeptide of interest (agonist), and/or
b) inhibits the generation of intracellular signals generated upon activation of said SPR in response to the SP, by
   i. binding to the receptor binding site of the sex peptide, or by
   ii. allosteric hindrance, or
c) inhibits the interaction of the SPR with the sex peptide, or
d) acts intracellularly on a signal in the signal transduction pathway that is triggered by activation of SPR in response to an SP; or
e) inhibits the interaction of the SPR and one or more G-protein(s),
wherein cells expressing on their surface an SPR, either endogenously or upon transfection with a nucleic acid molecule encoding an SPR, or a functional equivalent or a biologically active fragment or derivative of said SPR, or a membrane fraction of such cells, are incubated with said test compound and said test compound's ability to modulate SPR activity as defined in any of a) to e) is determined.

In addition to the SPR, the cells must express alpha, beta and gamma G protein subunits for formation of the trimeric G protein complex. Since most eukaryotic cells express G protein subunits and it is the identity of the alpha subunit that is critical for the specificity and fidelity of the signal transduction pathway that is triggered upon receptor activation, it is usually not necessary to consider the beta and gamma subunits with regard to these criteria. However, by transfecting the cell with a pre-selected alpha subunit, the signal transduction pathway may be directed, in view of a desired assay design, from its genuine route to the cascade that is favored by the alpha subunit of choice.

Thus, in certain embodiments, the cells are transfected with a DNA molecule encoding a G protein alpha subunit. In this embodiment, the cells (if they do not express the SPR of interest endogenously) may be co-transfected with the two separate DNA molecules (DNA encoding the SPR and DNA encoding the alpha subunit, present either on two plasmids or one plasmid carrying both DNA sequences). Alternatively, the cells may be transfected with a DNA molecule encoding a fusion protein comprising the SPR and the G protein alpha subunit ("SPR-G alpha fusion"). In the following, reference to the SPR polypeptide and the G alpha polypeptide in the context of transfection/expression also includes reference to the SPR-G alpha fusion polypeptide.

Any of the known alpha subunits may be used as long as it results in the signaling cascade of choice.

Suitable alpha subunits may be selected e.g. from the about 20 mammalian G protein alpha subunits that have been identified and that have been divided into four families based on their primary sequence similarity: Gs, Gi, Gq, and G12.

"Gi" proteins (alpha type 1) inhibit adenylate cyclase. Representatives of these G proteins are GNAI (GNAI1, GenBank Acc. No. NM_002069.5; GNAI2, GenBank Acc. No. NM_002070.2; GNAI3 GenBank Acc. No.NM_006496.1), GNAO (GNAO1, GenBank Acc. No. NM_020988.1), GNAT (GNAT1, GenBank Acc. No. NM_000172.2; GNAT2, GenBank Acc. No. NM_005272.3; GNAT3, GenBank Acc. No. NM_001102386.1), GNAZ (GenBank Acc. No. NM_002073.2).

"Gs" proteins (alpha type 2) activate adenylate cyclase. Representatives of these G proteins are GNAS (GenBank Acc. No. NM_000516.4) and GNAL (GenBank Acc. No. NM_002071.2).

"Gq" proteins (alpha type 3) stimulate phospholipase C. Representatives of these G proteins are GNAQ (GenBank Acc. No. NM_002072.2), GNA11 (GenBank Acc. No. NM_002067.2), GNA14 (GenBank Acc. No. NM_004297.2) and GNA15 (GenBank Acc. No. NM_002068.2).

In addition to the above naturally occurring alpha subunits, G protein chimeras have become available (e.g. Conklin, et al., 1993 and 1996; US 2004/0253675) that can be used to alter the signaling phenotype of receptors. By way of example, it has been shown that replacement of the five carboxyl-terminal amino acids of Gq alpha with the Gs alpha sequence permits a Gs alpha-coupled receptor (which normally activates adenylate cyclase) to stimulate phospholipase C. Such chimeras are also useful in screening assays that employ a GPCR whose naturally coupling G alpha subunit is not known.

The use of different G alpha subunits, expressed either independently of the SPR or in the context of an SPR-G-alpha fusion polypeptide, additionally allows to distinguish between agonists/antagonists acting directly on SPR polypeptide and/or on a downstream signal pathways that assay format is based on. The effect of agonists and/or antagonists acting directly on SPR polypeptide will result in identical responses, independent of the applied G-alpha-subunit and the coupled downstream signaling pathway. In contrast, compounds positively or negatively interfering with the downstream signaling cascade will lead to different signals depending on the employed G alpha subunit.

In first instance, it may be useful to provide the expressed SPR polypeptide together with its naturally occurring SP to reconstitute the expected functional response in the assay format of interest. The expressed SPR is then brought into contact with a test compound to detect its binding and ability to stimulate or inhibit receptor activation, respectively, by monitoring a functional response, as described below.

In some embodiments, the SPR assay procedure is based on the use of cells which express a SPR polypeptide (for example, SPR-transfected CHO cells that have been co-transfected with a G protein alpha subunit or that express an SPR/alpha subunit fusion protein) and in which, upon SPR activation or inhibition, a second messenger response (signal) can be measured, such as a change in the intra- and extra-cellular pH value or alterations in the level of a second messenger molecule in the signal transduction pathway that is caused by an increase or inhibition of receptor activation. A detailed description and summary of useful methods is given by Schnitzer and Sommergruber (2006) and references cited therein. In general, these methods involve cells which transiently or stably express the SPR polypeptide, and the respective G alpha polypeptide, for example, insect cells like Sf9 or S2 cells, *Xenopus* oocytes or mammalian cell lines such as CHO, HEK 293, HMEC, HIVE-55, HIVS-125, RBL-2H3.

The transfected cells are then incubated with the native (endogenous) SP (or an SP sufficiently similar to engage the SPR and induce signal transduction) and the test compound. Inhibition or activation of the receptor is then determined by measuring alterations of a signal that reflects changes in the receptor-coupled signal transduction pathway, such as changes in the cAMP or intracellular calcium levels or changes in the intra- and extracellular pH value. For example, methods to measure changes in intracellular calcium concentration in a high throughput mode in microtiterplates have been already developed (Stables et al., 1997; Coward et al., 1999).

In other embodiments, the assay method of the invention is based on a binding assay which involves, in the presence or absence of a test compound, determination of inhibition of binding of the labeled SP (competition for binding), to cells which present SPR on the surface, or cell membranes containing the SPR.

Such a method involves transfecting a eukaryotic cell (e.g. mammalian, insect, amphibian, as described herein) with DNA encoding the SPR and the G alpha subunit in a way that the cell expresses the SPR on its surface. The cell, or the membrane fraction, respectively, is then incubated with the test compound, e.g. a potential antagonist, in the presence of the SP, which is labeled, e.g. with radioactivity or fluorescence. The amount of labeled SP bound to the receptor is measured, e.g., by measuring radioactivity associated with transfected cells or membrane fractions. If the test compound binds to the SPR, binding of labeled SP to the receptor is inhibited, which results in a reduction of labeled SP bound to the SPR. The same technique can be used to screen for agonists. Whether a compound acts as an agonist or an antagonist can be determined by analyzing the downstream signaling events such as the increase of cAMP. In the case of activation, an increase in cAMP levels is observed; in the presence of an inhibitor, a decrease is observed.

The assay methods of the present invention that employ recombinant cell lines expressing the SPR and a G alpha subunit allow to screen for specific agonists or antagonists of SPR even if the endogenous SP is not known yet. If the endogenous SP is not known, a surrogate agonist is identified in order to be able to screen for inhibitors as e.g. described in Example 6.

Since the activity of GPCRs, including SPR, initiates signaling events in the cell that may act on response elements contained in a reporter gene construct and are thus detectable via expression of a reporter molecule, the present invention relates, in a further aspect, to a reporter-based SPR assay. An SPR assay of the invention is a reporter-based assay that measures the activity of a reporter gene product in cells genetically engineered to express the reporter molecule under the control of a regulatory sequence containing a specific promoter and associated response elements that respond to specific signals, e.g. molecules of a specific signal transduction cascade.

An SPR reporter assay of the invention works according to the same principle as the method described in WO 93/11257, which determines whether a compound has a modulating effect on a receptor-dependent signal transduction pathway in test cells that are transformed with a DNA coding for a receptor coupled to the phospholipase effector system, in particular a G protein-coupled receptor, and a reporter gene with a regulatory sequence ("TRE element") functionally linked thereto that is sensitive to the IP₃/DAG concentration (IP₃ and DAG being second messenger molecules generated upon activation of the receptor-coupled signaling cascade). For this embodiment, the SPR may be co-expressed with a G alpha subunit (or expressed as a fusion protein) that stimulates phospholipase C, e.g. Gq or a chimeric G alpha subunit of the same signaling phenotype. In another embodiment, i.e. in the case that a G alpha subunit is used that activates adenylate cyclase (a Gs subunit or a chimera), the response element may be selected from CRE elements (cAMP responsive elements), which are also described in WO 93/11257. As an alternative to naturally occurring CRE or TRE elements, synthetic regulatory regions containing the respective response element may be used, as also described in WO 93/11161. To enhance the effect of the second messenger on the regulatory sequence, it may contain several copies of the response element. The promoter region of the ICAM-1 receptor, which confers response to several signaling pathways (Voraberger et al., 1991; Stratowa and Audette, 1995), may also be used. An artificial multiple regulatory element containing the major response elements, through which different G proteins can signal, allows to set up a reporter assay without knowledge of the coupling mechanism of a given SPR (Chin et al., 2003).

Specificity and sensitivity of the system are controlled by the choice of the promoter and the transcriptional control region, which determine the basal expression level and the degree of transcriptional response. Examples for frequently used reporter genes that are suitable in the method of the present invention are secreted alkaline phosphatase, β-galactosidase, GFP, luciferase, and β-lactamase out of a larger number of available reporter genes, which have been reviewed in Suto and Ignar , 1997). A particularly useful reporter is SEAP (secreted placental alkaline phosphatase; Berger et al., 1988), which is a mutated form of the human placental enzyme and can easily be monitored by colorimetric, fluorescent and chemiluminescent detection systems, e.g. a chemiluminescence assay based on the use of 1,2-dioxetane substrates.

Another useful reporter is β-galactosidase, commonly referred to as lacZ, for which different substrates have been developed (Jain and Magrath, 1991; Naylor, 1999) including those for fluorometric analysis (Labrousse et al., 1982) or laser-induced fluorescence detection of β-gal protein levels. A luminescence-based assay even lowers the detection limit of β-gal to ∼2fg and is about three times more sensitive than the above mentioned fluorescence assays (Bronstein et al., 1996). Another useful reporter gene is green fluorescent protein (GFP; Cubitt et al., 1995) and variants GFP with increased thermostability, intensity, and shifts within the fluorescence spectra (Tsien, 1998).

In other embodiments, the reporter gene is the widely used luciferase gene from *Photinus pyralis* (de Wet et al., 1987). Optimized reaction buffers have lead to a prolonged half-life of the signal to a so-called "glow reaction" which is stable for more than 1 hour. Appropriate buffers are commercially available (e.g. Dual-Glo Luciferase Assay System from Promega). The assay protocol is very simple and only requires the addition of the reaction buffer which contains ATP and luciferin and lyses the cells.
Another example for a reporter is *Renilla* luciferase from sea pansy *Renilla reniformis*. The activity of *Renilla* luciferase can be monitored in live cells, thus eliminating the need for lysis of cells and allows for multiple measurements. Recently, a novel *Renilla* luciferase substrate, EnduRen™ has been become available from Promega, which is significantly more stable and should allow prolonged real-time measurements.

Another reporter enzyme useful in the SPR assay method of the invention is bacterial β-lactamase (Moore et al., 1997). The β-lactamase cell-based assay system couples molecular and biological events to a fluorescence resonance energy transfer (FRET)-based detection method in single live cells (GeneBLAzer™ technology, Invitrogen). Several favourable properties (e.g. no background, high sensitivity) and miniaturization beyond the 384-well plate format to 3456-well format (Kornienko et al., 2004) make the β-lactamase a very attractive reporter gene, especially for screening purposes.

In another embodiment, the SPR assay method of the invention is based on the determination of the intracellular Ca²⁺ release. As for the methods described above, it involves the use of mammalian or insect or other suitable cells, which are transfected to express the SPR and the G alpha subunit. The cells are loaded with an indicator dye that produces a fluorescent signal when bound to calcium, e.g.
FLUO-3 and FLUO-4 (Gee et al., 2000), or a recently developed "no-wash calcium assay kit" (Molecular Devices). In the presence of the receptor ligand, and in the presence or absence of the test compound, the fluorescent signal is measured over a defined period of time using, for example, a fluorescence spectrophotometer or a fluorescence imaging plate reader. A decrease or increase in the fluorescence signal indicates that a compound is a potential antagonist or agonist, respectively, for SPR. The assay may be performed in a cell-based microarrayed screening format (Gopalakrishnan et al., 2003).

In another embodiment, the SPR assay method of the invention is based on a similar principle, i.e. the measurement of the change in intracellular calcium concentration. As in the other assay methods, SPR and the G alpha subunit are overexpressed in the test cells.
In one of the methods useful to measure changes in intracellular calcium concentration, the test cells are contransfected with apoaequorin. Coelenterazine is added as cofactor to the reaction. Binding of calcium leads to an oxidation reaction generating coelenteramide, CO₂, and luminescence at 469 nm (Brini et al., 1995). Another technology uses the addition of fluorescent calcium indicators, which change fluorescence intensity dependent upon calcium concentration such as FLUO-3 and FLUO-4 (Gee et al., 2000), or a novel "no-wash calcium assay kit" (Molecular Devices). The convenience of the second approach is that is does not require the cotransfection of apoaequorin. This together with the development of a powerful fluorescent imaging plate reader (FLIPR, Molecular Devices), equipped with an argon laser, a cooled CCD camera, fast simultanoues liquid transfer, plate handling capabilities and data evaluation software, allows to run homogeneous kinetic cellular fluorescent assays with time resolution of seconds down to 1536-well plate formats in high throughput. The fluorescence or bioluminescence-based methods listed above have been described to be suitable for a high throughput mode in microtiterplates, e.g. Stables et al., 1997; Coward et al., 1999; Le Poul et al., 2002. An advantage of this approach is that the changes in concentration can be monitored very fast within seconds or minutes after activation of the signaling cascade and the measured interaction point is further upstream than a reporter gene response. Therefore, general toxic effects of compounds and unspecific interactions at the different steps of the signaling cascade are reduced. The activity of a SPR-G-alpha fusion polypeptide such as SPR-G_{q} signals through the phospholipase C cascade, which subsequently leads to changes in the intracellular calcium concentration and can therefore be monitored by these approaches (Jerman et al., 2001). It has thus made this technology the first choice for high throughput applications in the field of intracellular calcium measurement. Such an approach will be very useful to identify and characterize native ligands of SPR and to screen for novel synthetic inhibitory and activating compounds for pharmaceutical development.

In another embodiment, the SPR assay of the invention is based on the detection of a test compound's effect on conformational changes (e.g. oligomerisation, interaction with β-arrestin and internalization etc.) of the SPR upon activation by means of FRET (fluorescence resonance energy transfer) or BRET (bioluminescence resonance energy transfer). The general principle of these technologies is based on the transfection of the cells with genes of the interacting proteins fused to two different fusion partners between which resonance energy transfer or enzyme complementation occurs if both are in close proximity.

FRET may be applied to investigate conformational changes within SPR upon agonist binding by incorporating two different color variants of GFP, CFP and YFP into different parts of the receptor. Vilardaga and colleagues have already used this method to analyze activation of GPCRs (Vilardaga et al., 2003).

In contrast to FRET, where the donor molecule is fluorescent and must be excited with monochromatic light, BRET uses a bioluminescent donor molecule, which does not require an external light source. Most frequently used donor-acceptor pairs are *Renilla* luciferase and different color variants of green fluorescent protein (GFP) from *Aequoria victoria* which have been developed during the last few years.

BRET has been used successfully *in vitro* and *in vivo* to identify and investigate molecular interactions, e.g. hetero- and homodimerization of GPCRs (Michelini et al., 2004) or the interaction of GPCRs with β-arrestin (Angers et al., 2000), which only binds to the activated form of the receptor. A recent overview over the application of BRET and FRET in the area of GPCR complex formation is given by Pfleger and Eidne, 2003. Following the protocol of Angers et al., 2000, a similar approach might be performed for SPR.

Another SPR assay of the invention is based on agonist-mediated β-arrestin translocation by the SPR. The beta.-arrestin translocation assay is a protein-protein interaction assay, which involves a cell expressing the SPR and a β-arrestin fused to e.g. a fluorescent molecule, such as a GFP molecule. The SPR is located in the membrane of the cell, and the β-arrestin molecules are located in the cytoplasm. When the SPR is activated by an agonist (e.g. the SP) the SPR will bind the β-arrestin fusion molecule. This can be seen as a movement (translocation) of fluorescence, if a fluorescent molecule is used, from the cytoplasm to the membrane or just as a general movement of fluorescence. Utilizing this mechanism, a universally applicable screening assay has been developed (Vrecl et al., 2004; Garippa et al., 2006; US 2006/0246507), which is amenable to the HTS format. This system is independent of the signaling pathway and allows ratiometric measurement and thus not dependent on the number of cells per well, which significantly reduces variability. Also, since it directly monitors the interaction of the target of interest, the number of false positive hits is reduced as compared to reporter gene assays, which measure the signal of a signaling cascade with many more steps. To establish an appropriate high quality SPR assay, which is suitable for the HTS format, different combinations ofN- and C-terminal fusion constructs of beta-arrestin 2 are tested in preliminary assays, since the resonance energy transfer efficiency is dependent on the proper orientation of donor and acceptor molecule, an unfavorable geometry can lead to a poor signal (Vrecl et al., 2004).

In a further aspect, the present invention relates to a SPR assay that is useful for determining whether a compound has a modulating effect on a SPR-coupled signal transduction pathway, i.e. a compound that does not act by directly modulating SPR activation, but that acts on a signaling molecule further downstream in the SPR-coupled cascade.

Again, this involves the use of cells (transfected to express both the SPR and the G alpha subunit as well as a reporter gene , e.g. luciferase or beta-galactosidase) whose expression is coupled to the activation of the SPR, e.g. by being under the control of the promoter of a transcription factor at the end of the SPR signaling cascade, e.g. a STAT promoter in the case of a phospholipase C readout).

In another embodiment, the SPR assay of the invention is in the form of a translocation assay, in which, upon SPR-coupled activation, the translocation of a transcription factor from the cytoplasm to the nucleus is determined, e.g. in a high content screening. The potential of cell-based translocation assays for GPCR discovery has been described for CXCR4 (Granas et al., 2005). The reporter signal is measured under similar conditions as described above after a defined period of time. The signal can be measured using a luminometer, spectrophotometer, fluorometer, or any other instrument appropriate for the signal generated by the specific reporter gene product. Reduction of the signal indicates that a compound might be a potential antagonist for the receptor.

In another aspect, the SPR assay method of the invention is based on determining a decrease or increase of the cAMP level and/or inhibition or activation of adenylate cyclase mediated by SPR and the G alpha subunit. Again, such an approach depends on transiently or stably transfected cells expressing SPR and the G alpha subunit. These cells are exposed to test compounds in the presence of the SP ligand. For example, the change the cAMP levels is then measured over a defined period of time, for example, by radio-immuno or protein binding assays (e.g. using Flashplates or a scintillation proximity assay). Changes in cAMP levels can also be determined by directly measuring the activity of the enzyme, adenylate cyclase, in cell lysate preparations. If the potential antagonist binds the receptor, the levels of SPR-mediated cAMP, or adenylate cyclase activity, will be reduced; in the case of an agonist, such activity will be found to be increased.

In certain embodiments, the assay methods of the invention described above are run as in the high-throughput screening format for identifying compounds as SPR modulators and thus as a potential pest control agents. In certain other embodiments, the assay methods of the invention are used for identifying lead structures and/or optimizing a compound that has been identified as a hit in an antecedent screening assay. Thus, for the purpose of the present invention, the term "identifying" a compound in the context of assay methods also has the meaning of optimizing a compound, i.e. determining a compound's effect that has been obtained by modifying the structure of a lead compound.

The specificity of a compound as a modulator, in particular as an inhibitor, of the SPR can be confirmed in a secondary assay that is run after, or in a control assay that is run in parallel to the primary screening assay. By way of example, such control assay employs cells that do not express the SPR but are transfected with the G alpha subunit that is used in the primary assay.

To identify modulators that are specific for an SPR of a species of interest, e.g. for controlling only that one insect species without affecting other insect species, e.g. a useful insect species, the present invention further provides a method for identifying a compound that modulates an SPR from a first species but not from a second or other species. Such method employs two or more identical cell lines, one of which is transfected with the SPR of interest and with the G alpha subunit and the other(s) are transfected with the identical G alpha subunit and an SPR of another insect species that should not be affected. Such assays, which are usually run in parallel, provide a maximum flexibility in that they also allow for determining compounds that modulate SPRs from more than one insect species of interest.

A useful sequence of assays may include, as a first step, a binding assay in a screening assay format (e.g. using a membrane fraction of SPR-expressing cells and a fluorescently labeled SP, incubating with the test compounds and determining competition for binding). In a subsequent second step, a functional assay, e.g. using cells expressing the SPR and a G alpha subunit in an assay with a cAMP readout, is employed to determine whether the hits obtained in the preceding first competition assay have an effect on SPR activation. Next, a specificity assay employing cells not expressing the SPR or expressing SPRs with other species, as may follow to exclude the false positive.

In a further aspect, the present invention features methods based on computer modeling and searching technologies that permit identification of compounds that can modulate SPR activity. Having identified such a compound or composition, the active sites or regions are preferably identified. Such active sites might typically be SP binding sites, such as the interaction domains of the SP with the SPR. The active site can be identified using methods known in the art including, for example, from the amino acid sequences of peptides, from the nucleotide sequences of nucleic acids, or from study of complexes of SPR with their natural SPs. In the latter case, chemical or X-ray crystallographic methods can be used to find the active site by finding where on the SPR polypeptides the complexed SP is found.

The three dimensional geometric structure of the active site can also be determined. This can be done by known methods, including X-ray crystallography, which can determine a complete molecular structure. Solid or liquid phase NMR can also be used to determine certain intra-molecular distances within the active site and/or in the SP/SPR complex. Any other experimental method of structure determination can be used to obtain partial or complete geometric structures. The geometric structures may be measured with a complexed SP, which may increase the accuracy of the active site structure determined.

Methods of computer based numerical modeling can be used to complete the or to improve its accuracy. Any modeling method known in the art may be used, e.g. parameterized models specific to particular biopolymers, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. Exemplary forcefields that are known in the art and can be used in such methods include, but are not limited to, the Constant Valence Force Field (CVFF), the AMBER force field and the CHARM force field. The incomplete or less accurate experimental structures can serve as constraints on the complete and more accurate structures computed by these modeling methods.

Having determined the structure of the active site, either experimentally, by modeling, or by a combination of such methods, candidate pest agents can be identified by searching databases containing compounds along with information on their molecular structure. Such searches aim at identifying compounds with structures that match the determined active site structure and that interact with the chemical groups defining the active site. Such a search can be manual, but is usually computer assisted. Alternatively, such methods can be used to optimize compounds. The structure of a compound can be modified and the structural effects of modification can be determined by applying the structural modeling methods described. The altered structure is then compared to the active site structure with regard to an improved fit or interaction. By this approach, variations of a given compound can be evaluated to obtain modified compounds of improved specificity or activity.

Molecular modeling systems are routinely used by the skilled person, e.g. the CHARMm and QUANTA programs (Polygen Corporation, Waltham, Mass.). CHARMm performs the energy minimization and molecular dynamics functions. QUANTA performs the construction, graphic modelling and analysis of molecular structure. QUANTA allows interactive construction, modification, visualization, and analysis of the behavior of molecules with each other.

Compounds identified and/or optimized as SPR modulators in one of the above assay or modeling methods can be tested for their efficacy as pest control agents that affect the switch to post-mating behavior in female insects, by subjecting females from the species of interest to behavioral assays, e.g. as described in the Examples, or for insect feeding, host-seeking, or oviposition site searching behaviors. The assay method for such behaviors (e.g. host seeking behavior) varies depending on the life history of the insect species of interest. In mosquitoes, host-seeking behavior is measured using an olfactometer (e.g. as described by Klowden et al. 1978).
For use in the control of insects that act as disease vectors, the agents of choice will be antagonists/inhibitors of the SPR, while for agricultural pests, both antagonists/inhibitors and agonists/activators are useful for combating such pests.

Agonists and antagonists of the SPR identified and/or optimized by any of the assay methods described above, or peptidometics, may be formulated, as described in US 2005/05393, as active pest control agents (in the following "active agents") with any carrier suitable for agricultural use, such as water, organic solvents and/or inorganic solvents. The pest control agent composition may be in the form of a solid, semi-solid or liquid composition and may be prepared by any conventional formulation process, including but not limited to dissolving, mixing, milling, granulating, and dispersing. By way of example, granules can be formed by impregnating pellets of filler with the composition ingredients, or by pelleting a dry blend composition in admixture with a powdered filler.

The present invention encompasses compositions containing the active agent in admixture with excipients known in the art, e.g. agriculturally acceptable excipients described in US 2005/05393, including but not limited to vehicles, carriers, binders, UV blockers, adhesives, thickeners, dispersing agents, preservatives, surfactants and insect attractants. According to the invention, the composition may, for example, be in solid form comprising the active agent and a finely divided solid carrier. Alternatively, the active agent may be contained in liquid compositions including solutions, dispersions, emulsions and suspensions. Any suitable final formulation may be used, including for example, granules, powder, pellets (e.g. bait pellets containing the active agent and an insect attractant), microcapsules, water dispersible granules, emulsions and emulsified concentrates.

Examples of solid diluents suitable for use with the present invention include but are not limited to organic or inorganic materials such as starch, active carbon, soybean powder, wheat powder, wood powder, fish powder, powdered milk, talc, kaolin, bentonite, calcium carbonate, zeolite, diatomaceous earth, fine silica powder, clay, alumina, pyrophyllite, kieselguhr, chalk, lime, fuller's earth, magnesia, gypsum, cottonseed hulls, pumice, tripoli, walnut shell flour, redwood flour, and lignin.

Examples of liquid carriers suitable for a composition of the present invention and described in US 2005/053933, include but are not limited to water, isopropyl alcohol, ethylene glycol, cyclohexanone, methyl ethyl ketone, dioxane, tetrahydrofuran, kerosene, light oil, xylene, trimethylbenzene, tetramethylbenzene, methylnaphthalene, solvent naphtha, chlorobenzene, dimethylacetamide, a glycerin ester, an acetonitrile, or dimethylsulfoxide.

For agricultural applications, compositions containing the active agents can be applied in the form of sprays, droplets, microfilms or microcapsules to the trees, plants or areas to be treated, using conventional devices known in the art. The agent may also be formulated for controlled release. Particularly for applications to areas that are subject to reinfestation, such as mosquito-infested ponds. The composition may be in the form of dispersion coating, film coating, spray coating, microencapsulated products, polymer slow release drops, globs, blocks, such as paraffin blocks, monoliths, buffers, and any such other similar form as known in the art. Various controlled-release systems are described in Controlled Delivery of Crop-Protection Agents, Taylor and Francis, New York, (1990), Editor R. M. Wilkins, especially chapters 3 and 9 and in Insect Suppression with Controlled Release Pheromone Systems, Vol. I and II, CRC Press, Boca Raton, Fla. (1982).

Such controlled release and/or dispensing means may advantageously be employed to consistently maintain an effective concentration of one or agents in a specific pest habitat, such as a pond or other mosquito-producing body of water.

Depending on the circumstances, such as the type of the insect population, its habitat and the extent of the area to be covered, various methods of applying the composition may be used.

The SPR modulators are generally administered to the insect by oral ingestion, but may also be administered by means which permit penetration through the cuticle or penetration of the insect respiratory system. If intended for oral uptake, it is in general necessary to mix the active agent with or apply it to substances or products that the insects feed on. The agents are preferably associated with the food in a manner that it is not possible for the insect to feed without ingesting the agent. For example, compositions may be used which contain the active agent together with appropriate foodstuff like carbohydrates, in particular sugars, proteins of animal or vegetable origin, amino acids, extracts etc. Various substances may be added to attract the insects. Such substances may be foodstuffs for which the insects have a specific preference or they may be specific attractants, e.g., brewer's yeast, vanillin, milk-powder, molasses, dyes, odorants, muscamone, etc.

Examples for preservatives are benzoic acid, butylated hydroxyanisole, butylated hydroxytoluene, etc., may be added to improve the stability of the active agent(s). Where the agents are formulated to be orally administered to the insect pests, the agents can be administered alone or in association with an insect food.

Surfactants may be cationic, anionic or nonionic type, or a mixture thereof.
Suitable surfactants include, without limitation, cetyltrimethylammonium bromide; sodium lauryl sulfate; sodium dodecylbenzene-sulfonate; ammonium lignosulfonate; condensation products of ethylene oxide with fatty alcohols, amines or alkylphenols.

The compositions of the invention, which may take any physical form as described above, e.g. liquid, semi-solid (e.g. pastes) or solid (e.g. powders, granulates etc.), may be applied to specific localized areas, in particular in the form of baits, or they may be sprayed over part or all of the area to be treated e.g., by dusting, spraying, smearing, etc.

When the particular insect species completely or partially feeds on blood, it may be appropriate to administer the compound, in appropriate amounts, to the host animals.

For controlling insects that infest mammals, e.g. lice, topical formulations may be used, e.g. in the form of a pour-on formulation, a spot-on formulation, a spray, a shampoo, a dusting powder, an impregnated strip, a soap or a gel.

Depending on the nature of the compositions and the circumstances of its application, the concentration of the active agent may vary within wide limits. In general the concentration of the active agent will not be lower than about 0.001 %. In principle, there is no upper limit. In general, the concentration does not exceed about 50%, advantageously, it is below about 10 %.

Compositions containing SPR modulators can also be applied, as described in US 6,593,299, as a seed-coating or root treatment or total plant treatment at later stages of the crop cycle. Plant and soil treatments may be employed as wettable powders, granules or dusts, by mixing with various inert organic or inorganic solid materials, as described above.

The SPR modulators can be delivered to the environment using a variety of devices known in the art of pesticide administration; among particularly useful devices are those which permit continuous extended or pulsed extended delivery of the composition. For example, a fluid-imbibing dispensing device as described in US 5,417,682, or osmotic dispensing devices.

### Brief description of the Figures

**Fig. 1****:** *CG16752* is required for post-mating responses induced by SP
**Fig. 2****:** *CG16752* encodes a specific sex peptide receptor
**Fig. 3****:** SPR function is required in *fru* neurons
**Fig. 4****:** Structural and functional conservation of insect SPRs
**Fig. 5****:** Sequence alignment of the amino acid sequences of various SPRs
If not otherwise indicated, the following materials and methods are used:

### Methods

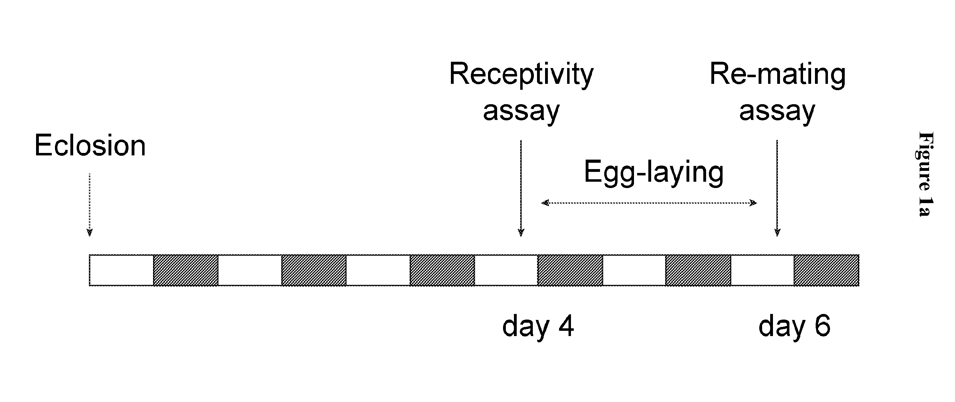

**Fly stocks.** *UAS-SPR-IR1* (*UAS-CG16752-IR1*) is obtained from the genome-wide transgenic RNAi library (Dietzl et al., 2007), maintained at the Vienna *Drosophila* RNAi Center. *UAS-SPR-IR2* is generated by cloning a 352 bp PCR product from the RE15519 cDNA (Drosophila Genomics Resource Center) as an inverted repeat into a custom-designed *UAS* vector, and then inserting this transgene into a specific 2nd chromosome site (VIE-28b) using the φC31 system (Groth et al., 2004). *UAS SPR* is generated by cloning the entire *SPR* coding region from RE15519 into a similar custom-designed *UAS* vector, followed by integration at a different site on the 2nd chromosome (VIE-72a). The *Df(1)Exel6234* stock (Parks et al., 2004), is obtained from the Bloomington *Drosophila* Stock Center and verified by a series of PCRs on genomic DNA extracted from homozygous and control flies. The original line is then recombined with *white*⁺, and crossed for 3 generations into a Canton S background. Canton S is used as wild-type in all experiments. Other stocks used are *elav-GAL4* (Luo et al., 1994), *fru*^{GAL4} (Stockinger et al., 2005), *SP*⁰ / TM3, Sb (Liu and Kubli, 2003), Δ¹³⁰ / TM3, Sb (Liu and Kubli, 2003), *UAS-laminGFP* (Aza-Blanc et al., 2000), and *dj-GFP* (Backhaus et al., 1984). *SP* null males are *SP*⁰ / Δ¹³⁰ (Liu and Kubli, 2003). Both the *elav-GAL4* and *fru*^{GAL4} stocks additionally carry a *UAS-Dcr-2* insertion on the X chromosome (Dietzl et al., 2007).
**Behavioral assays.** All flies are raised on semi-defined medium (Backhaus et al., 1984), at 25°C in a 12 h:12 h dark:light cycle. Virgin males and females are collected at eclosion. Males are aged individually for 5 days; females are aged for 4 days in groups of 10-15. All assays are performed at circadian time 6:00-10:00, and on at least 3 independent occasions. For assays performed according to the protocol in Fig. 1a, single female and male virgins are paired in 10 mm diameter chambers and videotaped for 1 h. The time to copulation is recorded for each female. Those females that copulate are then transferred to single food vials for 48 h, and the number of eggs laid by each female is counted manually. Females are then re-tested for receptivity in the same manner in pairings with naive Canton S males. The data set for the *elav-GAL4* / + controls is pooled data from two separate series of experiments in which the *elav-GAL4* driver is crossed to each of the respective parental strains for the two *UAS-SPR-IR* transgenes. These two sets of *elav-GAL4* / + controls are not significant different in any of the assays.
SP injections into the abdomen of virgin females are performed as described previously (Schmidt et al., 1993). Following injection, females are transferred to individual food vials and tested after 5 h for receptivity with a naive Canton S male.
**CHO cell assays.** CHO-K1 cells are transiently transfected essentially as described previously (Kim et al., 2004). The relevant GPCRs are expressed from constructs prepared by cloning the entire open reading frame in a pcDNA3.1(+) vector (Invitrogen). Expression constructs for CG2114 (Johnson et al., 2003), CG8784 (Park et al., 2002), the chimeric G proteins (Gα_{qs}/qs5-HA, Gα_{qi}/qi5-HA, and Gα_{qo}/qo5-HA) (Conklin, et al., 1993 and 1996), and codon-optimized aequorin (hucytaeqpcDNA3) (Vernon and Printen, 2002), have been described previously. Luminescent signals are measured with a Synergy2 photometer (BioTek). The *Drosophila* peptides used in this study are as follows ('a', amidated C termini; pQ, pyro-glutamic acid; P, hyroxyproline; **C**, cysteine residues linked by disulphide bridge): FMRFamide-2 (DPKQDFMRFa; SEQ ID NO:19), FMRFamide-3 (TPAEDFMRFa; SEQ ID NO:20), sulfakinin (SK)-0 (NQKTMSFa; SEQ ID NO:21), SK-1 (FDDYGHMRFa; SEQ ID NO:22), SK-2 (GGDDQFDDYGHMRFa; SEQ ID NO:23), myosuppressin (MS; TDVDHVFLRFa; SEQ ID NO:24), hugin-γ (pQLQSNGEPAYRVRTPRLa; SEQ ID NO:25), pyrokinin (PK)-2 (SVPFKPRLa; SEQ ID NO:26), synthetic sex peptide (SP; WEWPWNRKPTKFPIPSPNPRDKW**C**RLNLGPAWGGR**C**; SEQ ID NO:27), and synthetic DUP99B (DUP99B; pQDRNDTEWIQSQKDREKW**C**RLNLGPYLGGR**C**; SEQ ID NO:28). These peptides are synthesized using the Fmoc-strategy and solid-phase method on an ABI 433A Peptide Synthesizer and purified with HPLC. For SP and DUP99B, purified peptides are folded prior to a second HPLC purification by incubating them in 0.01 M ammonium bicarbonate (pH 8) containing 3% DMSO for 36 h.

**Immunohistochemistry.** A synthetic peptide corresponding to the predicted N-terminal 21 amino acids of the mature SPR (PTNESQLEIPDYGNESLDYPNC-OH SEQ ID NO:29) is conjugated to KLH and used to generate rabbit antisera (Gramsch Laboratories). SPR antisera are cleaned by incubating with equal volume of *Df(1)Exel6234* embryos overnight at 4°C. Wandering 3^{rd} instar larva and 8-10 d virgin females and males are dissected under PBS (pH7.4). Tissues are fixed for overnight at 4°C in 4% paraformaldehyde in PBS (or in some cases at room temperature for 2 h). The tissues are incubated in primary antibody (1:500) for 48 h at 4°C, and in secondary antibody for 24 h at 4°C. Other antibodies used are: rat anti-elav (1:500; O'Neill et al., 1994), mouse anti-GFP (1:1000; Chemicon), Alexa 488-conjugated goat anti-rabbit, Alexa 568-conjugated goat anti-mouse and Alex 633-conjugated goat anti-rat (all 1:1000; Molecular Probes). Images are acquired with a Zeiss LSM 510/Axiovert 200M and processed in Adobe Photoshop.
**Cloning of other insect *SPR* genes.** *SPR* orthologues are identified by TBLASTN searches on the relevant genome assemblies, and gene structures predicted using Genscan (http://genes.mit.edu/GENSCAN.html). The complete ORF of each *SPR* orthologue is amplified by RT-PCR using the following primers:
*D*. *pseudoobscura*, forward 5'-atgggcggcgatcaaggggt (SEQ ID NO:30), reverse 5'-ggcaccaacatcaccaatta (SEQ ID NO:31); *A*. *aegypti* forward 5'-atgtcaattgatgctgcggta (SEQ ID NO:32), reverse 5'- cgttggttctgtgtgacaaa (SEQ ID NO:33); *A*. *gambiae* forward 5'-atgattgaaaaaaataatttcaag (SEQ ID NO:34), 5'-cctgctatctaaccacagt (SEQ ID NO:35); *B*. *mori* forward 5'-atggcggtcaccatagacaa (SEQ ID NO:36), reverse 5'-ggcttaaagcacagtttcgt (SEQ ID NO:37); *T*. *castaneum* forward 5'-atgggcgagatggcgtcgaac (SEQ ID NO:38), reverse 5'-tcaacattgagtttgtcctaa (SEQ ID NO:39). *D*. *pseudoobscura* is obtained from the Tucson *Drosophila* Stock Center (stock number, 14011-0121). Frozen stocks of *Aedes aeqypti* (MRA-735B) and *Anopheles gambiae* (MRA-132B) are obtained from the MR4 Resource Center (VA). *Tribolium castaneum* and *Bombyx mori* are obtained by freezing female insects. The predicted protein sequences are analyzed with TMpred (http://www.ch.embnet.org/sofware/TMPRED_frrm.html) to confirm the presence of seven transmembrane domains.

**Phylogenetic analysis.** Using the insect SPRs, NCBI-BLASTP searches (Altschul et al., 1990), are performed against the NCBI non redundant protein database and all *H*.*sapiens*, *D*.*melanogaster* and *C*.*elegans* entries are collected that are below a highly significant e-value of 1e-5. In an alternative approach, a profile hidden Markov model (HMM) (Eddy, 1998) is built out of the insect SPR conserved region and additional proteins with a significant e-value below 0.001 are collected. A 90 per cent redundant protein set (without recent duplications, sequencing errors and splice variants) is aligned using MUSCLE (Edgar, 2004) and graphically processed with Clustal X (Jeanmougin et al., 1998). The phylogenetic tree is calculated with PHYLIP (Felsenstein, 2005) using the Jones-Taylor-Thornton matrix as distance algorithm and the neighbour-joining method for tree calculation. The image is generated with the help of the program Phylodendron by Gilbert. Sequences and NCBI accession numbers: *Drosophila melanogaster*: CG13229 (gb|AAM28948.1|), CG13803 (gb|AAF47633.2|), CG8985 (gb|AAF47635.2|), CG2114 (tpg|DAA00378.1|), CG33696 (ref|NP_001027122.1|), ETHRa (gb|AA020966.1|), CG8795 (ref|NP_731788.1|), CG8784 (ref|NP_731790.1|), CG14575 (ref|NP_996140.1|), CG6857 (ref|NP_523404.2|); *Caenorhabditis elegans*: R03A10.6 (emb|CAA93674.2|), Y69A2AR.15 (gb|AAK68559.2|), F42D1.3 (emb|CAB03091.2|), F57B7.1a (emb|CAA98492.1|), C35A5.7 (emb|CAA94909.2|), C35A11.1 (gb|AAB66039.3|), F39B3.2 (gb|AAB07577.2|); *Homo sapiens:* GPR142 (ref|NP_861455.1|), GPR139 (sp|Q6DWJ6|), TRHR (ref|NP_003292.1|), NMUR2 (ref|NP_064552.2|), NMUR1 (gb|AAH36543.1|), A2b_R (ref|NP_000667.1|), NK-1_R (gb|AAA59936.1|), NK-2-_R (gb|AAB05897.1|), NK-3_R (gb|AAB21706.1|), GPR50_Hs (gb|AAI03697.1|), SSTR3_Hs (ref|NP_001042.1|).

### Example 1

### CG16752 is required for post-mating responses induced by SP

The gene *CG16752,* predicted to encode a G protein coupled receptor (GPCR), is identified in an ongoing genome-wide transgenic RNAi screen for genes required in the female nervous system for the post-mating switch in reproductive behavior.

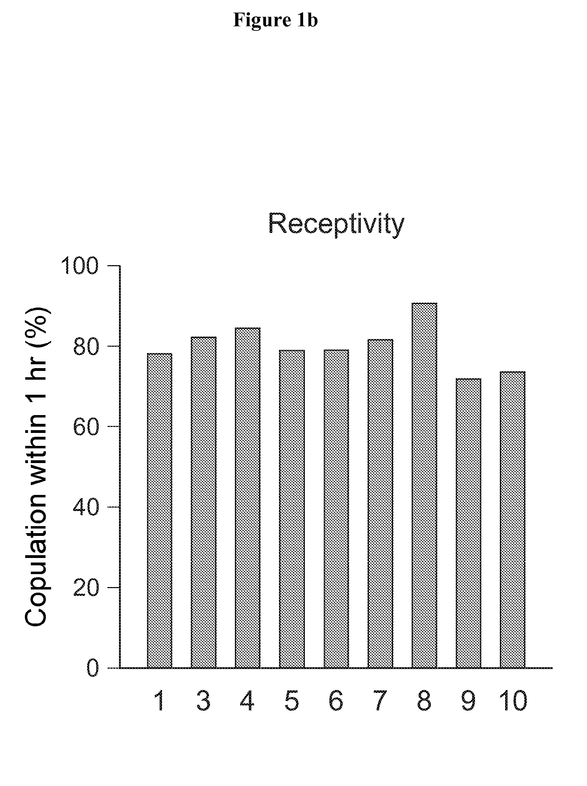

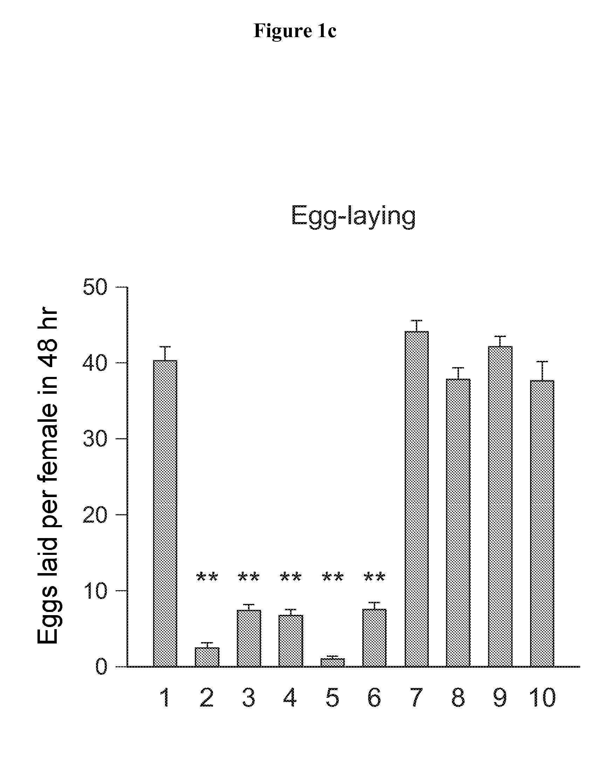

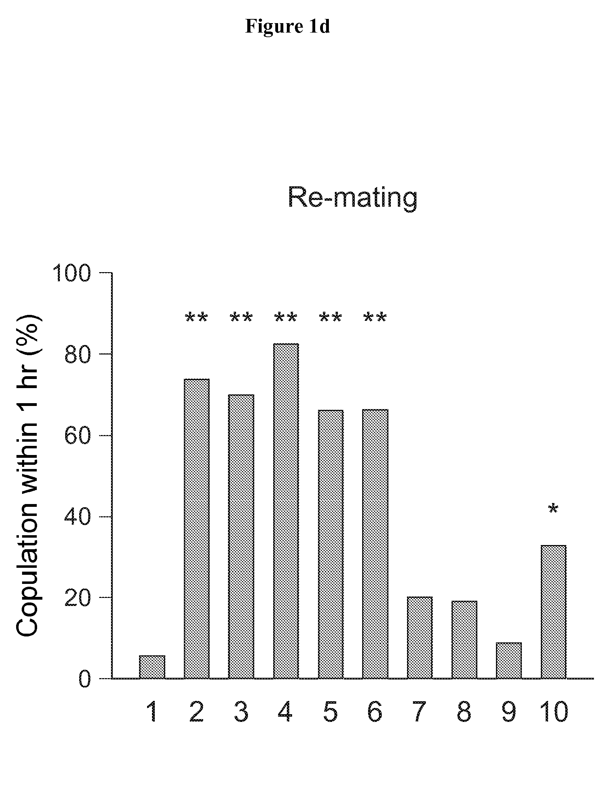

Specifically, it is found that expression of a *CG16752* RNAi transgene (Dietzl et al., 2007), (*UAS-CG16752-IR1*) with the pan-neuronal driver *elav-GAL4* leads to a dramatic reduction in egg laying. To more carefully examine this egg-laying phenotype, and to additionally assess mating receptivity of virgin and mated females, a protocol is used in which individual virgin females first tested for receptivity to naive males. Those females that mated are then allowed to lay eggs for 48 hours before being retested for receptivity to a second naive male (Fig. 1a). In these assays, wild-type females are used as controls that do switch, as well as females carrying either *elav-GAL4* or *UAS-CG16752-IR1* alone. As controls that do not show post-mating behaviors, wild-type females mated to *SP* null mutant males (Liu and Kubli, 2003), are used, as well as virgin females. In the initial mating assays with virgin females, all genotypes are equally receptive (Fig. 1b), indicating that *CG16752* knock-down does not affect the mating receptivity of virgin females. In contrast, mated *CG16752* RNAi females lay dramatically fewer eggs than the negative controls (Fig. 1c), and unlike these controls, they remate at high frequency (Fig. 1d). In both aspects, mated *CG16752* RNAi females are indistinguishable both from wild-type virgins and from wild-type females previously mated to *SP* null males (Figs. 1c, d).

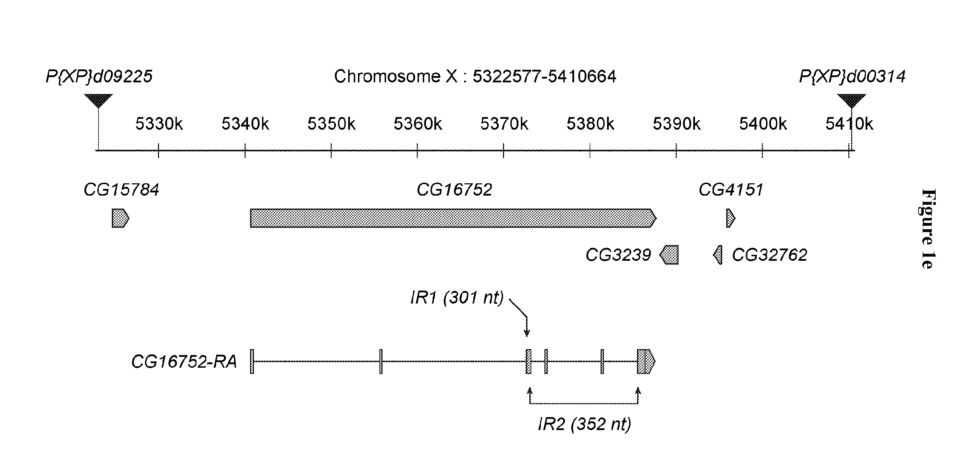

To control for potential off-targeting effects of the initial RNAi transgene, a second independent line is generated, *UAS-CG16752-IR2*, that targets a different region of the gene (Fig. 1e). In all three assays, this new RNAi line gives results indistinguishable from those obtained with the original line from the genome-wide library (Figs. 1b-d). Also, a molecularly-defined deficiency (Parks et al., 2004), *Df(1)Exel6234*, is identified that removes 88 kb from the chromosomal region 4F10-5A2, including *CG16752* and 4 other annotated genes (Fig. 1e). The molecular breakpoints of this deficiency are identified, and it is confirmed that it deletes the *CG16752* gene, and found that females homozygous for this deficiency are fully viable and have no obvious defects in the gross anatomy of their nervous system or reproductive organs. When tested in parallel in the same series of receptivity and egg-laying assays, *Df(1)Exel6234* homozygous females show post-mating defects indistinguishable from those obtained by RNAi knock-down of *CG16752* (Figs. 1b-d).

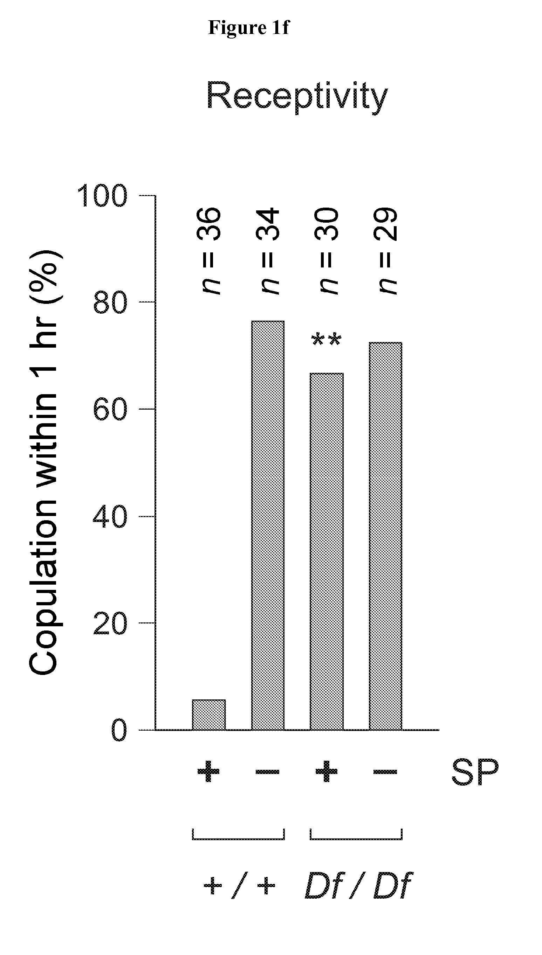

By mating *CG16752* RNAi or deficiency females to a *dj-GFP* to visualize sperm, it is confirmed that sperm are transferred and stored normally in these animals. It is thus postulated that the failure of these females to switch to post-mating behaviors can be due to a lack of sensitivity to SP. To test this directly, SP is injected into the haemolymph of *Df(1)Exel6234* homozygous virgins and wild-type controls, and then these virgins are paired 5 h later with naive wild-type males. As expected, wild-type virgins injected with SP are unreceptive to these males, whereas those injected with buffer alone are as receptive as uninjected virgins (Fig. 1f). In contrast, *Df(1)Exel6234* virgins remaine receptive even following injection with SP (Fig. 1f). Taken together, these genetic data demonstrate that the GPCR encoded by *CG16752* is required for the post-mating switch in female reproductive behavior triggered by SP.

**Fig. 1** shows the following protocols and results:
**(a)** Protocol for behavioral experiments. The *elav-GAL4* driver line additionally carried *UAS-Dcr-2* to enhance RNAi potency (Dietzl et al., 2007) (genotypes 4, 5 and 7).
**(b)** Receptivity of virgin females of the indicated genotypes, scored as the percentage of females that copulated within 1 h. *P* > 0.01 for all comparisons against +/+ (genotype 1), χ²-test with Bonferroni correction.
**(c)** Number of eggs laid per female during the 48 h immediately after copulation. Data are mean ± s.e.m. ** *P* < 0.001, Tukey's multiple comparison test.
**(d)** Re-mating frequency for females tested 48 h after the initial mating. * *P* < 0.01,
   ** *P* < 0.001 for all comparisons against +/+ (genotype 1), χ²-test with Bonferroni correction.
**(e)** Organization of the *CG16752* genomic region. The region deleted in *Df(1)Exel6234* is shown. This deficiency derives from a precise deletion of interval between P-element insertions *P{XP}d09225* and *P{XP}d00314* (Parks et al., 2004), and includes the 4 annotated genes indicated. *UAS-CG16752-IR1* targets nucleotides 552-582 of the *CG16752-RA* transcript, and *UAS-CG16752-IR2* targets nucleotides 869-1220 (spanning 4 exons).
**(f)** Receptivity of wild-type or *Df(1)Exel6234* homozygous virgin females assayed 5 h after injection with either 12pmol SP (+) or Ringer's solution alone (-).
   ** *P* < 0.001 for comparison to +/+, χ²-test.

### Example 2

### CG16752 encodes a specific sex peptide receptor

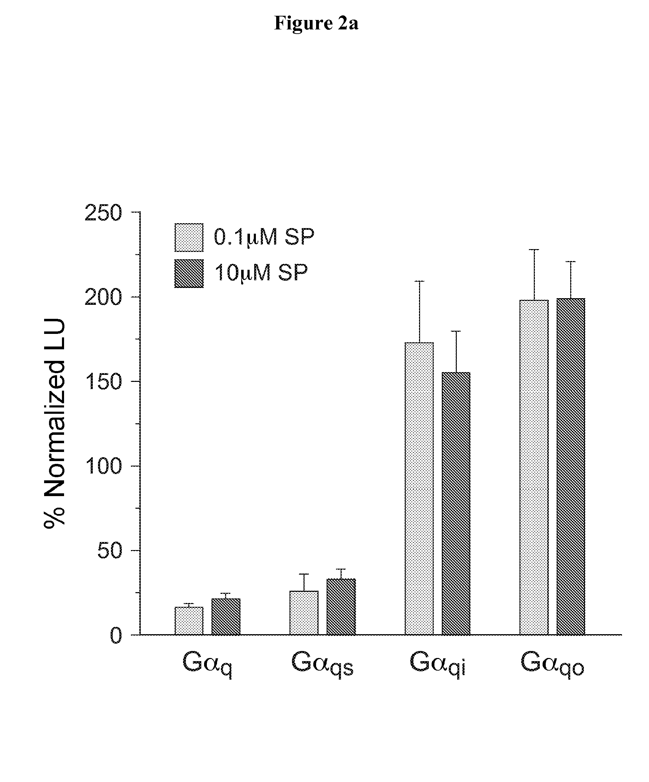

To test whether *CG16752* might encode the SP receptor itself, a *CG16752* cDNA is expressed in mammalian CHO cells together with the Ca²⁺ reporter aequorin. In this assay, ligand-mediated GPCR activation triggers a luminescent flash via the Gα_{q/11}-dependent Ca²⁺ pathway (Le Poul et al., 2002). Only a very weak response to SP is detected in these cells, even at concentrations as high as 10µM (Fig. 2a). It has been suggested that SP responses might involve the cAMP rather than the Ca²⁺ pathway (Harshman et al., 1999; and Chapman et al., 1996) and so it is suspected that the initial failure to detect a strong SP response might be because CG16752 couples to G proteins other than Gα_{q/11}. Accordingly, these cells are cotransfected with constructs encoding one of three different chimeric G proteins (Gα_{qs}, Gα_{qi} or Gα_{qo}) designed to divert Gαₛ-, Gαᵢ- or Gαₒ-dependent signals, respectively, from the cAMP pathway into the Ca²⁺ pathway (Conklin et al., 1993 and 1996). Expression of Gα_{q¡} or Gα_{qo}, but not Gα_{qs}, results in robust Ca²⁺ responses to SP (Fig. 2a).

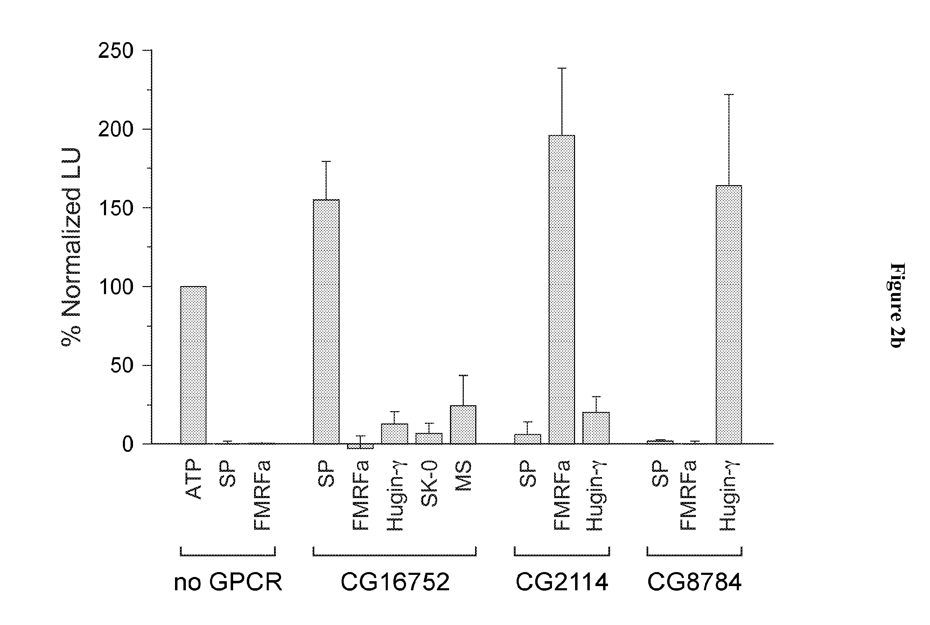

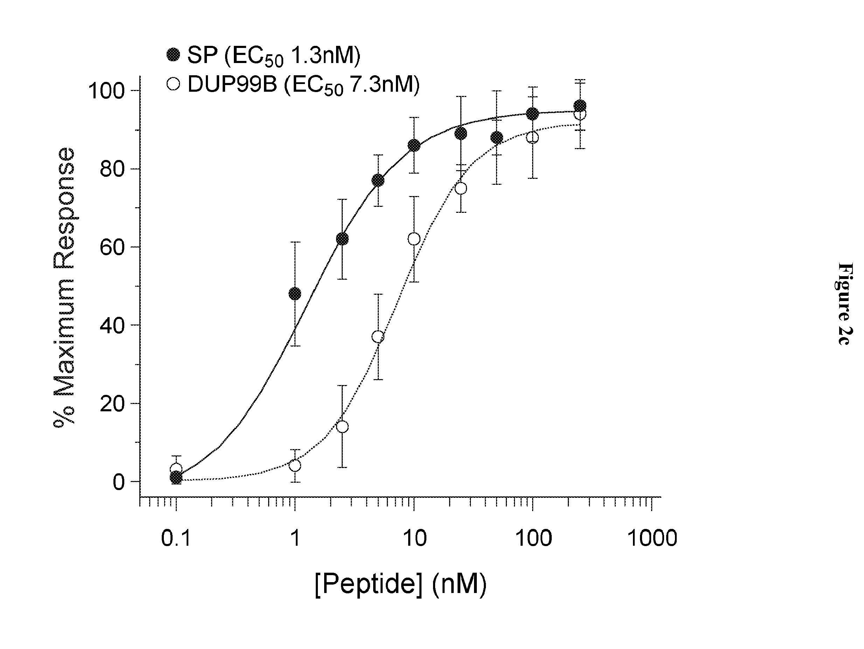

The response to SP is highly specific, as comparable levels of activation to any of 8 other *Drosophila* peptides are not detected, even at 10µM (Fig. 2b; see Methods). Amongst the closest relatives of *CG16752* in *Drosophila* are *CG2114* and *CG8784,* which encode receptors for FMRFamides and hugin-γ, respectively (Meeusen et al., 2002; Cazzamali and Grimmelikhuijzen, 2002; Rosenkilde et al., 2003; and Park et al., 2002). Neither of these peptides activates CG16752, and conversely, expression of CG2114 or CG8784 in CHO cells confer sensitivity to their respective ligands, but not to SP (Fig. 2b). In a dose-response assay, it is determined that SP activates CG16752 with an EC₅₀ of 1.3nM (Fig. 2c). The closely related peptide, DUP99B, which can induce the same post-mating responses as SP (Saudan et al., 2002) activates CG16752 with an EC₅₀ of 7.3nM. Thus, both SP and DUP99B specifically activate CG16752 at physiological concentrations, and in the low nanomolar range typical for such peptide-GPCR interactions (Park et al., 2002). It is thus concluded that *CG16752* encodes a functional receptor for SP that couples to Ga_{qi} and/or Gα_{qo} to regulate cAMP levels. Henceforth, this receptor is referred to as the sex peptide receptor, SPR.

**Fig. 2** shows the following results:
**(a)** Luminescence responses of CHO cells expressing CG16752, aequorin and either one of the three chimeric G proteins (Gα_{qs}, Gα_{qi}, or Gα_{qo}) or no additional G protein (endogenous Gα_{q}). Cells are treated with either 0.1µM or 10µM SP, and responses normalized against the response to 25µM ATP, which activates Ca²⁺ signaling via the endogenous P₂Y₂ receptor (100%). Data are mean ± s.d. (n = 4-8).
**(b)** Luminescene responses of CHO cells expressing the indicated GPCR and aequorin upon exposure to various peptide ligands (10µM), normalized against responses to 25µM ATP (100%). Cells expressing CG16752 or no additional GPCR are co-transfected with Ga_{qi}. Data are mean ± s.d. (n = 5-8).
**(c)** Dose-response curves of CHO cells expressing CG16752, aequorin and Gα_{qi} treated with SP or DUP99B. Each data point is mean ± s.d. (n = 8).

### Example 3

### SPR is expressed in the nervous system and female reproductive tract

To define the primary cellular targets of SP, antisera against an N-terminal region of SPR are generated. These antisera reveal high levels of SPR expression in the female reproductive organs, in particular in the spermathecae, the primary sites for long-term sperm storage, (Bloch Qazi et al., 2003) and the lower oviduct. Staining with the anti-SPR antisera is restricted to the cell membrane and is absent in *Df(1)Exel6234* homozygous females, confirming the specificity of these antisera. SPR cannot be detected in the male reproductive organs.
SP is also thought to pass into the haemolymph and ultimately act directly on targets in the central nervous system (CNS) (Kubli, 2003; and Peng et al., 2005). Indeed, staining the adult female CNS with anti-SPR reveals broad expression on the surface regions of both the brain and ventral nerve cord (VNC). Expression is most prominent in ventral regions of the suboesophageal ganglion (SOG), the cervical connective (cc), and many nerve roots in the brain and VNC. The restricted staining on the surface of the CNS is not an artefact due to poor antibody penetration, as SPR can be reliably detected in central brain regions upon ectopic expression of a *UAS-SPR* transgene in selected brain regions. CNS staining is completely absent in *SPR* null mutants, and greatly reduced in the *elav-GAL4 UAS-SPR-IR1* females. In contrast to receptors for neuropeptides that are released within the CNS, the superficial localization of SPR is consistent with its role in detecting a ligand that circulates in the haemolymph and reaches central targets by crossing the blood-brain barrier. A very similar CNS staining is observed in males, suggesting that SPR may have additional functions unrelated to its role in female reproductive behavior. Abnormalities in the mating behavior of *SPR* null males cannot be detected. SPR cannot be detected in embryos or larvae, nor in any other adult tissues. Overall, the distribution of SPR concords remarkably well with the reported binding sites of radiolabelled SP applied to whole-female tissue sections *in vitro* (Ottiger et al., 2000).
Post-mating responses can be induced in virgin females by blocking synaptic transmission of neurons that express the sex-specific P1 transcripts of the *fru* gene, leading to the speculation that SP might exert its effects in part by modulating the activity of these *fru* neurons (Kvitsiani and Dickson, 2006). Consistent with this hypothesis, it is found that some of the central neurons that express SPR are also positive for *fru*, as reported by the *fru*^{GAL4} driver. In particular, SPR appears to be expressed in many *fru*^{GAL4}-positive neurons in the SOG and throughout the VNC.

### Example 4

### SPR function is required in fru neurons

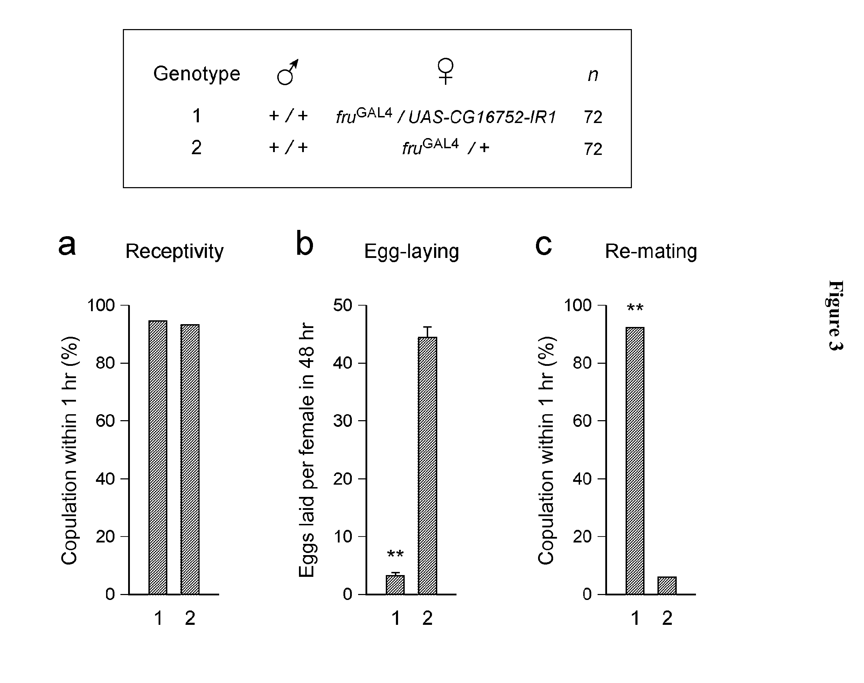

To test whether SPR function is required in *fru* neurons to trigger a post-mating response, the *fru*^{GAL4} driver and *UAS-SPR-IR1* are used to specifically knock-down SPR in these cells. These females show normal receptivity as virgins, but after mating they lay only few eggs and re-mate at high frequency (Fig. 3). In both respects, these females are indistinguishable from wild-type virgins, or mated females from pairings involving either a *SP* null male or a *SPR* null female (Figs. 1b-d). Thus, interference with SPR function exclusively in *fru* neurons leads to a complete block in the post-mating behavioral switch.

### Fig. 3 shows the following results:

Receptivity **(a),** egg-laying **(b),** and re-mating **(c)** assays for *fru*^{GAL4} *UAS-Dcr-2* / *UAS-SPR-IR1* and control females assayed according to the protocol of Fig. 1a. Data in **(b)** are mean ± s.e.m. ** *P* < 0.001 compared to control females, Mann Whitney test **(b)** and χ²-test **(c).**

### Example 5

### Structural and functional conservation of insect SPRs

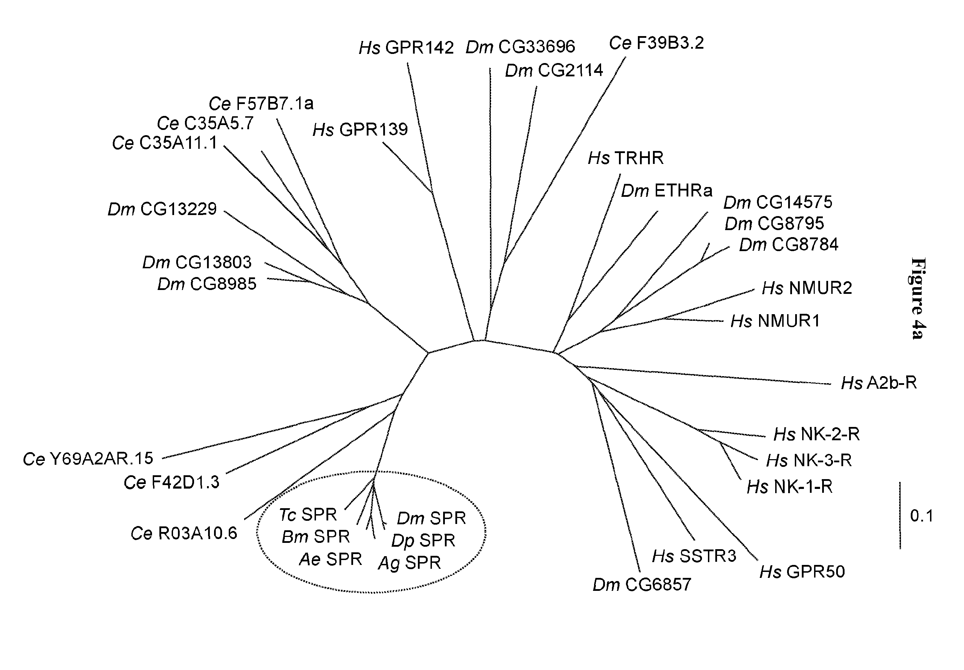

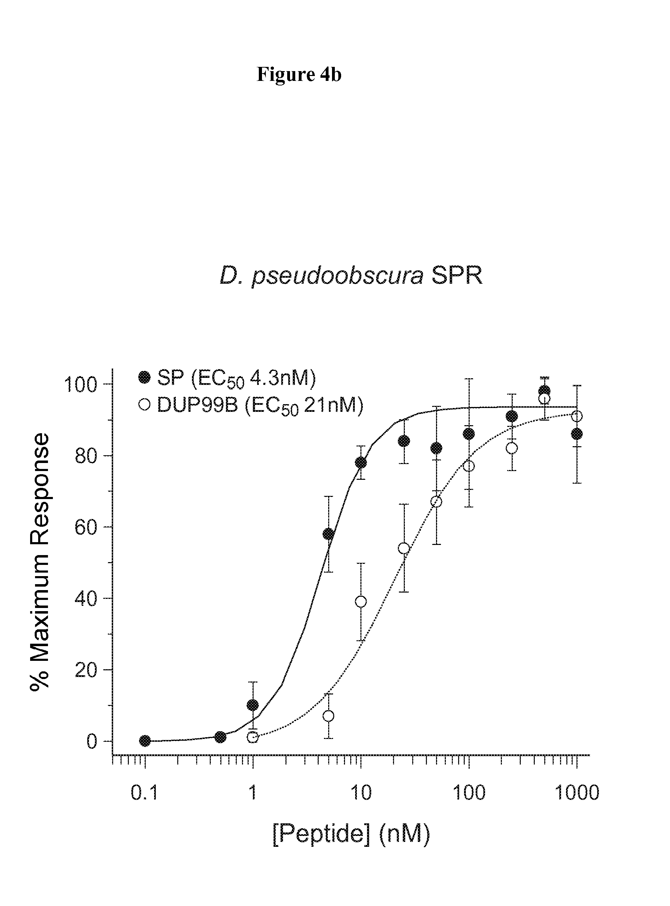

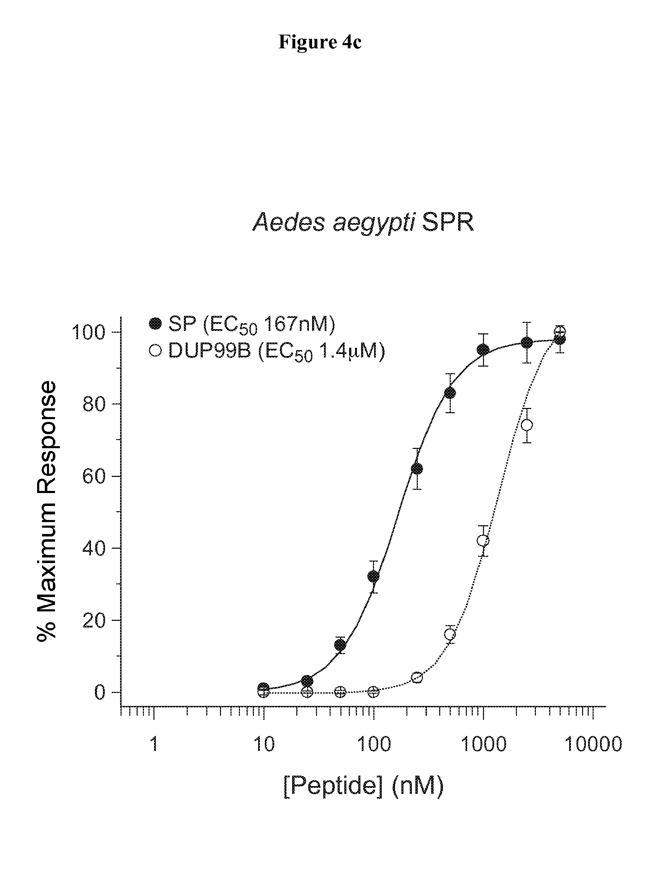

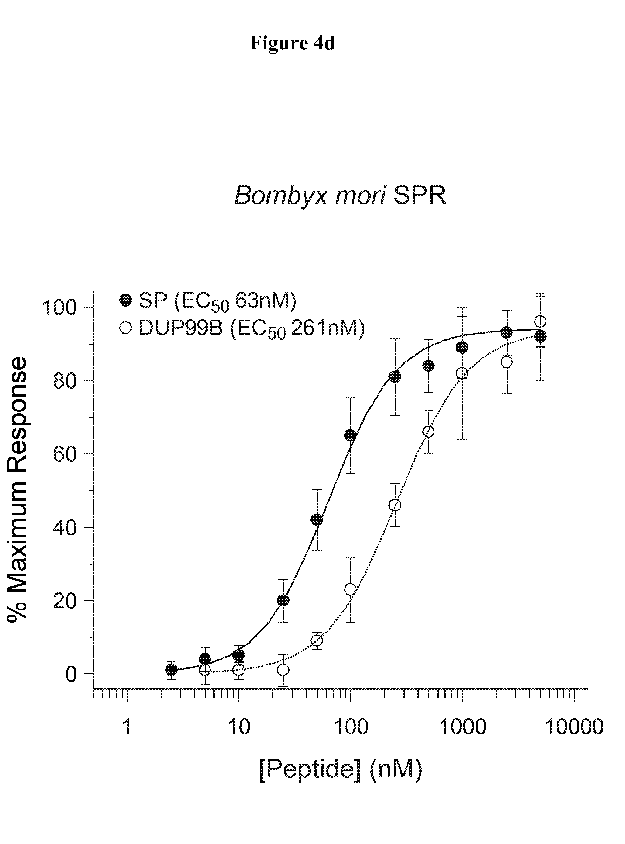

The *SPR* gene has been highly conserved during the course of insect evolution, as putative orthologues can be readily identified in most sequenced insect genomes, including *D*. *pseudoobscura*, the mosquitos *Aedes aegypti* and *Anopheles gambiae,* the moth *Bombyx mori*, and the beetle *Tribolium castaneum* (Fig. 4). Putative vertebrate orthologues are less apparent (Fig. 4a). To test for functional conservation of the insect SPR family, *SPR* cDNAs from each of these 5 insect species are isolated and tested for responses to *D*. *melanogaster* SP in the CHO cell assay. SP is a potent activator of the *D*. *pseudoobscura*, *A*. *aegypti*, and *B*. *mori* receptors, with EC₅₀s of 4.3nM, 167nM and 63nM respectively (Figs. 4b-d). These receptors also respond to DUP99B with lower sensitivity (Figs. 4b-d), but not to any of the other 8 control peptides, including FMRFamides and hugin-γ. The functional conservation of *SPR* genes from *Drosophila*, *Aedes*, and *Bombyx*, together with the observation that *D*. *melanogaster* SP can induce post-mating responses in the moth *Helicoverpa armigera* (Fan et al., 1999 and 2000), show that the family of receptors identified in the present invention mediate post-mating changes in female reproductive behavior and physiology across much of the insect order. The cDNA sequences of *Aedes aegypti, Anopheles gambiae*, *Bombyx mori* and *Tribolium* castaneum are shown SEQ ID NO:3, 5, 7 and 9, respectively.
SPR sequences from the additional insect species including human body louse (*Pediculus humanus corporis*) and southern house mosquito (*Culex pipiens quinquefasciatus*) are predicted by performing blast search (TBLASTN; Altschul et al., 1997) on genome assemblies of each species and predicting gene structures (e.g. by Genscan). The predicted partial cDNA sequences of *Pediculus humanus* corporis and *Culex pipiens* quinquefasciatus are shown SEQ ID NO:11 and 13, respectively.
The above sequences can be confirmed to encode functional SPRs by assays corresponding to the ones described above for the SPRs of *Aedes aegypti, Anopheles gambiae*, *Bombyx mori* and *Tribolium castaneum.*

**Fig. 4 (a)** shows the phylogenetic tree of predicted insect SPRs and related *Drosophila*, *C*. *elegans* and human GPCRs. Scale bar: 0.1 amino acid replacements per site.
**Fig. 4** **(b-d)** shows the dose-response curves of CHO cells expressing various insect SPRs, aequorin, and Gα_{qi} treated with *D*. *melanogaster* SP or DUP99B. Each data point is mean ± s.d. (*n* = 6).

### Example 6

### High throughput assay for identifying SPR modulators

All steps of the high-throughput screen are performed using a robotic screening system and/or semi-automated workstations. CHO-K1 cells stably co-expressing both recombinant SPR and Gα₁₆ promiscuous G-protein, as described inExample 2, are grown to near-confluence in appropriate media supplemented with 10% FBS and appropriate selection agents (e.g., 0.2 mg/mL zeocin, and 0.5 mg/mL G418). One day prior to assay, cells are plated into black/clear bottom 384-well tissue culture-treated microplates (e.g., Becton Dickenson #353962) at 10,000-20,000 cells/well in complete media. On the morning of the assay, growth media is removed and 25 µL of Fluo4-NW (Molecular Probes) in assay buffer (HBSS supplemented with 1 mM CaCl₂, 1 mM MgCl₂, and 1 mM probenecid to inhibit efflux pumps) is added to all wells. Plates are returned to 37°C for 60 minutes, and 5 µL of appropriately diluted compound (typically sufficient to achieve a final assay concentration of 3-10 µM) is added, mixed, and incubated for 15 minutes. Assay plates are then transported to the FLIPR plate reader where ligand (the endogonous SP or an SP sufficiently similar to engage and induce signal transduction) is added (typically to a concentration between EC₅₀ and EC₈₀, depending on the signal window of the assay). Intracellular calcium flux is measured at 1-second intervals for 1-3 minutes as fluorescence intensity. Antagonism is scored as a percent of ligand-stimulated control wells receiving DMSO without test compound. Background response is determined in wells that receive buffer without ligand. The measured fluorescent signal response (Max or Max-Min within specified time frames) is calculated following continuous measurement for several minutes, and the percent inhibition (or stimulation) compared to SP alone is calculated. Compounds producing significant inhibition (≥ 3 standard deviations below control values) are identified as hits to be followed up with confirmation testing and concentration-response testing to quantitate their potency. To identify agonists of SPR, the above protocol is modified to add the test compound on the FLIPR device and immediately measure the fluorescent signal, without adding the natural ligand.

An alternative screening assay is established using the same cell line transfected stably with apoaequorin, with the difference that coelenterazine loading replaces the Fluo4 fluorescent dye, and a luminescence signal is measured following stimulation with ligand. In this case, area under the luminescence signal curve acquired in 1 minute is used to calculate the degree of ligand-induced response.

### References

al-Obeidi F, Hruby VJ, Sawyer TK. (1998), Mol Biotechnol. Jun;9(3):205-2.
Altschul, S. F., W. Gish, W. Miller et al., J Mol Biol. 215 (3), 403 (1990).
Altschul, et al., (1997), Nuc. Acids Res. 25:3389-3402.
Angers, S., et al., Proc.Natl.Acad.Sci. USA 97.7 (2000): 3684-89.
Atkinson et al., (2001), Annu. Rev. Entomol. 46:31746.
Ausubel et al., (1989), Current Protocols in Molecular Biology (Green Publishing Associates and Wiley Interscience, N.Y.
Aza-Blanc, P., Lin, H.Y., Ruiz i Altaba, A., et al., Development 127 (19), 4293 (2000).
Backhaus, B., Sulkowski, E., and Schlote, F.W., Dros. Inf. Serv. 60, 210 (1984).
Berger, J., et al., Gene 66.1 (1988): 1-10
Bloch Qazi, M.C., Heifetz, Y., and Wolfner, M.F., Dev Biol 256 (2), 195 (2003).
Brini, M., et al., J.Biol.Chem. 270.17 (1995): 9896-903.
Bronstein, I., et al., Clin.Chem. 42.9 (1996): 1542-46.
Bursavich, Matthew G., Daniel H. Rich, (2002). J. Med. Chem., 45 (3), 541-558.
Catteruccia et al., (2005), Nat Biotechnol. 2005 Nov; 23(11):1414-7.
Cazzamali, G., and Grimmelikhuijzen, C. J., Proc Natl Acad Sci USA 99 (19), 12073 (2002).
Chapman, T., Choffat, Y., Lucas, W.E., et al., J Insect Physiol 42, 1007 (1996).
Chapman, T., J. Bangham, G. Vinti et al., Proc Natl Acad Sci USA 100 (17), 9923 (2003).
Chen, P.S., E. Stumm-Zollinger, T. Aigaki et al., Cell 54 (3), 291 (1988).
Chenchik A, Diachenko L, Moqadam F, Tarabykin V, Lukyanov S, Siebert PD, (1996) Biotechniques, Sep;21(3):526-34.
Chin, J., et al., " Assay.Drug Dev.Technol. 1.6 (2003): 777-87.
Conklin, B.R., Farfel, Z., Lustig, K.D., et al., Nature 363, 274 (1993).
Conklin, B.R., Herzmark, P., Ishida, S., et al., Mol Pharmacol 50 (4), 885 (1996).
Controlled Delivery of Crop-Protection Agents, Taylor and Francis, New York, (1990), Editor R. M. Wilkins, chapters 3 and 9.
Coward, P., et al., Anal. Biochem. 270.2 (1999): 242-48.
Cubitt, A.B., et al., Trends Biochem.Sci. 20.11 (1995): 448-55.
de Wet, J. R., et al., Mol. Cell Biol. 7.2 (1987): 725-37.
Dietzl, G., Chen, D., Schnorrer, F., et al., Nature 448 (7150), 151 (2007).
Eddy, S.R., Bioinformatics 14 (9), 755 (1998).
Edgar, R.C., BMC Bioinformatics 5, 113 (2004).
Fan, Y., Rafaeli, A., Gileadi, C., et al., J Insect Physiol 45 (2), 127 (1999).
Fan, Y., Rafaeli, A., Moshitzky, P., et al., (2000), Insect Biochem Mol Biol. Aug-Sep; 30(8-9):805-12.
Felsenstein, J., PHYLIP (Phylogeny Inference Package) version 3.6. Distributed by the author. (2005).
Gante Joachim, (1994), Angew. Chem., Int. Ed. Engl., 33(17), 1699-720.
Garippa, et al., (2006), Methods Enzymol, 414: 99-120.
Gee, K.R., et al., Cell Calcium 27.2 (2000): 97-106.
Gillott, C., Annu Rev Entomol 48, 163 (2003).
Golebiowski, A., Klopfenstein, S.R., Portlock, D.E., Current Opinion in Drug Discovery & Development (2001), 4(4), 428-434.
Gopalakrishnan, S.M., et al., Anal. Biochem. 321.2 (2003): 192-201.
Granas, C., et al., Comb. Chem. High Throughput. Screen. 8.4 (2005): 301-09.
Groth, A. C., Fish, M., Nusse, R., et al., Genetics 166 (4), 1775 (2004).
Handler, (2001), Insect Biochem Mol Biol. 31(2):111-28.
Harshman, LG, Loeb AM, Johnson BA., J Insect Physiol 45 (6), 571 (1999).
Hepler, J.R., and Gilman, A.G., Trends Biochem. Sci. 17.10 (1992): 383-87.
Insect Suppression with Controlled Release Pheromone Systems, Vol. I and II, CRC Press, Boca Raton, Fla. (1982).
Jain, V.K., and Magrath, I.T., Anal. Biochem. 199.1 (1991): 119-24.
Jeanmougin, F., Thompson, J. D., Gouy, M., et al., Trends Biochem Sci 23 (10), 403 (1998).
Jerman, J.C., et al., Eur. J. Pharmacol. 414.1 (2001): 23-30.
Johnson, E.C., Bohn, L.M., Barak, L.S., et al., J Biol Chem 278 (52), 52172 (2003).
Kim, Y-J., Spalovská-Valachová, I., Cho, K. H., et al., Proc Natl Acad Sci USA 101 (17), 6704 (2004).
Klowden M. J. and Lea A. 0. (1978). Am. J. Trop. Med. Hyg. 27, 827-83 1.
Kornienko, O., et al., J. Biomol. Screen. 9.3 (2004): 186-95.
Krafsur, E.S., (1998), Journal of Agricultural Entomology 15, 303-317.
Kubli, E., Cell Mol Life Sci 60 (8), 1689 (2003).
Kvitsiani, D., and Dickson, B.J., Curr Biol 16 (10), R355 (2006).
Labrousse, H., et al., J. Immunol. Methods 48.2 (1982): 133-47.
Le Poul, E., et al., J. Biomol. Screen. 7.1 (2002): 57-65.
Liu, H. and Kubli, E., Proc Natl Acad Sci USA 100 (17), 9929 (2003).
Luo, L., Liao, Y.J., Jan, L.Y., et al., Genes Dev. 8 (15), 1787 (1994).
Meeusen, T., Mertens, I., Clynen, E., et al., Proc Natl Acad Sci USA 99 (24), 15363 (2002)
Michelini, E., et al., Anal. Chem. 76.23 (2004): 7069-76.
Moore, Graham J., (1994), Trends in Pharmacological Sciences, 15, 124-129.
Moore, J.T., Davis, S.T., and Dev, I. K., Anal. Biochem. May 1; 247(2), (1997): 203-09.
Naylor, L.H., Biochem. Pharmacol. 58.5 (1999): 749-57.
O'Neill, E. M., I. Rebay, R. Tjian et al., Cell 78 (1), 137 (1994).
Ottiger, M., Soller, M., Stocker, R.F., et al., J Neurobiol 44 (1), 57 (2000).
Park, Y., Kim, Y-J., and Adams, M.E., Proc Natl Acad Sci USA 99 (17), 11423 (2002).
Parks, A.L., Cook, K.R., Belvin, M., et al., Nat Genet 36 (3), 288 (2004).
Peng, J., Chen, S., Busser, S., et al., Curr Biol 15 (3), 207 (2005).
Pfleger, K.D., and Eidne, K.A., Pituitary. 6.3 (2003): 141-51.
Rosenkilde, C., Cazzamali, G, Williamson, M., et al., Biochem Biophys Res Commun 309 (2), 485 (2003).
Sambrook et al., (1989), Molecular Cloning, A Laboratory Manual, Cold Springs Harbor Press, N.Y.
Saudan, P., Hauck, K., Soller, M., et al., Eur J Biochem 269 (3), 989 (2002).
Schmidt, T., Choffat, Y., Klauser, S., et al., J Insect Physiol 39 (5), 361 (1993).
Schnitzer, R., and Sommergruber, W. (2006) "The use of genetically engineered cell-based assays in in-vitro drug discovery." in "New Approaches to the Generation and Evaluation of Chemical Diversity", in "Exploiting Chemical Diversity for Drug Discovery" Paul A. Bartlett and Michael Entzeroth, RSC Publishing, Royal Society of Chemistry, 2006.
Stables, J., et al., Anal. Biochem. 252.1 (1997): 115-26.
Stockinger, P., Kvitsiani, D., Rotkopf, S., et al., Cell 121 (5), 795 (2005).
Stratowa, C., and Audette, M., Immunobiology 193.2-4 (1995): 293-304.
Suto, C., and Ignar, D., Journal of Biomolecular Screening 2 (1997): 7-9.
Tsien, R.Y., Annu. Rev. Biochem. 67 (1998): 509-44.
Vernon, W.I. and Printen, J.A., Biotechniques 33 (4), 730 (2002).
Vilardaga, J.P., et al., Nat. Biotechnol. 21.7 (2003): 807-12.
Voraberger, G., Schaefer, R., and Stratowa, C., J. Immunol. 147.8 (1991): 2777-86.
Vrecl, M., et al., J. Biomol. Screen. 9.4 (2004): 322-33.

## Claims

1. A method for controlling the population of an insect species of interest whose females naturally undergo a switch to post-mating behavior in response to a sex peptide, which is the analogue in said insect species of the *Drosophila* sex peptide (SP) with the sequence shown in SEQ ID NO:18, and which is produced in the male and transferred to the female during mating, said method comprising applying to females of said species of interest one or more compounds that modulate the activity of the receptor for said sex peptide, thereby affecting said switch to said post-mating behavior, wherein said receptor, in said insect species of interest, is the orthologue of the *Drosophila* sex peptide receptor (SPR) with the amino acid sequence shown in SEQ ID NO:2.

2. The method of claim 1, wherein said compound is an inhibitor of the activity of said SPR.

3. The method of claim 1, wherein said insect species is selected from *Aedes, Anopheles, Culex, Nilaparvata lugens, Trialeurodes vaporariorum, Musca domestica, Glossina fuscipes fuscipes, Spodoptera litura, Agrotis ipsilon, Helicoverpa armigera, Plutella xylostella, Pectinophora gossypiella, Curculionidae, Thrips, Blattella germanica, Periplaneta Americana, Acridiae, Schistocerca gregaria, Pediculus humanus corporis, Reticulitermes speratus*.

4. The isolated SPR of *Drosophila melanogaster* with the amino acid sequence of SEQ ID NO:2, or a functional equivalent or a biologically active fragment or derivative thereof.

5. An isolated insect SPR that is an orthologue of the *Drosophila* SPR with the amino acid sequence of SEQ ID NO:2, wherein activation of said SPR orthologue, when naturally occurring, in response to the sex peptide results in a switch to post-mating behavior in said female insect, or a functional equivalent or a biologically active fragment or derivative of said SPR.

6. An isolated SPR of claim 5, wherein said orthologue is selected from an SPR *of Aedes aegypti, Anopheles gambiae, Bombyx mori, Tribolium castaneum,* which has the amino acid sequence shown SEQ ID NO:4, 6, 8 or 10, respectively, or from an SPR of *Pediculus humanus corporis or Culex pipiens quinquefasciatus* which has a partial amino acid sequence as shown in SEQ ID NO:12 or 14, respectively.

7. An isolated nucleic acid molecule which encodes the SPR of *Drosophila melanogaster*, wherein said nucleic acid molecule has the sequence shown in SEQ ID NO:1, or a or a functional equivalent or a biologically active fragment or derivative thereof.

8. An isolated nucleic acid molecule which encodes an insect SPR that is an orthologue of the *Drosophila* SPR, wherein said nucleic acid molecule
a) has a sequence selected from SEQ ID NO:3, 5, 7, 9 or 15 or a partial sequence shown in SEQ ID NO:11 or 13;
b) encodes an SPR with an amino acid sequence selected from any of SEQ ID NO:4, 6, 8, 10 or 16 or an SPR containing a partial amino acid sequence shown in SEQ ID NO:12 or 14;
c) hybridizes to the complement of a nucleic acid molecule defined in a) or b) under stringent conditions;
d) hybridizes to the complement of a nucleic acid molecule defined in a) or b) under highly stringent conditions;
e) is any nucleic acid molecule that encodes an SPR polypeptide that
i) comprises a protein sequence or a partial protein sequence that is at least 65% identical overall to a sequence selected from any of SEQ ID NO:2, 4, 6, 8, 10, 12, 14, 16;
ii) is predicted, based on its amino acid sequence, to be a GPCR and has an amino acid sequence that is more closely related to the *Drosophila* SPR of SEQ ID NO:2 than any other GPCR encoded in the *Drosophila* genome;
f) is the complement of any of the SPR sequences defined in a) to e).

9. A method of identifying a nucleic acid molecule encoding the SPR of an insect species of interest, said method comprising
a) amplifying by polymerase chain reaction genomic DNA or cDNA from said insect species of interest with a forward primer comprising a degenerate oligonucleotide encoding at least six amino acids of a known SPR, taken from the *Drosophila* SPR encoding sequence shown in SEQ ID NO:1, or from an SPR coding sequence selected from SEQ ID NO:3, 5, 7, 9, 11, 13 or 15, respectively, or its complement, and a reverse primer comprising a degenerate oligonucleotide encoding at least six amino acids of the known SPR, taken from SEQ ID NO:1 or SEQ ID NO:3, 5, 7, 9, 11, 13 or 15 respectively, or its complement and
b) detecting a PCR amplification product, thereby identifying a nucleic acid encoding the SPR polypeptide of the insect species of interest.

10. A method for identifying a compound that modulates the activity of an SPR of an insect species of interest for use in the method of claim 1, said method comprising determining whether a test compound
a) binds to and activates said SPR, and/or
b) inhibits the generation of intracellular signals generated upon activation of said SPR in response to the SP, by
i. binding to the receptor binding site of the sex peptide, or by
ii. allosteric hindrance, or
c) inhibits the interaction of said SPR with the sex peptide, or
d) acts intracellularly on a signal in the signal transduction pathway that is triggered by activation of said SPR in response to an SP; or
e) inhibits the interaction of said SPR and one or more G-protein(s), wherein cells expressing on their surface said SPR, either endogenously or upon transfection with a nucleic acid molecule encoding said SPR, or a functional equivalent or a biologically active fragment or derivative of said SPR, or a membrane fraction of such cells, are incubated with said test compound and said test compound's ability to modulate SPR activity as defined in a) to e) is determined.

11. The method of claim 10, wherein said cells are transfected with a DNA molecule encoding a G protein alpha subunit selected from naturally occurring or chimeric Gs, Gi or Gq alpha subunits.

12. The method of claim 10, wherein said cells are co-transfected with two separate DNA molecules, one of which encodes said SPR and the other one encodes said G protein alpha subunit, wherein said DNA molecules are present on two plasmids or one plasmid carrying both DNA sequences.

13. The method of claim 10, wherein said cells are transfected with a DNA molecule encoding a fusion protein comprising the SPR and a G protein alpha subunit selected from naturally occurring or chimeric Gs, Gi or Gq alpha subunits.

14. The method of claim 10, wherein said test compound is tested for its ability to inhibit binding of the endogenous SP to said SPR.

15. The method of claim 10, wherein said test compound's ability to modulate SPR activity is determined by measuring the change in intracellular calcium concentration.

16. The method of claim 10, wherein said test compound is a peptidomimetic that is based on the SP that naturally binds to said SPR, or a functional Sfragment or derivative of said SP.

17. The method of claim 10, comprising, in addition, the step of applying the identified compound to females of one or more insect species of interest, determining the compound's abililty to affect the post-mating behavior of said females and selecting compounds with such ability.

18. A composition for controlling the population an insect species of interest, wherein said composition contains, as the active ingredient(s), one or more compounds that modulate the activity of the sex peptide receptor that is expressed by females of said insect species of interest.

19. An antibody that binds to an SPR of claim 4, 5 or 6 or to a functional equivalent or a biologically active fragment or derivative of said SPR.

20. An oligodeoxyribonucleotide molecule that inhibits expression of an SPR molecule, selected from antisense molecules or from DNA molecules encoding an siRNA.

21. A transgenic plant, containing integrated in its genome, an oligodeoxyribonucleotide molecule of claim 20.


SEQUENCE LISTING

<110>  Boehringer Ingelheim International GmbH

<120>  Method for controlling insect populations

<130>  14_0090

<160>  39

<170>  PatentIn version 3.3

<210>  1
<211>  1308
<212>  DNA
<213>  Drosophila melanogaster


<220>
<221>  CDS
<222>  (1)..(1308)

<400>  1
atg gac aac tat acg gac gta ctg tac cag tac cgc ttg gca ccg tcc      48
Met Asp Asn Tyr Thr Asp Val Leu Tyr Gln Tyr Arg Leu Ala Pro Ser
1               5                   10                  15

gcc agc ccg gaa atg gaa atg gaa ctg gcc gat ccg cgt cag atg gtc      96
Ala Ser Pro Glu Met Glu Met Glu Leu Ala Asp Pro Arg Gln Met Val
            20                  25                  30

cgc gga ttt cat ctg cca acc aac gag tcg cag ttg gag att ccc gac      144
Arg Gly Phe His Leu Pro Thr Asn Glu Ser Gln Leu Glu Ile Pro Asp
        35                  40                  45

tat ggc aac gag agc ctg gac tat ccc aac tac cag cag atg gtc ggc      192
Tyr Gly Asn Glu Ser Leu Asp Tyr Pro Asn Tyr Gln Gln Met Val Gly
    50                  55                  60

gga ccg tgt cgc atg gag gac aac aat atc agc tat tgg aat ctc acc      240
Gly Pro Cys Arg Met Glu Asp Asn Asn Ile Ser Tyr Trp Asn Leu Thr
65                  70                  75                  80

tgc gat tcg cca ctg gag tac gct atg ccg ctc tat ggc tac tgt atg      288
Cys Asp Ser Pro Leu Glu Tyr Ala Met Pro Leu Tyr Gly Tyr Cys Met
                85                  90                  95

ccc ttc ctg ctg atc atc acc att atc tcg aac tcc ctg att gtg ctc      336
Pro Phe Leu Leu Ile Ile Thr Ile Ile Ser Asn Ser Leu Ile Val Leu
            100                 105                 110

gtt ttg agc aag aag agc atg gcc acg ccc acc aat ttt gtg cta atg      384
Val Leu Ser Lys Lys Ser Met Ala Thr Pro Thr Asn Phe Val Leu Met
        115                 120                 125

gga atg gct ata tgc gat atg ctg acg gtt ata ttt ccg gca ccc ggt      432
Gly Met Ala Ile Cys Asp Met Leu Thr Val Ile Phe Pro Ala Pro Gly
    130                 135                 140

ctc tgg tat atg tac aca ttc ggc aat cat tat aag ccc ctg cat ccg      480
Leu Trp Tyr Met Tyr Thr Phe Gly Asn His Tyr Lys Pro Leu His Pro
145                 150                 155                 160

gtc tcc atg tgt ctg gcc tac agc att ttc aat gag ata atg cca gcc      528
Val Ser Met Cys Leu Ala Tyr Ser Ile Phe Asn Glu Ile Met Pro Ala
                165                 170                 175

atg tgc cac acc atc tcc gtt tgg cta act ctg gcc ctc gcc gtt caa      576
Met Cys His Thr Ile Ser Val Trp Leu Thr Leu Ala Leu Ala Val Gln
            180                 185                 190

aga tac atc tac gtt tgc cac gcc ccc atg gcc cga acg tgg tgc acg      624

Arg Tyr Ile Tyr Val Cys His Ala Pro Met Ala Arg Thr Trp Cys Thr
        195                 200                 205

atg ccg cgt gtg agg cgg tgc acg gcg tat att gca ttg ctg gcg ttt        672
Met Pro Arg Val Arg Arg Cys Thr Ala Tyr Ile Ala Leu Leu Ala Phe
    210                 215                 220

ctg cac caa ctg ccc aga ttc ttc gac agg acg tac atg ccg ctg gtg        720
Leu His Gln Leu Pro Arg Phe Phe Asp Arg Thr Tyr Met Pro Leu Val
225                 230                 235                 240

atc gag tgg aac ggc agc cca acg gag gtg tgc cac ttg gag acg tcg        768
Ile Glu Trp Asn Gly Ser Pro Thr Glu Val Cys His Leu Glu Thr Ser
                245                 250                 255

atg tgg gtg cac gat tac att gga gtg gac cta tac tac aca agc tac        816
Met Trp Val His Asp Tyr Ile Gly Val Asp Leu Tyr Tyr Thr Ser Tyr
            260                 265                 270

tat ctg ttc cgg gtg ctg ttc gtc cac ctg ctg ccc tgc att atc ctg        864
Tyr Leu Phe Arg Val Leu Phe Val His Leu Leu Pro Cys Ile Ile Leu
        275                 280                 285

gtt acc ctg aac att ctg ctg ttc gcg gcg atg cgg cag gca cag gag        912
Val Thr Leu Asn Ile Leu Leu Phe Ala Ala Met Arg Gln Ala Gln Glu
    290                 295                 300

cgc cga aag ctc ctc ttc cgg gag aac cgg aag aag gag tgc aag aaa        960
Arg Arg Lys Leu Leu Phe Arg Glu Asn Arg Lys Lys Glu Cys Lys Lys
305                 310                 315                 320

ctg agg gag acc aat tgc acc acg ctg atg cta att gtg gtc gtg tcg       1008
Leu Arg Glu Thr Asn Cys Thr Thr Leu Met Leu Ile Val Val Val Ser
                325                 330                 335

gtg ttc ctt cta gcc gag ata ccc att gct gtg gtc act gcg atg cac       1056
Val Phe Leu Leu Ala Glu Ile Pro Ile Ala Val Val Thr Ala Met His
            340                 345                 350

atc gtc agt agc ctg att atc gag ttc ctg gac tat gga ctg gcc aac       1104
Ile Val Ser Ser Leu Ile Ile Glu Phe Leu Asp Tyr Gly Leu Ala Asn
        355                 360                 365

att tgc atc atg ctg acg aac ttc ttc ctg gta ttc agc tat ccg atc       1152
Ile Cys Ile Met Leu Thr Asn Phe Phe Leu Val Phe Ser Tyr Pro Ile
    370                 375                 380

aac ttc ggc att tac tgc ggc atg tcg cgc cag ttt cgg gag act ttc       1200
Asn Phe Gly Ile Tyr Cys Gly Met Ser Arg Gln Phe Arg Glu Thr Phe
385                 390                 395                 400

aag gag ata ttc ctc ggt cgg ctg atg gcc aaa aag gat agc tcc aca       1248
Lys Glu Ile Phe Leu Gly Arg Leu Met Ala Lys Lys Asp Ser Ser Thr
                405                 410                 415

aaa tac tcg atc gtt aat ggc gcc cgc acc tgc acc aac acc aac gag       1296
Lys Tyr Ser Ile Val Asn Gly Ala Arg Thr Cys Thr Asn Thr Asn Glu
            420                 425                 430

acg gtc ctc tag                                                       1308
Thr Val Leu
        435


<210>  2
<211>  435
<212>  PRT
<213>  Drosophila melanogaster

<400>  2

Met Asp Asn Tyr Thr Asp Val Leu Tyr Gln Tyr Arg Leu Ala Pro Ser

1               5                   10                  15

Ala Ser Pro Glu Met Glu Met Glu Leu Ala Asp Pro Arg Gln Met Val
            20                  25                  30

Arg Gly Phe His Leu Pro Thr Asn Glu Ser Gln Leu Glu Ile Pro Asp
        35                  40                  45

Tyr Gly Asn Glu Ser Leu Asp Tyr Pro Asn Tyr Gln Gln Met Val Gly
    50                  55                  60

Gly Pro Cys Arg Met Glu Asp Asn Asn Ile Ser Tyr Trp Asn Leu Thr
65                  70                  75                  80

Cys Asp Ser Pro Leu Glu Tyr Ala Met Pro Leu Tyr Gly Tyr Cys Met
                85                  90                  95

Pro Phe Leu Leu Ile Ile Thr Ile Ile Ser Asn Ser Leu Ile Val Leu
            100                 105                 110

Val Leu Ser Lys Lys Ser Met Ala Thr Pro Thr Asn Phe Val Leu Met
        115                 120                 125

Gly Met Ala Ile Cys Asp Met Leu Thr Val Ile Phe Pro Ala Pro Gly
    130                 135                 140

Leu Trp Tyr Met Tyr Thr Phe Gly Asn His Tyr Lys Pro Leu His Pro
145                 150                 155                 160

Val Ser Met Cys Leu Ala Tyr Ser Ile Phe Asn Glu Ile Met Pro Ala
                165                 170                 175

Met Cys His Thr Ile Ser Val Trp Leu Thr Leu Ala Leu Ala Val Gln
            180                 185                 190

Arg Tyr Ile Tyr Val Cys His Ala Pro Met Ala Arg Thr Trp Cys Thr
        195                 200                 205

Met Pro Arg Val Arg Arg Cys Thr Ala Tyr Ile Ala Leu Leu Ala Phe
    210                 215                 220

Leu His Gln Leu Pro Arg Phe Phe Asp Arg Thr Tyr Met Pro Leu Val
225                 230                 235                 240

Ile Glu Trp Asn Gly Ser Pro Thr Glu Val Cys His Leu Glu Thr Ser
                245                 250                 255

Met Trp Val His Asp Tyr Ile Gly Val Asp Leu Tyr Tyr Thr Ser Tyr
            260                 265                 270

Tyr Leu Phe Arg Val Leu Phe Val His Leu Leu Pro Cys Ile Ile Leu
        275                 280                 285

Val Thr Leu Asn Ile Leu Leu Phe Ala Ala Met Arg Gln Ala Gln Glu

290                 295                 300

Arg Arg Lys Leu Leu Phe Arg Glu Asn Arg Lys Lys Glu Cys Lys Lys
305                 310                 315                 320

Leu Arg Glu Thr Asn Cys Thr Thr Leu Met Leu Ile Val Val Val Ser
                325                 330                 335

Val Phe Leu Leu Ala Glu Ile Pro Ile Ala Val Val Thr Ala Met His
            340                 345                 350

Ile Val Ser Ser Leu Ile Ile Glu Phe Leu Asp Tyr Gly Leu Ala Asn
        355                 360                 365

Ile Cys Ile Met Leu Thr Asn Phe Phe Leu Val Phe Ser Tyr Pro Ile
    370                 375                 380

Asn Phe Gly Ile Tyr Cys Gly Met Ser Arg Gln Phe Arg Glu Thr Phe
385                 390                 395                 400

Lys Glu Ile Phe Leu Gly Arg Leu Met Ala Lys Lys Asp Ser Ser Thr
                405                 410                 415

Lys Tyr Ser Ile Val Asn Gly Ala Arg Thr Cys Thr Asn Thr Asn Glu
            420                 425                 430

Thr Val Leu
        435

<210>  3
<211>  1536
<212>  DNA
<213>  Aedes aegypti

<220>
<221>  CDS
<222>  (1)..(1536)

<400>  3
atg tca att gat gct gcg gta gtt gct ggt gct ggc gat atc tct aat      48
Met Ser Ile Asp Ala Ala Val Val Ala Gly Ala Gly Asp Ile Ser Asn
1               5                   10                  15

gtc ctc ggt cga tta tcg tct tcc gtt act ggt gcc gga gga tct ggt      96
Val Leu Gly Arg Leu Ser Ser Ser Val Thr Gly Ala Gly Gly Ser Gly
            20                  25                  30

tca aca ttt gct gca aac agc gga gac gcc tca agt ggt ggc gaa cga      144
Ser Thr Phe Ala Ala Asn Ser Gly Asp Ala Ser Ser Gly Gly Glu Arg
        35                  40                  45

atg ctg tat tac ttc gat ggt agc gac gat gat agc act gcg tct aag      192
Met Leu Tyr Tyr Phe Asp Gly Ser Asp Asp Asp Ser Thr Ala Ser Lys
    50                  55                  60

caa cag ttt cac agc ctc caa cac cag cag aaa gcg gca ctt ttg ctg      240
Gln Gln Phe His Ser Leu Gln His Gln Gln Lys Ala Ala Leu Leu Leu
65                  70                  75                  80

ggc gag aac atc aca acc tcc aca atg tat tcc tca ctg gat cct ggc      288
Gly Glu Asn Ile Thr Thr Ser Thr Met Tyr Ser Ser Leu Asp Pro Gly

                85                  90                  95

atc tac aat ccg ata gag gca gaa aac gag act gac ttc ctc tat atc 336
Ile Tyr Asn Pro Ile Glu Ala Glu Asn Glu Thr Asp Phe Leu Tyr Ile
            100                 105                 110

aac gga cca agc agt agt tat ggt tat acg gaa ggt cac aac ggc aca 384
Asn Gly Pro Ser Ser Ser Tyr Gly Tyr Thr Glu Gly His Asn Gly Thr
        115                 120                 125

ctc ccg tac ggc gat gac tac tat cag agc tgt atg gga atg tac ggt 432
Leu Pro Tyr Gly Asp Asp Tyr Tyr Gln Ser Cys Met Gly Met Tyr Gly
    130                 135                 140

aac gtt acg tat ctg aac gtg tcc tgt gaa aca att cta aac tat agt 480
Asn Val Thr Tyr Leu Asn Val Ser Cys Glu Thr Ile Leu Asn Tyr Ser
145                 150                 155                 160

ata cca ctc tac gga tac tgt act ccc atc ttc atc cta att aca ctg 528
Ile Pro Leu Tyr Gly Tyr Cys Thr Pro Ile Phe Ile Leu Ile Thr Leu
                165                 170                 175

act gcc aac tcg ctg atc gtg att gtc ctc agc aaa cgc agc atg gca 576
Thr Ala Asn Ser Leu Ile Val Ile Val Leu Ser Lys Arg Ser Met Ala
            180                 185                 190

tcg ccg acc aac ttc gtg ctg atg gct atg gct ctt tgc gat atg ttt 624
Ser Pro Thr Asn Phe Val Leu Met Ala Met Ala Leu Cys Asp Met Phe
        195                 200                 205

acc ctg ata ttc ccg gcg cca ggc ctg ctg tac atg tac acg ttt ggc 672
Thr Leu Ile Phe Pro Ala Pro Gly Leu Leu Tyr Met Tyr Thr Phe Gly
    210                 215                 220

aat cac tac aag cca tta tcg ccg att acc gcc tgc tac gtg tgg aac 720
Asn His Tyr Lys Pro Leu Ser Pro Ile Thr Ala Cys Tyr Val Trp Asn
225                 230                 235                 240

gcg ctg aac gag cac tta ccg gca atg ttc cac aca gca tca gtc tgg 768
Ala Leu Asn Glu His Leu Pro Ala Met Phe His Thr Ala Ser Val Trp
                245                 250                 255

ctt acg tta gcg ttg gct gta cag agg tac gtc tat gtt tgc cat gct 816
Leu Thr Leu Ala Leu Ala Val Gln Arg Tyr Val Tyr Val Cys His Ala
            260                 265                 270

ccc ata gct cga acg tgg tgc acg att cct cgg gtg aaa aaa tgt atc 864
Pro Ile Ala Arg Thr Trp Cys Thr Ile Pro Arg Val Lys Lys Cys Ile
        275                 280                 285

gcc tac atc tgc ata gca gca cta att cat cag agc acc agg ttt ttc 912
Ala Tyr Ile Cys Ile Ala Ala Leu Ile His Gln Ser Thr Arg Phe Phe
    290                 295                 300

gat aaa tca tac agc ctg gtg aca atc gaa tgg aac ggc cat tcg acg 960
Asp Lys Ser Tyr Ser Leu Val Thr Ile Glu Trp Asn Gly His Ser Thr
305                 310                 315                 320

gag gtt tgc cac acc gag acc tca agc tgg gtg cac gac tac gta ggc 1008
Glu Val Cys His Thr Glu Thr Ser Ser Trp Val His Asp Tyr Val Gly
                325                 330                 335

gaa gat gtg tac tat acc ttc tac ttt tcg ttc cgg ata ttg ttc gtg 1056
Glu Asp Val Tyr Tyr Thr Phe Tyr Phe Ser Phe Arg Ile Leu Phe Val
            340                 345                 350

cat ctg ata ccg tgc ggg agt ttg gtt gcg ttg aac gtg ctg ctt ttc 1104
His Leu Ile Pro Cys Gly Ser Leu Val Ala Leu Asn Val Leu Leu Phe
        355                 360                 365

aag gca atg cgc caa gcg caa cag aag cgc gaa cgt ctc ttc aaa gat 1152
Lys Ala Met Arg Gln Ala Gln Gln Lys Arg Glu Arg Leu Phe Lys Asp

370                 375                 380

acc gcc aaa aag cga gaa tgc aag cga gtc cgg gac tcc aac tgc acc      1200
Thr Ala Lys Lys Arg Glu Cys Lys Arg Val Arg Asp Ser Asn Cys Thr
385                 390                 395                 400

aca ctt atg ttg ata gtc gtg gtg aca gtt ttc ctc atc gtg gaa ata      1248
Thr Leu Met Leu Ile Val Val Val Thr Val Phe Leu Ile Val Glu Ile
                405                 410                 415

cca ctc gct gtg att acg gcc tta cac atc atc tcc tcg ctc att tat      1296
Pro Leu Ala Val Ile Thr Ala Leu His Ile Ile Ser Ser Leu Ile Tyr
            420                 425                 430

gag ttc ctc gac tac tac att gcg aac ctg ttc atc ctg ttt gcg aac      1344
Glu Phe Leu Asp Tyr Tyr Ile Ala Asn Leu Phe Ile Leu Phe Ala Asn
        435                 440                 445

ttt ttc ctc atc gtt agc tat cca atc aat ttt gca atc tac tgc ggg      1392
Phe Phe Leu Ile Val Ser Tyr Pro Ile Asn Phe Ala Ile Tyr Cys Gly
    450                 455                 460

atg tca cgt cag ttc cgg gag acg ttc aag gaa att ttc gtc aaa tcg      1440
Met Ser Arg Gln Phe Arg Glu Thr Phe Lys Glu Ile Phe Val Lys Ser
465                 470                 475                 480

agc aaa caa atc ccg agc gga aaa aag gat tgt ggc tcc tcg cgg tat      1488
Ser Lys Gln Ile Pro Ser Gly Lys Lys Asp Cys Gly Ser Ser Arg Tyr
                485                 490                 495

tcc ctg gtc aac ggg cca aga act ttc acc aat gaa acg gtg ctg taa      1536
Ser Leu Val Asn Gly Pro Arg Thr Phe Thr Asn Glu Thr Val Leu
            500                 505                 510


<210>  4
<211>  511
<212>  PRT
<213>  Aedes aegypti

<400>  4

Met Ser Ile Asp Ala Ala Val Val Ala Gly Ala Gly Asp Ile Ser Asn
1               5                   10                  15


Val Leu Gly Arg Leu Ser Ser Ser Val Thr Gly Ala Gly Gly Ser Gly
            20                  25                  30


Ser Thr Phe Ala Ala Asn Ser Gly Asp Ala Ser Ser Gly Gly Glu Arg
        35                  40                  45


Met Leu Tyr Tyr Phe Asp Gly Ser Asp Asp Asp Ser Thr Ala Ser Lys
    50                  55                  60


Gln Gln Phe His Ser Leu Gln His Gln Gln Lys Ala Ala Leu Leu Leu
65                  70                  75                  80


Gly Glu Asn Ile Thr Thr Ser Thr Met Tyr Ser Ser Leu Asp Pro Gly
                85                  90                  95


Ile Tyr Asn Pro Ile Glu Ala Glu Asn Glu Thr Asp Phe Leu Tyr Ile
            100                 105                 110


Asn Gly Pro Ser Ser Ser Tyr Gly Tyr Thr Glu Gly His Asn Gly Thr
        115                 120                 125

Leu Pro Tyr Gly Asp Asp Tyr Tyr Gln Ser Cys Met Gly Met Tyr Gly
    130                 135                 140

Asn Val Thr Tyr Leu Asn Val Ser Cys Glu Thr Ile Leu Asn Tyr Ser
145                 150                 155                 160

Ile Pro Leu Tyr Gly Tyr Cys Thr Pro Ile Phe Ile Leu Ile Thr Leu
                165                 170                 175

Thr Ala Asn Ser Leu Ile Val Ile Val Leu Ser Lys Arg Ser Met Ala
            180                 185                 190

Ser Pro Thr Asn Phe Val Leu Met Ala Met Ala Leu Cys Asp Met Phe
        195                 200                 205

Thr Leu Ile Phe Pro Ala Pro Gly Leu Leu Tyr Met Tyr Thr Phe Gly
    210                 215                 220

Asn His Tyr Lys Pro Leu Ser Pro Ile Thr Ala Cys Tyr Val Trp Asn
225                 230                 235                 240

Ala Leu Asn Glu His Leu Pro Ala Met Phe His Thr Ala Ser Val Trp
                245                 250                 255

Leu Thr Leu Ala Leu Ala Val Gln Arg Tyr Val Tyr Val Cys His Ala
            260                 265                 270

Pro Ile Ala Arg Thr Trp Cys Thr Ile Pro Arg Val Lys Lys Cys Ile
        275                 280                 285

Ala Tyr Ile Cys Ile Ala Ala Leu Ile His Gln Ser Thr Arg Phe Phe
    290                 295                 300

Asp Lys Ser Tyr Ser Leu Val Thr Ile Glu Trp Asn Gly His Ser Thr
305                 310                 315                 320

Glu Val Cys His Thr Glu Thr Ser Ser Trp Val His Asp Tyr Val Gly
                325                 330                 335

Glu Asp Val Tyr Tyr Thr Phe Tyr Phe Ser Phe Arg Ile Leu Phe Val
            340                 345                 350

His Leu Ile Pro Cys Gly Ser Leu Val Ala Leu Asn Val Leu Leu Phe
        355                 360                 365

Lys Ala Met Arg Gln Ala Gln Gln Lys Arg Glu Arg Leu Phe Lys Asp
    370                 375                 380

Thr Ala Lys Lys Arg Glu Cys Lys Arg Val Arg Asp Ser Asn Cys Thr
385                 390                 395                 400

Thr Leu Met Leu Ile Val Val Val Thr Val Phe Leu Ile Val Glu Ile
                405                 410                 415

Pro Leu Ala Val Ile Thr Ala Leu His Ile Ile Ser Ser Leu Ile Tyr
            420                 425                 430

Glu Phe Leu Asp Tyr Tyr Ile Ala Asn Leu Phe Ile Leu Phe Ala Asn
        435                 440                 445

Phe Phe Leu Ile Val Ser Tyr Pro Ile Asn Phe Ala Ile Tyr Cys Gly
    450                 455                 460

Met Ser Arg Gln Phe Arg Glu Thr Phe Lys Glu Ile Phe Val Lys Ser
465                 470                 475                 480

Ser Lys Gln Ile Pro Ser Gly Lys Lys Asp Cys Gly Ser Ser Arg Tyr
                485                 490                 495

Ser Leu Val Asn Gly Pro Arg Thr Phe Thr Asn Glu Thr Val Leu
            500                 505                 510

<210>  5
<211>  1395
<212>  DNA
<213>  Anopheles gambiae

<220>
<221>  CDS
<222>  (1)..(1395)

<400>  5
atg att gaa aaa aat aat ttc aag tca tac cat tta ttt cgg ggt gat       48
Met Ile Glu Lys Asn Asn Phe Lys Ser Tyr His Leu Phe Arg Gly Asp
1               5                   10                  15

cag tac cta tct aat atg ata gag cca aaa aaa aac aga gca ccg att       96
Gln Tyr Leu Ser Asn Met Ile Glu Pro Lys Lys Asn Arg Ala Pro Ile
            20                  25                  30

att cta ttc aac tct cca cac atg gca act tat tac tat ttc aat gaa      144
Ile Leu Phe Asn Ser Pro His Met Ala Thr Tyr Tyr Tyr Phe Asn Glu
        35                  40                  45

tca acg cct ctt tcg tca caa ata ttc ttt cca aaa gaa agc tca gaa      192
Ser Thr Pro Leu Ser Ser Gln Ile Phe Phe Pro Lys Glu Ser Ser Glu
    50                  55                  60

aac tat aat act act ggt atc aac acg agt act tcc tac acg gtt cat      240
Asn Tyr Asn Thr Thr Gly Ile Asn Thr Ser Thr Ser Tyr Thr Val His
65                  70                  75                  80

atg aat gaa ttg aat aac gac tcg aca tac agt tac tat aat tgc cta      288
Met Asn Glu Leu Asn Asn Asp Ser Thr Tyr Ser Tyr Tyr Asn Cys Leu
                85                  90                  95

gca tca gaa gga aac acg tct tac ttg aat gtg tca tgt gaa acc att      336
Ala Ser Glu Gly Asn Thr Ser Tyr Leu Asn Val Ser Cys Glu Thr Ile
            100                 105                 110

ctt aat tat agc att ccg ttg tat gga tat tgt ata ccc ttt ctt ttg      384
Leu Asn Tyr Ser Ile Pro Leu Tyr Gly Tyr Cys Ile Pro Phe Leu Leu
        115                 120                 125

ctg gtt acc ctc aca gca aat tct tta ata att att atc ctc aat aaa      432
Leu Val Thr Leu Thr Ala Asn Ser Leu Ile Ile Ile Ile Leu Asn Lys
    130                 135                 140

aga agc atg gcg tca cca aca aat tgt att ctt atg gca atg gct ctt 480
Arg Ser Met Ala Ser Pro Thr Asn Cys Ile Leu Met Ala Met Ala Leu
145                 150                 155                 160

tgt gat atg ttt acc ctt ctt ttt ccg gca cct ggt ttg ata tac atg 528
Cys Asp Met Phe Thr Leu Leu Phe Pro Ala Pro Gly Leu Ile Tyr Met
                165                 170                 175

tat aca ttt gga aac cat tac aaa cca ttg tca cct tta ata gcg tgc 576
Tyr Thr Phe Gly Asn His Tyr Lys Pro Leu Ser Pro Leu Ile Ala Cys
            180                 185                 190

tac gtt tgg aat gcc ctg aat gag att cta cct gca atg tgt cat act 624
Tyr Val Trp Asn Ala Leu Asn Glu Ile Leu Pro Ala Met Cys His Thr
        195                 200                 205

gca tca gta tgg ctt aca tta gct tta gct ttg caa aga tac ata tac 672
Ala Ser Val Trp Leu Thr Leu Ala Leu Ala Leu Gln Arg Tyr Ile Tyr
    210                 215                 220

gtt tgc cat gca cct gcc cga aat tgg tgt act att cct aga gta aaa 720
Val Cys His Ala Pro Ala Arg Asn Trp Cys Thr Ile Pro Arg Val Lys
225                 230                 235                 240

aaa tgc att ggt tat atc tgt gct act gct ttt tta cac caa agc agt 768
Lys Cys Ile Gly Tyr Ile Cys Ala Thr Ala Phe Leu His Gln Ser Ser
                245                 250                 255

aga ttt ttt gac aag tct tat agt cta ata aca ata atg tgg aat ggc 816
Arg Phe Phe Asp Lys Ser Tyr Ser Leu Ile Thr Ile Met Trp Asn Gly
            260                 265                 270

caa atg aca aac gta tgt cat gtt gaa aca gca aat tgg ata cac aaa 864
Gln Met Thr Asn Val Cys His Val Glu Thr Ala Asn Trp Ile His Lys
        275                 280                 285

tac acc agc gaa gat ttt tat tac aca ttc tat ttt tcc ttt cgc att 912
Tyr Thr Ser Glu Asp Phe Tyr Tyr Thr Phe Tyr Phe Ser Phe Arg Ile
    290                 295                 300

tta ttt gtc cat tta aca ccc tgt gcc agt ctg gta gct cta aat gtt 960
Leu Phe Val His Leu Thr Pro Cys Ala Ser Leu Val Ala Leu Asn Val
305                 310                 315                 320

ttc ctt ttt agg gcc atg aaa caa gct caa aag aag aga gaa cgg ctt 1008
Phe Leu Phe Arg Ala Met Lys Gln Ala Gln Lys Lys Arg Glu Arg Leu
                325                 330                 335

ttt aag gac aac aag aaa cgt gaa tgt aaa cgg atc cgc gat tct aac 1056
Phe Lys Asp Asn Lys Lys Arg Glu Cys Lys Arg Ile Arg Asp Ser Asn
            340                 345                 350

tgt act act ctt atg tta atc gtt gtg gtt acc gtc ttc tta gtt gta 1104
Cys Thr Thr Leu Met Leu Ile Val Val Val Thr Val Phe Leu Val Val
        355                 360                 365

gaa ata cca cta ggt gtt att aca gca ctt cat att ata tca tct ctc 1152
Glu Ile Pro Leu Gly Val Ile Thr Ala Leu His Ile Ile Ser Ser Leu
    370                 375                 380

att tac gaa ttt ctt gat tat tat gtt gct aac ttg ttt att ttg ttc 1200
Ile Tyr Glu Phe Leu Asp Tyr Tyr Val Ala Asn Leu Phe Ile Leu Phe
385                 390                 395                 400

gct aat ttt ttt cta ata gtc agt tat cca ata aat ttt gcg att tat 1248
Ala Asn Phe Phe Leu Ile Val Ser Tyr Pro Ile Asn Phe Ala Ile Tyr
                405                 410                 415

tgc gga atg tcg cgt caa ttt cgt gag aca ttc aaa gga ata ttt gtt 1296
Cys Gly Met Ser Arg Gln Phe Arg Glu Thr Phe Lys Gly Ile Phe Val
            420                 425                 430

caa tca gat tct caa atc aaa ata aca aaa gaa tat ggt tca tcg aag      1344
Gln Ser Asp Ser Gln Ile Lys Ile Thr Lys Glu Tyr Gly Ser Ser Lys
        435                 440                 445

tat tcg tta gta aat gga ccg aga acg tgt aca aat gaa act gtg att      1392
Tyr Ser Leu Val Asn Gly Pro Arg Thr Cys Thr Asn Glu Thr Val Ile
    450                 455                 460

taa                                                                  1395

<210>  6
<211>  464
<212>  PRT
<213>  Anopheles gambiae

<400>  6

Met Ile Glu Lys Asn Asn Phe Lys Ser Tyr His Leu Phe Arg Gly Asp
1               5                   10                  15

Gln Tyr Leu Ser Asn Met Ile Glu Pro Lys Lys Asn Arg Ala Pro Ile
            20                  25                  30

Ile Leu Phe Asn Ser Pro His Met Ala Thr Tyr Tyr Tyr Phe Asn Glu
        35                  40                  45

Ser Thr Pro Leu Ser Ser Gln Ile Phe Phe Pro Lys Glu Ser Ser Glu
    50                  55                  60

Asn Tyr Asn Thr Thr Gly Ile Asn Thr Ser Thr Ser Tyr Thr Val His
65                  70                  75                  80

Met Asn Glu Leu Asn Asn Asp Ser Thr Tyr Ser Tyr Tyr Asn Cys Leu
                85                  90                  95

Ala Ser Glu Gly Asn Thr Ser Tyr Leu Asn Val Ser Cys Glu Thr Ile
            100                 105                 110

Leu Asn Tyr Ser Ile Pro Leu Tyr Gly Tyr Cys Ile Pro Phe Leu Leu
        115                 120                 125

Leu Val Thr Leu Thr Ala Asn Ser Leu Ile Ile Ile Ile Leu Asn Lys
    130                 135                 140

Arg Ser Met Ala Ser Pro Thr Asn Cys Ile Leu Met Ala Met Ala Leu
145                 150                 155                 160

Cys Asp Met Phe Thr Leu Leu Phe Pro Ala Pro Gly Leu Ile Tyr Met
                165                 170                 175

Tyr Thr Phe Gly Asn His Tyr Lys Pro Leu Ser Pro Leu Ile Ala Cys
            180                 185                 190

Tyr Val Trp Asn Ala Leu Asn Glu Ile Leu Pro Ala Met Cys His Thr
        195                 200                 205

Ala Ser Val Trp Leu Thr Leu Ala Leu Ala Leu Gln Arg Tyr Ile Tyr

210                 215                 220

Val Cys His Ala Pro Ala Arg Asn Trp Cys Thr Ile Pro Arg Val Lys
225                 230                 235                 240

Lys Cys Ile Gly Tyr Ile Cys Ala Thr Ala Phe Leu His Gln Ser Ser
                245                 250                 255

Arg Phe Phe Asp Lys Ser Tyr Ser Leu Ile Thr Ile Met Trp Asn Gly
            260                 265                 270

Gln Met Thr Asn Val Cys His Val Glu Thr Ala Asn Trp Ile His Lys
        275                 280                 285

Tyr Thr Ser Glu Asp Phe Tyr Tyr Thr Phe Tyr Phe Ser Phe Arg Ile
    290                 295                 300

Leu Phe Val His Leu Thr Pro Cys Ala Ser Leu Val Ala Leu Asn Val
305                 310                 315                 320

Phe Leu Phe Arg Ala Met Lys Gln Ala Gln Lys Lys Arg Glu Arg Leu
                325                 330                 335

Phe Lys Asp Asn Lys Lys Arg Glu Cys Lys Arg Ile Arg Asp Ser Asn
            340                 345                 350

Cys Thr Thr Leu Met Leu Ile Val Val Val Thr Val Phe Leu Val Val
        355                 360                 365

Glu Ile Pro Leu Gly Val Ile Thr Ala Leu His Ile Ile Ser Ser Leu
    370                 375                 380

Ile Tyr Glu Phe Leu Asp Tyr Tyr Val Ala Asn Leu Phe Ile Leu Phe
385                 390                 395                 400

Ala Asn Phe Phe Leu Ile Val Ser Tyr Pro Ile Asn Phe Ala Ile Tyr
                405                 410                 415

Cys Gly Met Ser Arg Gln Phe Arg Glu Thr Phe Lys Gly Ile Phe Val
            420                 425                 430

Gln Ser Asp Ser Gln Ile Lys Ile Thr Lys Glu Tyr Gly Ser Ser Lys
        435                 440                 445

Tyr Ser Leu Val Asn Gly Pro Arg Thr Cys Thr Asn Glu Thr Val Ile
    450                 455                 460

<210>  7
<211>  1221
<212>  DNA
<213>  Bombyx mori

<220>
<221>  CDS
<222>  (1)..(1221)

<400> 7
atg gcg gtc acc ata gac aat tca acg aac gac ttc gaa ttc cag aag      48
Met Ala Val Thr Ile Asp Asn Ser Thr Asn Asp Phe Glu Phe Gln Lys
1               5                   10                  15

cca ttc aac tac tca att aac gag aac atc acc tac ttc gat tac acg      96
Pro Phe Asn Tyr Ser Ile Asn Glu Asn Ile Thr Tyr Phe Asp Tyr Thr
            20                  25                  30

aac ttc act tcg gac gat ttc tgc gca tca aac aac tca cac gta tac      144
Asn Phe Thr Ser Asp Asp Phe Cys Ala Ser Asn Asn Ser His Val Tyr
        35                  40                  45

tta aac gtc acc tgt gag ttc gct ata agc tat gct gag cct atg tac      192
Leu Asn Val Thr Cys Glu Phe Ala Ile Ser Tyr Ala Glu Pro Met Tyr
    50                  55                  60

ggg tac atc gca ccg ttt ctg cta gct aca acg aca gtt gcc aac acc      240
Gly Tyr Ile Ala Pro Phe Leu Leu Ala Thr Thr Thr Val Ala Asn Thr
65                  70                  75                  80

ctg atc gtg gtg gtg cta tcg cgg agg cac atg agg acg ccc acc aat      288
Leu Ile Val Val Val Leu Ser Arg Arg His Met Arg Thr Pro Thr Asn
                85                  90                  95

gcc gtg ctg atg gcg atg gct ctt tgc gat atg ttc acc atg ctg ttc      336
Ala Val Leu Met Ala Met Ala Leu Cys Asp Met Phe Thr Met Leu Phe
            100                 105                 110

ccg gcg ccg tgg cta ttc tac atg tac act ttt gga aac cac tac aag      384
Pro Ala Pro Trp Leu Phe Tyr Met Tyr Thr Phe Gly Asn His Tyr Lys
        115                 120                 125

ccg cta agt ccc gtc aga gcc tgc caa gct tgg aat tac atg aac gag      432
Pro Leu Ser Pro Val Arg Ala Cys Gln Ala Trp Asn Tyr Met Asn Glu
    130                 135                 140

gtc att cca gcg atg ttc cac acg gct agt atc tgg ttg acg tta gcc      480
Val Ile Pro Ala Met Phe His Thr Ala Ser Ile Trp Leu Thr Leu Ala
145                 150                 155                 160

tta gcc gtc cag aga tat atc tac gtt tgc cac gcg ccc gtc gcc agg      528
Leu Ala Val Gln Arg Tyr Ile Tyr Val Cys His Ala Pro Val Ala Arg
                165                 170                 175

acc tgg tgt acc atg ccc cgg gtg atg aaa tgt ctg atc tac ata gga      576
Thr Trp Cys Thr Met Pro Arg Val Met Lys Cys Leu Ile Tyr Ile Gly
            180                 185                 190

ata gcg gcg ttt ctt cat cag cta ccg cgg ttc ttc gac aga tgc tac      624
Ile Ala Ala Phe Leu His Gln Leu Pro Arg Phe Phe Asp Arg Cys Tyr
        195                 200                 205

act ccc cac aag act gtg tgg cgg ggg cgc gtg gag gaa gtc tgc aga      672
Thr Pro His Lys Thr Val Trp Arg Gly Arg Val Glu Glu Val Cys Arg
    210                 215                 220

atc gag atg gcg tct tgg gtg aaa gcg ctc tcc gtc gac gcc tat ttc      720
Ile Glu Met Ala Ser Trp Val Lys Ala Leu Ser Val Asp Ala Tyr Phe
225                 230                 235                 240

atc tcc tac ttc ggc ttc aga gtt ttg ttc gtc cac tta atc ccg tgc      768
Ile Ser Tyr Phe Gly Phe Arg Val Leu Phe Val His Leu Ile Pro Cys
                245                 250                 255

aca tct tta gtt gtc ctg aac gtg ctc ctc ttc agg gcc atg agg acg      816
Thr Ser Leu Val Val Leu Asn Val Leu Leu Phe Arg Ala Met Arg Thr
            260                 265                 270

gcc caa ata aac aga cag aag ctg ttc aaa gaa aac cga aaa tcg gag      864
Ala Gln Ile Asn Arg Gln Lys Leu Phe Lys Glu Asn Arg Lys Ser Glu

275                 280                 285

tgc aag aga ctc aga gat tct aac tgc acc acc ctg atg ttg ata gtc      912
Cys Lys Arg Leu Arg Asp Ser Asn Cys Thr Thr Leu Met Leu Ile Val
    290                 295                 300

gtg gtc acg gtt ttc ctc ctc gtg gaa ata ccc gtg gcc gtc gtc act      960
Val Val Thr Val Phe Leu Leu Val Glu Ile Pro Val Ala Val Val Thr
305                 310                 315                 320

atc ctc cac ata ata tcc agc acg att gtc gag atc ttg gac tac cat     1008
Ile Leu His Ile Ile Ser Ser Thr Ile Val Glu Ile Leu Asp Tyr His
                325                 330                 335

atc gcg aat att ctc gtt ctg gta acg aat ttc ttc att atc gtt tcc     1056
Ile Ala Asn Ile Leu Val Leu Val Thr Asn Phe Phe Ile Ile Val Ser
            340                 345                 350

tat cca atc aac ttt gct ata tat tgc ggt atg tcg cgt caa ttc agg     1104
Tyr Pro Ile Asn Phe Ala Ile Tyr Cys Gly Met Ser Arg Gln Phe Arg
        355                 360                 365

gag acc ttc aaa gaa ttg ttt atc aga ggc acg gtt act agt agg aag     1152
Glu Thr Phe Lys Glu Leu Phe Ile Arg Gly Thr Val Thr Ser Arg Lys
    370                 375                 380

aac ggt ggc tcc agt agg tac tct ctt gtc aat ggc ccg agg acc tgt     1200
Asn Gly Gly Ser Ser Arg Tyr Ser Leu Val Asn Gly Pro Arg Thr Cys
385                 390                 395                 400

aca aac gaa act gtg ctt taa                                         1221
Thr Asn Glu Thr Val Leu
                405

<210>  8
<211>  406
<212>  PRT
<213>  Bombyx mori

<400>  8

Met Ala Val Thr Ile Asp Asn Ser Thr Asn Asp Phe Glu Phe Gln Lys
1               5                   10                  15

Pro Phe Asn Tyr Ser Ile Asn Glu Asn Ile Thr Tyr Phe Asp Tyr Thr
            20                  25                  30

Asn Phe Thr Ser Asp Asp Phe Cys Ala Ser Asn Asn Ser His Val Tyr
        35                  40                  45

Leu Asn Val Thr Cys Glu Phe Ala Ile Ser Tyr Ala Glu Pro Met Tyr
    50                  55                  60

Gly Tyr Ile Ala Pro Phe Leu Leu Ala Thr Thr Thr Val Ala Asn Thr
65                  70                  75                  80

Leu Ile Val Val Val Leu Ser Arg Arg His Met Arg Thr Pro Thr Asn
                85                  90                  95

Ala Val Leu Met Ala Met Ala Leu Cys Asp Met Phe Thr Met Leu Phe
            100                 105                 110

Pro Ala Pro Trp Leu Phe Tyr Met Tyr Thr Phe Gly Asn His Tyr Lys
        115                 120                 125

Pro Leu Ser Pro Val Arg Ala Cys Gln Ala Trp Asn Tyr Met Asn Glu
    130                 135                 140

Val Ile Pro Ala Met Phe His Thr Ala Ser Ile Trp Leu Thr Leu Ala
145                 150                 155                 160

Leu Ala Val Gln Arg Tyr Ile Tyr Val Cys His Ala Pro Val Ala Arg
                165                 170                 175

Thr Trp Cys Thr Met Pro Arg Val Met Lys Cys Leu Ile Tyr Ile Gly
            180                 185                 190

Ile Ala Ala Phe Leu His Gln Leu Pro Arg Phe Phe Asp Arg Cys Tyr
        195                 200                 205

Thr Pro His Lys Thr Val Trp Arg Gly Arg Val Glu Glu Val Cys Arg
    210                 215                 220

Ile Glu Met Ala Ser Trp Val Lys Ala Leu Ser Val Asp Ala Tyr Phe
225                 230                 235                 240

Ile Ser Tyr Phe Gly Phe Arg Val Leu Phe Val His Leu Ile Pro Cys
                245                 250                 255

Thr Ser Leu Val Val Leu Asn Val Leu Leu Phe Arg Ala Met Arg Thr
            260                 265                 270

Ala Gln Ile Asn Arg Gln Lys Leu Phe Lys Glu Asn Arg Lys Ser Glu
        275                 280                 285

Cys Lys Arg Leu Arg Asp Ser Asn Cys Thr Thr Leu Met Leu Ile Val
    290                 295                 300

Val Val Thr Val Phe Leu Leu Val Glu Ile Pro Val Ala Val Val Thr
305                 310                 315                 320

Ile Leu His Ile Ile Ser Ser Thr Ile Val Glu Ile Leu Asp Tyr His
                325                 330                 335

Ile Ala Asn Ile Leu Val Leu Val Thr Asn Phe Phe Ile Ile Val Ser
            340                 345                 350

Tyr Pro Ile Asn Phe Ala Ile Tyr Cys Gly Met Ser Arg Gln Phe Arg
        355                 360                 365

Glu Thr Phe Lys Glu Leu Phe Ile Arg Gly Thr Val Thr Ser Arg Lys
    370                 375                 380

Asn Gly Gly Ser Ser Arg Tyr Ser Leu Val Asn Gly Pro Arg Thr Cys
385                 390                 395                 400

Thr Asn Glu Thr Val Leu
                405

<210>  9
<211>  1176
<212>  DNA
<213>  Tribolium castaneum

<220>
<221>  CDS
<222>  (1)..(1176)

<400>  9
atg ggc gag atg gcg tcg aac tcg acg ctg att ttt ccg aat cag act      48
Met Gly Glu Met Ala Ser Asn Ser Thr Leu Ile Phe Pro Asn Gln Thr
1               5                   10                  15

tac gcg aac gaa acc gta cca aat gtt acg tca gtg gaa aaa gtc cag      96
Tyr Ala Asn Glu Thr Val Pro Asn Val Thr Ser Val Glu Lys Val Gln
            20                  25                  30

tac att aac atc acg atg gaa atg cct att gcc tac gct gtg ccc tta      144
Tyr Ile Asn Ile Thr Met Glu Met Pro Ile Ala Tyr Ala Val Pro Leu
        35                  40                  45

tat ggc tac gtt atg ccg ttc tta ttg ata ata act att atc gcg aat      192
Tyr Gly Tyr Val Met Pro Phe Leu Leu Ile Ile Thr Ile Ile Ala Asn
    50                  55                  60

act tta atc gtc gtt gtg ctg agc aaa agg cag atg agg act cca aca      240
Thr Leu Ile Val Val Val Leu Ser Lys Arg Gln Met Arg Thr Pro Thr
65                  70                  75                  80

aac gtc gtt ttg atg gcg atg gct ctt tgt gat atg ttc acg gtt tta      288
Asn Val Val Leu Met Ala Met Ala Leu Cys Asp Met Phe Thr Val Leu
                85                  90                  95

ata ccg gct cct tgg ctt atc tac atg tac tcc ttt ggg aac cac tac      336
Ile Pro Ala Pro Trp Leu Ile Tyr Met Tyr Ser Phe Gly Asn His Tyr
            100                 105                 110

aag ccc ctt tgg cct ata agt ctt tgt tac gcc tgg ttt gtt atg cac      384
Lys Pro Leu Trp Pro Ile Ser Leu Cys Tyr Ala Trp Phe Val Met His
        115                 120                 125

gag gta att ccc aac atg ttc cac acg gcg agc att tgg cta aca ttg      432
Glu Val Ile Pro Asn Met Phe His Thr Ala Ser Ile Trp Leu Thr Leu
    130                 135                 140

gcc ctc gct gtt cag agg tac att ttc gta tgc cat gcg cct ctt gca      480
Ala Leu Ala Val Gln Arg Tyr Ile Phe Val Cys His Ala Pro Leu Ala
145                 150                 155                 160

cga aaa ttg tgc acc atg tct aac gtc tac aag tgt ctt atc tat att      528
Arg Lys Leu Cys Thr Met Ser Asn Val Tyr Lys Cys Leu Ile Tyr Ile
                165                 170                 175

cta gtc ata gca gct ctt cat cag ggt ttc cgg ttt ttt gac tca gaa      576
Leu Val Ile Ala Ala Leu His Gln Gly Phe Arg Phe Phe Asp Ser Glu
            180                 185                 190

tat agc acg gtg gac gtg cta tgg aac aac cac aca aca cat gtg tgc      624
Tyr Ser Thr Val Asp Val Leu Trp Asn Asn His Thr Thr His Val Cys
        195                 200                 205

aaa aga gag cat gca cat tgg gtg aag gaa tac gtg acc gag gat ttc      672
Lys Arg Glu His Ala His Trp Val Lys Glu Tyr Val Thr Glu Asp Phe
    210                 215                 220

tac ttc gta act tat ttc atg ttc agg gta tta ttt gtg cat cta att      720
Tyr Phe Val Thr Tyr Phe Met Phe Arg Val Leu Phe Val His Leu Ile
225                 230                 235                 240

cct tgt gta gct ctc gtg att ttg aat atc cta ttg ttt cga gca ctg 768
Pro Cys Val Ala Leu Val Ile Leu Asn Ile Leu Leu Phe Arg Ala Leu
                245                 250                 255

aaa caa gcg caa cag cga agg gag caa ctg ctt tcc aaa aag aat caa 816
Lys Gln Ala Gln Gln Arg Arg Glu Gln Leu Leu Ser Lys Lys Asn Gln
            260                 265                 270

aag aac gaa tgc aag aag tta aga gac tct aat tgc acc act ctg atg 864
Lys Asn Glu Cys Lys Lys Leu Arg Asp Ser Asn Cys Thr Thr Leu Met
        275                 280                 285

ctg ata gtc gta gta act gtg ttt ctt att gta gag att ccg cta gtg 912
Leu Ile Val Val Val Thr Val Phe Leu Ile Val Glu Ile Pro Leu Val
    290                 295                 300

gtg gtt act ttg ttg cat ata ata tcc agc acg ttt att gag ttt ctg 960
Val Val Thr Leu Leu His Ile Ile Ser Ser Thr Phe Ile Glu Phe Leu
305                 310                 315                 320

gac tac tat gta gcg aat acg tta att ctt ttt acc aat ttt ttc ata 1008
Asp Tyr Tyr Val Ala Asn Thr Leu Ile Leu Phe Thr Asn Phe Phe Ile
                325                 330                 335

atc tta agt tac ccg att aat ttc gct att tat tgt ggc atg tcg cga 1056
Ile Leu Ser Tyr Pro Ile Asn Phe Ala Ile Tyr Cys Gly Met Ser Arg
            340                 345                 350

caa ttt aga gag act ttt aag gaa ttg ttt ata cgg ggc gcc gtt acg 1104
Gln Phe Arg Glu Thr Phe Lys Glu Leu Phe Ile Arg Gly Ala Val Thr
        355                 360                 365

acc aga aat gga tcg tct agg tat tcg ctg gtc aat gga cct cga act 1152
Thr Arg Asn Gly Ser Ser Arg Tyr Ser Leu Val Asn Gly Pro Arg Thr
    370                 375                 380

tgt acc aat gaa act gtt ttg taa 1176
Cys Thr Asn Glu Thr Val Leu
385                 390

<210> 10
<211> 391
<212> PRT
<213> Tribolium castaneum

<400> 10

Met Gly Glu Met Ala Ser Asn Ser Thr Leu Ile Phe Pro Asn Gln Thr
1               5                   10                  15

Tyr Ala Asn Glu Thr Val Pro Asn Val Thr Ser Val Glu Lys Val Gln
            20                  25                  30

Tyr Ile Asn Ile Thr Met Glu Met Pro Ile Ala Tyr Ala Val Pro Leu
        35                  40                  45

Tyr Gly Tyr Val Met Pro Phe Leu Leu Ile Ile Thr Ile Ile Ala Asn
    50                  55                  60

Thr Leu Ile Val Val Val Leu Ser Lys Arg Gln Met Arg Thr Pro Thr
65                  70                  75                  80

Asn Val Val Leu Met Ala Met Ala Leu Cys Asp Met Phe Thr Val Leu
                85                  90                  95

Ile Pro Ala Pro Trp Leu Ile Tyr Met Tyr Ser Phe Gly Asn His Tyr
            100                 105                 110

Lys Pro Leu Trp Pro Ile Ser Leu Cys Tyr Ala Trp Phe Val Met His
        115                 120                 125

Glu Val Ile Pro Asn Met Phe His Thr Ala Ser Ile Trp Leu Thr Leu
    130                 135                 140

Ala Leu Ala Val Gln Arg Tyr Ile Phe Val Cys His Ala Pro Leu Ala
145                 150                 155                 160

Arg Lys Leu Cys Thr Met Ser Asn Val Tyr Lys Cys Leu Ile Tyr Ile
                165                 170                 175

Leu Val Ile Ala Ala Leu His Gln Gly Phe Arg Phe Phe Asp Ser Glu
            180                 185                 190

Tyr Ser Thr Val Asp Val Leu Trp Asn Asn His Thr Thr His Val Cys
        195                 200                 205

Lys Arg Glu His Ala His Trp Val Lys Glu Tyr Val Thr Glu Asp Phe
    210                 215                 220

Tyr Phe Val Thr Tyr Phe Met Phe Arg Val Leu Phe Val His Leu Ile
225                 230                 235                 240

Pro Cys Val Ala Leu Val Ile Leu Asn Ile Leu Leu Phe Arg Ala Leu
                245                 250                 255

Lys Gln Ala Gln Gln Arg Arg Glu Gln Leu Leu Ser Lys Lys Asn Gln
            260                 265                 270

Lys Asn Glu Cys Lys Lys Leu Arg Asp Ser Asn Cys Thr Thr Leu Met
        275                 280                 285

Leu Ile Val Val Val Thr Val Phe Leu Ile Val Glu Ile Pro Leu Val
    290                 295                 300

Val Val Thr Leu Leu His Ile Ile Ser Ser Thr Phe Ile Glu Phe Leu
305                 310                 315                 320

Asp Tyr Tyr Val Ala Asn Thr Leu Ile Leu Phe Thr Asn Phe Phe Ile
                325                 330                 335

Ile Leu Ser Tyr Pro Ile Asn Phe Ala Ile Tyr Cys Gly Met Ser Arg
            340                 345                 350

Gln Phe Arg Glu Thr Phe Lys Glu Leu Phe Ile Arg Gly Ala Val Thr
        355                 360                 365

Thr Arg Asn Gly Ser Ser Arg Tyr Ser Leu Val Asn Gly Pro Arg Thr
    370                 375                 380

Cys Thr Asn Glu Thr Val Leu
385                 390

<210>  11
<211>  1050
<212>  DNA
<213>  Pediculus humanus corporis

<220>
<221>  CDS
<222>  (1)..(1050)
<223>  Partial Sequence

<400>  11
ttc gct caa ccc tta tac ggt tac ata gta cct ttt tta ctg gtc ata        48
Phe Ala Gln Pro Leu Tyr Gly Tyr Ile Val Pro Phe Leu Leu Val Ile
1               5                   10                  15

aca atc ata gca aac aca tta ata gtc gtg gtt tta tca aag aga cac        96
Thr Ile Ile Ala Asn Thr Leu Ile Val Val Val Leu Ser Lys Arg His
            20                  25                  30

atg aga acg ccg aca aat ttt gtt ttg atg gtc atg gca cta tcc gac       144
Met Arg Thr Pro Thr Asn Phe Val Leu Met Val Met Ala Leu Ser Asp
        35                  40                  45

atg ttc acg cta tta ttt ccc gct ccc tgg ctt ttt tac atg tac aca       192
Met Phe Thr Leu Leu Phe Pro Ala Pro Trp Leu Phe Tyr Met Tyr Thr
    50                  55                  60

ttt ggc aat cac tac aaa ccg ttg tgg cct gtc gcc gcg tgt tat tta       240
Phe Gly Asn His Tyr Lys Pro Leu Trp Pro Val Ala Ala Cys Tyr Leu
65                  70                  75                  80

tgg aac gca atg aaa gaa gat ata cct gca tta ttt cac acg gca tcg       288
Trp Asn Ala Met Lys Glu Asp Ile Pro Ala Leu Phe His Thr Ala Ser
                85                  90                  95

ata tgg ttg aca tta gca ttg gct gta caa agg tac ata tac gtt tgt       336
Ile Trp Leu Thr Leu Ala Leu Ala Val Gln Arg Tyr Ile Tyr Val Cys
            100                 105                 110

cac gca ccg gtg gct cgc act tgg tgc acg atg cca aga gtc atc aaa       384
His Ala Pro Val Ala Arg Thr Trp Cys Thr Met Pro Arg Val Ile Lys
        115                 120                 125

tgc gtg gct tgg atc tac gta ttc gcg acc ctt cat caa tct tct aga       432
Cys Val Ala Trp Ile Tyr Val Phe Ala Thr Leu His Gln Ser Ser Arg
    130                 135                 140

ttt ttc gat cga act tac act tcg ata aac ata acg tgg aac ggt gtg       480
Phe Phe Asp Arg Thr Tyr Thr Ser Ile Asn Ile Thr Trp Asn Gly Val
145                 150                 155                 160

cct gat gtg gcc gtt tgc aaa gtt aat tta gcc acg tgg gtc acc gac       528
Pro Asp Val Ala Val Cys Lys Val Asn Leu Ala Thr Trp Val Thr Asp
                165                 170                 175

atc gtg acc gaa gat gtt tat ttt acc gtt tat tat tgc ttt cgc gtt       576
Ile Val Thr Glu Asp Val Tyr Phe Thr Val Tyr Tyr Cys Phe Arg Val
            180                 185                 190

gtt ttt gta cat tct ctg ccc tgt gtt tcg ctg gtg gtg ctc aat gta       624
Val Phe Val His Ser Leu Pro Cys Val Ser Leu Val Val Leu Asn Val
        195                 200                 205

ttg ttg ttt cgt gcc atg aga gcc gcg caa ctt aaa aga aag gaa ttg       672
Leu Leu Phe Arg Ala Met Arg Ala Ala Gln Leu Lys Arg Lys Glu Leu
    210                 215                 220

ttt aaa gaa aat cgc aag tgc gaa agt aaa aaa ata cgg gat tcg aac 720
Phe Lys Glu Asn Arg Lys Cys Glu Ser Lys Lys Ile Arg Asp Ser Asn
225                 230                 235                 240

tgc acc act ttg atg ctc att gtc gtc gtt acc gtc ttc cta gct gta 768
Cys Thr Thr Leu Met Leu Ile Val Val Val Thr Val Phe Leu Ala Val
                245                 250                 255

gaa ata cca tta gcc gtt ata acc gtt ttg cat atc ata tcg agt ggt 816
Glu Ile Pro Leu Ala Val Ile Thr Val Leu His Ile Ile Ser Ser Gly
            260                 265                 270

ata acc gaa ata tta gat tac aat ata gca aac gta ttg att tta ttt 864
Ile Thr Glu Ile Leu Asp Tyr Asn Ile Ala Asn Val Leu Ile Leu Phe
        275                 280                 285

tca aac ttt ttt atc att gtt agt tat cca att aat ttt gcc att tat 912
Ser Asn Phe Phe Ile Ile Val Ser Tyr Pro Ile Asn Phe Ala Ile Tyr
    290                 295                 300

tgt gga atg tca cga caa ttt agg gaa act ttt aaa gag ctt ttc ata 960
Cys Gly Met Ser Arg Gln Phe Arg Glu Thr Phe Lys Glu Leu Phe Ile
305                 310                 315                 320

cgt gga gcg ata acg acg aga aga aac gat gga agt tca aga tat tca 1008
Arg Gly Ala Ile Thr Thr Arg Arg Asn Asp Gly Ser Ser Arg Tyr Ser
                325                 330                 335

ttg gtg aat gga cct cgc acg tgc acc aac gaa act gtc cta 1050
Leu Val Asn Gly Pro Arg Thr Cys Thr Asn Glu Thr Val Leu
            340                 345                 350

<210> 12
<211> 350
<212> PRT
<213> Pediculus humanus corporis

<400> 12

Phe Ala Gln Pro Leu Tyr Gly Tyr Ile Val Pro Phe Leu Leu Val Ile
1               5                   10                  15

Thr Ile Ile Ala Asn Thr Leu Ile Val Val Val Leu Ser Lys Arg His
            20                  25                  30

Met Arg Thr Pro Thr Asn Phe Val Leu Met Val Met Ala Leu Ser Asp
        35                  40                  45

Met Phe Thr Leu Leu Phe Pro Ala Pro Trp Leu Phe Tyr Met Tyr Thr
    50                  55                  60

Phe Gly Asn His Tyr Lys Pro Leu Trp Pro Val Ala Ala Cys Tyr Leu
65                  70                  75                  80

Trp Asn Ala Met Lys Glu Asp Ile Pro Ala Leu Phe His Thr Ala Ser
                85                  90                  95

Ile Trp Leu Thr Leu Ala Leu Ala Val Gln Arg Tyr Ile Tyr Val Cys
            100                 105                 110

His Ala Pro Val Ala Arg Thr Trp Cys Thr Met Pro Arg Val Ile Lys
        115                 120                 125

Cys Val Ala Trp Ile Tyr Val Phe Ala Thr Leu His Gln Ser Ser Arg
    130                 135                 140

Phe Phe Asp Arg Thr Tyr Thr Ser Ile Asn Ile Thr Trp Asn Gly Val
145                 150                 155                 160

Pro Asp Val Ala Val Cys Lys Val Asn Leu Ala Thr Trp Val Thr Asp
                165                 170                 175

Ile Val Thr Glu Asp Val Tyr Phe Thr Val Tyr Tyr Cys Phe Arg Val
            180                 185                 190

Val Phe Val His Ser Leu Pro Cys Val Ser Leu Val Val Leu Asn Val
        195                 200                 205

Leu Leu Phe Arg Ala Met Arg Ala Ala Gln Leu Lys Arg Lys Glu Leu
    210                 215                 220

Phe Lys Glu Asn Arg Lys Cys Glu Ser Lys Lys Ile Arg Asp Ser Asn
225                 230                 235                 240

Cys Thr Thr Leu Met Leu Ile Val Val Val Thr Val Phe Leu Ala Val
                245                 250                 255

Glu Ile Pro Leu Ala Val Ile Thr Val Leu His Ile Ile Ser Ser Gly
            260                 265                 270

Ile Thr Glu Ile Leu Asp Tyr Asn Ile Ala Asn Val Leu Ile Leu Phe
        275                 280                 285

Ser Asn Phe Phe Ile Ile Val Ser Tyr Pro Ile Asn Phe Ala Ile Tyr
    290                 295                 300

Cys Gly Met Ser Arg Gln Phe Arg Glu Thr Phe Lys Glu Leu Phe Ile
305                 310                 315                 320

Arg Gly Ala Ile Thr Thr Arg Arg Asn Asp Gly Ser Ser Arg Tyr Ser
                325                 330                 335

Leu Val Asn Gly Pro Arg Thr Cys Thr Asn Glu Thr Val Leu
            340                 345                 350

<210>  13
<211>  1125
<212>  DNA
<213>  Culex pipiens

<220>
<221>  CDS
<222>  (1)..(1125)
<223>  Partial sequence

<400>  13
tgc atg gga atg gaa gga aat gtt tcc tac ttg aac gtg tcg tgt gag        48
Cys Met Gly Met Glu Gly Asn Val Ser Tyr Leu Asn Val Ser Cys Glu
1               5                   10                  15

act att ctc aac tac agc atc cca ctg tac ggc tac tgc aca ccg atc 96
Thr Ile Leu Asn Tyr Ser Ile Pro Leu Tyr Gly Tyr Cys Thr Pro Ile
            20                  25                  30

ttc atc cta att aca ctg act gcc aac tct tta atc gtg att gtc ctg 144
Phe Ile Leu Ile Thr Leu Thr Ala Asn Ser Leu Ile Val Ile Val Leu
        35                  40                  45

agc aaa cgc agc atg gcc tct cct acc aac ttt gtt ctg atg gcg atg 192
Ser Lys Arg Ser Met Ala Ser Pro Thr Asn Phe Val Leu Met Ala Met
    50                  55                  60

gca ctt tgc gac atg ttt act ctg tta ttt ccg gca ccc ggc cta ctg 240
Ala Leu Cys Asp Met Phe Thr Leu Leu Phe Pro Ala Pro Gly Leu Leu
65                  70                  75                  80

tac atg tac acg ttc ggc aat cat tac aag cca tta tcg ccc atc gcc 288
Tyr Met Tyr Thr Phe Gly Asn His Tyr Lys Pro Leu Ser Pro Ile Ala
                85                  90                  95

gcc tgc tat gtg tgg aat gct ctg aat gag atc ctt ccg gca atg ttc 336
Ala Cys Tyr Val Trp Asn Ala Leu Asn Glu Ile Leu Pro Ala Met Phe
            100                 105                 110

cac acg gca tcg gtt tgg ctg acg ttg gcg ctt gcg gtg cag agg tac 384
His Thr Ala Ser Val Trp Leu Thr Leu Ala Leu Ala Val Gln Arg Tyr
        115                 120                 125

gtg tac gtc tgt cat gcc cca atc gcc cgg acg tgg tgc aca atc ccg 432
Val Tyr Val Cys His Ala Pro Ile Ala Arg Thr Trp Cys Thr Ile Pro
    130                 135                 140

agg gtg aaa aag tgt atc gcc tac atc tgt cta gct gca tta att cat 480
Arg Val Lys Lys Cys Ile Ala Tyr Ile Cys Leu Ala Ala Leu Ile His
145                 150                 155                 160

cag agc acc aga ttt ttc gat aaa tcc tac agc ttg gtg cag atc gaa 528
Gln Ser Thr Arg Phe Phe Asp Lys Ser Tyr Ser Leu Val Gln Ile Glu
                165                 170                 175

tgg aac ggg cag ctt acc gaa gtg tgt cac acg gac acc gcc ggc tgg 576
Trp Asn Gly Gln Leu Thr Glu Val Cys His Thr Asp Thr Ala Gly Trp
            180                 185                 190

gtg cac gat tac gtc ggc gag gac ttt tac tac acg ttt tat ttt tcg 624
Val His Asp Tyr Val Gly Glu Asp Phe Tyr Tyr Thr Phe Tyr Phe Ser
        195                 200                 205

ttc cga ata ctg ttc gtg cac ctg atc ccg tgc ggc agc ctg gtg gcg 672
Phe Arg Ile Leu Phe Val His Leu Ile Pro Cys Gly Ser Leu Val Ala
    210                 215                 220

ttg aac gtt ctg ctg ttc aaa gcg atg cga caa gcc cag caa aag cgc 720
Leu Asn Val Leu Leu Phe Lys Ala Met Arg Gln Ala Gln Gln Lys Arg
225                 230                 235                 240

gaa agg ctc ttc aag gac act gcc aaa aag cgc gaa tgt aaa cgg cag 768
Glu Arg Leu Phe Lys Asp Thr Ala Lys Lys Arg Glu Cys Lys Arg Gln
                245                 250                 255

agg gac tcg aac tgt acc acg ctg atg ctc atc gtc gtt gta act gtg 816
Arg Asp Ser Asn Cys Thr Thr Leu Met Leu Ile Val Val Val Thr Val
            260                 265                 270

ttc cta atc gtt gaa att cca ctc ggt gtg ata acg gcc tta cac ata 864
Phe Leu Ile Val Glu Ile Pro Leu Gly Val Ile Thr Ala Leu His Ile
        275                 280                 285

att tcc tcg ctc atc tac gag ttt tta gac tac tac att gca aac ttg 912
Ile Ser Ser Leu Ile Tyr Glu Phe Leu Asp Tyr Tyr Ile Ala Asn Leu
    290                 295                 300

ttt att ctt ttt gca aac ttt ttc ctc atc gtc agt tac cct atc aat 960
Phe Ile Leu Phe Ala Asn Phe Phe Leu Ile Val Ser Tyr Pro Ile Asn
305                 310                 315                 320

ttc gcc att tac tgc ggg atg tcg cgc cag ttt cgg gag acc ttc aaa 1008
Phe Ala Ile Tyr Cys Gly Met Ser Arg Gln Phe Arg Glu Thr Phe Lys
                325                 330                 335

gaa atc ttt gtc aaa tct agc aaa caa atc cca ggt ggc agc agc aag 1056
Glu Ile Phe Val Lys Ser Ser Lys Gln Ile Pro Gly Gly Ser Ser Lys
            340                 345                 350

aaa gac tgc gga tca tcg cga tac tcc ctg gtc aac gga cct cgg aca 1104
Lys Asp Cys Gly Ser Ser Arg Tyr Ser Leu Val Asn Gly Pro Arg Thr
        355                 360                 365

ttc acc aac gag acg gta cta 1125
Phe Thr Asn Glu Thr Val Leu
    370                 375

<210> 14
<211> 375
<212> PRT
<213> Culex pipiens

<400> 14

Cys Met Gly Met Glu Gly Asn Val Ser Tyr Leu Asn Val Ser Cys Glu
1               5                   10                  15

Thr Ile Leu Asn Tyr Ser Ile Pro Leu Tyr Gly Tyr Cys Thr Pro Ile
            20                  25                  30

Phe Ile Leu Ile Thr Leu Thr Ala Asn Ser Leu Ile Val Ile Val Leu
        35                  40                  45

Ser Lys Arg Ser Met Ala Ser Pro Thr Asn Phe Val Leu Met Ala Met
    50                  55                  60

Ala Leu Cys Asp Met Phe Thr Leu Leu Phe Pro Ala Pro Gly Leu Leu
65                  70                  75                  80

Tyr Met Tyr Thr Phe Gly Asn His Tyr Lys Pro Leu Ser Pro Ile Ala
                85                  90                  95

Ala Cys Tyr Val Trp Asn Ala Leu Asn Glu Ile Leu Pro Ala Met Phe
            100                 105                 110

His Thr Ala Ser Val Trp Leu Thr Leu Ala Leu Ala Val Gln Arg Tyr
        115                 120                 125

Val Tyr Val Cys His Ala Pro Ile Ala Arg Thr Trp Cys Thr Ile Pro
    130                 135                 140

Arg Val Lys Lys Cys Ile Ala Tyr Ile Cys Leu Ala Ala Leu Ile His
145                 150                 155                 160

Gln Ser Thr Arg Phe Phe Asp Lys Ser Tyr Ser Leu Val Gln Ile Glu
                165                 170                 175

Trp Asn Gly Gln Leu Thr Glu Val Cys His Thr Asp Thr Ala Gly Trp
            180                 185                 190

Val His Asp Tyr Val Gly Glu Asp Phe Tyr Tyr Thr Phe Tyr Phe Ser
        195                 200                 205

Phe Arg Ile Leu Phe Val His Leu Ile Pro Cys Gly Ser Leu Val Ala
    210                 215                 220

Leu Asn Val Leu Leu Phe Lys Ala Met Arg Gln Ala Gln Gln Lys Arg
225                 230                 235                 240

Glu Arg Leu Phe Lys Asp Thr Ala Lys Lys Arg Glu Cys Lys Arg Gln
                245                 250                 255

Arg Asp Ser Asn Cys Thr Thr Leu Met Leu Ile Val Val Val Thr Val
            260                 265                 270

Phe Leu Ile Val Glu Ile Pro Leu Gly Val Ile Thr Ala Leu His Ile
        275                 280                 285

Ile Ser Ser Leu Ile Tyr Glu Phe Leu Asp Tyr Tyr Ile Ala Asn Leu
    290                 295                 300

Phe Ile Leu Phe Ala Asn Phe Phe Leu Ile Val Ser Tyr Pro Ile Asn
305                 310                 315                 320

Phe Ala Ile Tyr Cys Gly Met Ser Arg Gln Phe Arg Glu Thr Phe Lys
                325                 330                 335

Glu Ile Phe Val Lys Ser Ser Lys Gln Ile Pro Gly Gly Ser Ser Lys
            340                 345                 350

Lys Asp Cys Gly Ser Ser Arg Tyr Ser Leu Val Asn Gly Pro Arg Thr
        355                 360                 365

Phe Thr Asn Glu Thr Val Leu
    370                 375

<210>  15
<211>  1515
<212>  DNA
<213>  Drosophila pseudoobscura

<220>
<221>  CDS
<222>  (1)..(1515)

<400>  15
atg ggc ggc gat caa ggg gtg cta att gaa ttg cag gaa atg tca acg      48
Met Gly Gly Asp Gln Gly Val Leu Ile Glu Leu Gln Glu Met Ser Thr
1               5                   10                  15

gca gtc acg aaa acc gtc gcg gcc gcc gct gcg gcc atg ctg ggg tcc      96
Ala Val Thr Lys Thr Val Ala Ala Ala Ala Ala Ala Met Leu Gly Ser
            20                  25                  30

agc atg gac aat aac tct gcc tat tcg tac gat ata tcc gca acg gac 144
Ser Met Asp Asn Asn Ser Ala Tyr Ser Tyr Asp Ile Ser Ala Thr Asp
        35                  40                  45

gtg ctc tat cag tgg agt gcc aat gcg gcg gca gcc gcc gcc ggc ggg 192
Val Leu Tyr Gln Trp Ser Ala Asn Ala Ala Ala Ala Ala Ala Gly Gly
    50                  55                  60

gca cgt atc gcc cac acc atg ccg ccc tca gtg gcg acc ctg cct cca 240
Ala Arg Ile Ala His Thr Met Pro Pro Ser Val Ala Thr Leu Pro Pro
65                  70                  75                  80

gcc acg aat cac ggc tcc atg cat cag tcc gtg ccg ctt ccc gga atc 288
Ala Thr Asn His Gly Ser Met His Gln Ser Val Pro Leu Pro Gly Ile
                85                  90                  95

gga caa cgc ctc gct ccc aac gag acg cag ttc gga ctg ctc gac tac 336
Gly Gln Arg Leu Ala Pro Asn Glu Thr Gln Phe Gly Leu Leu Asp Tyr
            100                 105                 110

ggg atg agc agc atg aac gac agc ctg gac tac gcc tcc tac cag cag 384
Gly Met Ser Ser Met Asn Asp Ser Leu Asp Tyr Ala Ser Tyr Gln Gln
        115                 120                 125

cag atg ggc gag gac ggg cag tgc cgg cag atg gac gcc aac gtg agc 432
Gln Met Gly Glu Asp Gly Gln Cys Arg Gln Met Asp Ala Asn Val Ser
    130                 135                 140

tac tgg aac ctc acc tgc gac tcg ccc ctg gac tac gcc ctg ccc cta 480
Tyr Trp Asn Leu Thr Cys Asp Ser Pro Leu Asp Tyr Ala Leu Pro Leu
145                 150                 155                 160

tac ggc tac tgc atg ccg ttc ctg ctg ata atc acc atc ata tcg aac 528
Tyr Gly Tyr Cys Met Pro Phe Leu Leu Ile Ile Thr Ile Ile Ser Asn
                165                 170                 175

acc ctg atc gtc cta gtc ctc agc aag aag agc atg gcc acg ccg aca 576
Thr Leu Ile Val Leu Val Leu Ser Lys Lys Ser Met Ala Thr Pro Thr
            180                 185                 190

aac ttt gtg ctc atg ggc atg gct ata tgc gat atg ctg acg gtt ata 624
Asn Phe Val Leu Met Gly Met Ala Ile Cys Asp Met Leu Thr Val Ile
        195                 200                 205

ttc ccg gca ccg gga ctc tgg tat atg tac aca ttt ggc aat cat tat 672
Phe Pro Ala Pro Gly Leu Trp Tyr Met Tyr Thr Phe Gly Asn His Tyr
    210                 215                 220

aag cct ctg cat ccg gtc tcc atg tgt ctg gcc tac agc att ttc aac 720
Lys Pro Leu His Pro Val Ser Met Cys Leu Ala Tyr Ser Ile Phe Asn
225                 230                 235                 240

gag att atg cca gcc atg tgc cac acc atc tcc gtg tgg cta acc ctg 768
Glu Ile Met Pro Ala Met Cys His Thr Ile Ser Val Trp Leu Thr Leu
                245                 250                 255

gcc ctg gcc gtg caa aga tat atc tac gtg tgc cac gcc ccc atg gcc 816
Ala Leu Ala Val Gln Arg Tyr Ile Tyr Val Cys His Ala Pro Met Ala
            260                 265                 270

cgc acc tgg tgc act atg ccg cgg gtg aag cgc tgc acg gtg tac atc 864
Arg Thr Trp Cys Thr Met Pro Arg Val Lys Arg Cys Thr Val Tyr Ile
        275                 280                 285

gca ctg ctg gcg ttc ctg cac cag ctg cca cga ttc ttc gac cgc acc 912
Ala Leu Leu Ala Phe Leu His Gln Leu Pro Arg Phe Phe Asp Arg Thr
    290                 295                 300

ttt ctg ccc ctt cag atc gag tgg aac gga aac gag acg gag gtg tgt 960
Phe Leu Pro Leu Gln Ile Glu Trp Asn Gly Asn Glu Thr Glu Val Cys
305                 310                 315                 320

cac ctg gag acg tcg atg tgg gtg cac gag tac atc ggc atg gac ctg 1008
His Leu Glu Thr Ser Met Trp Val His Glu Tyr Ile Gly Met Asp Leu
                325                 330                 335

tac tac acg agc tac tac ctg ttc cgg gtg ctg ttc gtg cac ctg ctg 1056
Tyr Tyr Thr Ser Tyr Tyr Leu Phe Arg Val Leu Phe Val His Leu Leu
            340                 345                 350

ccg tgc atc atc ctg gtg acg ctc aac atc ctg ctg ttc gcg gcg atg 1104
Pro Cys Ile Ile Leu Val Thr Leu Asn Ile Leu Leu Phe Ala Ala Met
        355                 360                 365

cgg cag gcc cag gag cgg cgg aag ctg ctg ttc cgc gag aac cgc aag 1152
Arg Gln Ala Gln Glu Arg Arg Lys Leu Leu Phe Arg Glu Asn Arg Lys
    370                 375                 380

aag gag tgc aag aag ctg cgc gag tcc aac tgc acc acg ctg atg ctg 1200
Lys Glu Cys Lys Lys Leu Arg Glu Ser Asn Cys Thr Thr Leu Met Leu
385                 390                 395                 400

atc gtg gtg gtg acc gtg ttc ctc atc gcc gag ata ccc att gcc gtg 1248
Ile Val Val Val Thr Val Phe Leu Ile Ala Glu Ile Pro Ile Ala Val
                405                 410                 415

gtc acg gcc atg cac atc gtg agc tcg ctg atc atc gag ttc ctc gac 1296
Val Thr Ala Met His Ile Val Ser Ser Leu Ile Ile Glu Phe Leu Asp
            420                 425                 430

tac ggc atc gcc aac atc ttc atc atg ctg acc aac ttc ttc ctg gtg 1344
Tyr Gly Ile Ala Asn Ile Phe Ile Met Leu Thr Asn Phe Phe Leu Val
        435                 440                 445

gtg agc tac ccg atc aac ttt ggc atc tac tgc ggc atg tcg cgc cag 1392
Val Ser Tyr Pro Ile Asn Phe Gly Ile Tyr Cys Gly Met Ser Arg Gln
    450                 455                 460

ttc cgc gag acc ttc aag gag ata ttc ctc ggc cgc atc att gcc aag 1440
Phe Arg Glu Thr Phe Lys Glu Ile Phe Leu Gly Arg Ile Ile Ala Lys
465                 470                 475                 480

aag gac agc tcc tcc aag tac tcg atc gtc aat gga ccc cgt acc tgc 1488
Lys Asp Ser Ser Ser Lys Tyr Ser Ile Val Asn Gly Pro Arg Thr Cys
                485                 490                 495

acc aat acc aac gag acg gtc cta tag 1515
Thr Asn Thr Asn Glu Thr Val Leu
            500

<210> 16
<211> 504
<212> PRT
<213> Drosophila pseudoobscura

<400> 16

Met Gly Gly Asp Gln Gly Val Leu Ile Glu Leu Gln Glu Met Ser Thr
1               5                   10                  15

Ala Val Thr Lys Thr Val Ala Ala Ala Ala Ala Ala Met Leu Gly Ser
            20                  25                  30

Ser Met Asp Asn Asn Ser Ala Tyr Ser Tyr Asp Ile Ser Ala Thr Asp
        35                  40                  45

Val Leu Tyr Gln Trp Ser Ala Asn Ala Ala Ala Ala Ala Ala Gly Gly
    50                  55                  60

Ala Arg Ile Ala His Thr Met Pro Pro Ser Val Ala Thr Leu Pro Pro
65                  70                  75                  80

Ala Thr Asn His Gly Ser Met His Gln Ser Val Pro Leu Pro Gly Ile
                85                  90                  95

Gly Gln Arg Leu Ala Pro Asn Glu Thr Gln Phe Gly Leu Leu Asp Tyr
            100                 105                 110

Gly Met Ser Ser Met Asn Asp Ser Leu Asp Tyr Ala Ser Tyr Gln Gln
        115                 120                 125

Gln Met Gly Glu Asp Gly Gln Cys Arg Gln Met Asp Ala Asn Val Ser
    130                 135                 140

Tyr Trp Asn Leu Thr Cys Asp Ser Pro Leu Asp Tyr Ala Leu Pro Leu
145                 150                 155                 160

Tyr Gly Tyr Cys Met Pro Phe Leu Leu Ile Ile Thr Ile Ile Ser Asn
                165                 170                 175

Thr Leu Ile Val Leu Val Leu Ser Lys Lys Ser Met Ala Thr Pro Thr
            180                 185                 190

Asn Phe Val Leu Met Gly Met Ala Ile Cys Asp Met Leu Thr Val Ile
        195                 200                 205

Phe Pro Ala Pro Gly Leu Trp Tyr Met Tyr Thr Phe Gly Asn His Tyr
    210                 215                 220

Lys Pro Leu His Pro Val Ser Met Cys Leu Ala Tyr Ser Ile Phe Asn
225                 230                 235                 240

Glu Ile Met Pro Ala Met Cys His Thr Ile Ser Val Trp Leu Thr Leu
                245                 250                 255

Ala Leu Ala Val Gln Arg Tyr Ile Tyr Val Cys His Ala Pro Met Ala
            260                 265                 270

Arg Thr Trp Cys Thr Met Pro Arg Val Lys Arg Cys Thr Val Tyr Ile
        275                 280                 285

Ala Leu Leu Ala Phe Leu His Gln Leu Pro Arg Phe Phe Asp Arg Thr
    290                 295                 300

Phe Leu Pro Leu Gln Ile Glu Trp Asn Gly Asn Glu Thr Glu Val Cys
305                 310                 315                 320

His Leu Glu Thr Ser Met Trp Val His Glu Tyr Ile Gly Met Asp Leu
                325                 330                 335

Tyr Tyr Thr Ser Tyr Tyr Leu Phe Arg Val Leu Phe Val His Leu Leu
            340                 345                 350

Pro Cys Ile Ile Leu Val Thr Leu Asn Ile Leu Leu Phe Ala Ala Met
        355                 360                 365

Arg Gln Ala Gln Glu Arg Arg Lys Leu Leu Phe Arg Glu Asn Arg Lys
    370                 375                 380

Lys Glu Cys Lys Lys Leu Arg Glu Ser Asn Cys Thr Thr Leu Met Leu
385                 390                 395                 400

Ile Val Val Val Thr Val Phe Leu Ile Ala Glu Ile Pro Ile Ala Val
                405                 410                 415

Val Thr Ala Met His Ile Val Ser Ser Leu Ile Ile Glu Phe Leu Asp
            420                 425                 430

Tyr Gly Ile Ala Asn Ile Phe Ile Met Leu Thr Asn Phe Phe Leu Val
        435                 440                 445

Val Ser Tyr Pro Ile Asn Phe Gly Ile Tyr Cys Gly Met Ser Arg Gln
    450                 455                 460

Phe Arg Glu Thr Phe Lys Glu Ile Phe Leu Gly Arg Ile Ile Ala Lys
465                 470                 475                 480

Lys Asp Ser Ser Ser Lys Tyr Ser Ile Val Asn Gly Pro Arg Thr Cys
                485                 490                 495

Thr Asn Thr Asn Glu Thr Val Leu
            500

<210>  17
<211>  177
<212>  DNA
<213>  Drosophila melanogaster

<220>
<221>  CDS
<222>  (1)..(177)

<400>  17
atg aaa act cta gcc cta ttc ttg gtt ctc gtt tgc gta ctc ggc ttg        48
Met Lys Thr Leu Ala Leu Phe Leu Val Leu Val Cys Val Leu Gly Leu
1               5                   10                  15

gtc cag tcc tgg gaa tgg ccg tgg aat agg aag cct aca aag ttt cca        96
Val Gln Ser Trp Glu Trp Pro Trp Asn Arg Lys Pro Thr Lys Phe Pro
            20                  25                  30

att cca agc ccc aat cct cct ttc gta ggt gat aag tgg tgc cgt ctt       144
Ile Pro Ser Pro Asn Pro Pro Phe Val Gly Asp Lys Trp Cys Arg Leu
        35                  40                  45

aat ttg ggg ccc gcc tgg ggt gga aga tgt taa                           177
Asn Leu Gly Pro Ala Trp Gly Gly Arg Cys
    50                  55

<210>  18
<211>  58
<212>  PRT
<213>  Drosophila melanogaster

<400>  18

Met Lys Thr Leu Ala Leu Phe Leu Val Leu Val Cys Val Leu Gly Leu
1               5                   10                  15

Val Gln Ser Trp Glu Trp Pro Trp Asn Arg Lys Pro Thr Lys Phe Pro
            20                  25                  30

Ile Pro Ser Pro Asn Pro Pro Phe Val Gly Asp Lys Trp Cys Arg Leu
        35                  40                  45

Asn Leu Gly Pro Ala Trp Gly Gly Arg Cys
    50                  55

<210>  19
<211>  9
<212>  PRT
<213>  Drosophila melanogaster

<400>  19

Asp Pro Lys Gln Asp Phe Met Arg Phe
1               5

<210>  20
<211>  9
<212>  PRT
<213>  Drosophila melanogaster

<400>  20

Thr Pro Ala Glu Asp Phe Met Arg Phe
1               5

<210>  21
<211>  7
<212>  PRT
<213>  Drosophila melanogaster

<400>  21

Asn Gln Lys Thr Met Ser Phe
1               5

<210>  22
<211>  9
<212>  PRT
<213>  Drosophila melanogaster

<400>  22

Phe Asp Asp Tyr Gly His Met Arg Phe
1               5

<210>  23
<211>  14
<212>  PRT
<213>  Drosophila melanogaster

<400>  23

Gly Gly Asp Asp Gln Phe Asp Asp Tyr Gly His Met Arg Phe
1               5                   10

<210>  24
<211>  10
<212>  PRT
<213>  Drosophila melanogaster

<400>  24

Thr Asp Val Asp His Val Phe Leu Arg Phe
1               5                   10


<210>  25
<211>  17
<212>  PRT
<213>  Drosophila melanogaster

<400>  25

Gln Leu Gln Ser Asn Gly Glu Pro Ala Tyr Arg Val Arg Thr Pro Arg
1               5                   10                  15


Leu



<210>  26
<211>  8
<212>  PRT
<213>  Drosophila melanogaster

<400>  26

Ser Val Pro Phe Lys Pro Arg Leu
1               5


<210>  27
<211>  36
<212>  PRT
<213>  Artificial

<220>
<223>  Synthetic sex peptide

<400>  27

Trp Glu Trp Pro Trp Asn Arg Lys Pro Thr Lys Phe Pro Ile Pro Ser
1               5                   10                  15


Pro Asn Pro Arg Asp Lys Trp Cys Arg Leu Asn Leu Gly Pro Ala Trp
            20                  25                  30


Gly Gly Arg Cys
        35


<210>  28
<211>  31
<212>  PRT
<213>  Artificial

<220>
<223>  Synthetic DUP99P

<400>  28

Gln Asp Arg Asn Asp Thr Glu Trp Ile Gln Ser Gln Lys Asp Arg Glu

1               5                   10                  15

Lys Trp Cys Arg Leu Asn Leu Gly Pro Tyr Leu Gly Gly Arg Cys
            20                  25                  30

<210>  29
<211>  22
<212>  PRT
<213>  Artificial

<220>
<223>  N-terminal sex peptide receptor peptide

<400>  29

Pro Thr Asn Glu Ser Gln Leu Glu Ile Pro Asp Tyr Gly Asn Glu Ser
1               5                   10                  15

Leu Asp Tyr Pro Asn Cys
            20

<210>  30
<211>  20
<212>  DNA
<213>  Artificial

<220>
<223>  Primer forward

<400>  30
atgggcggcg atcaaggggt                                                   20

<210>  31
<211>  20
<212>  DNA
<213>  Artificial

<220>
<223>  Primer reverse

<400>  31
ggcaccaaca tcaccaatta                                                   20

<210>  32
<211>  21
<212>  DNA
<213>  Artificial

<220>
<223>  Primer forward

<400>  32
atgtcaattg atgctgcggt a                                                 21

<210>  33
<211>  20
<212>  DNA
<213>  Artificial

<220>
<223>  Primer reverse

<400>  33
cgttggttct gtgtgacaaa                                                   20

<210>  34
<211>  24
<212>  DNA
<213>  Artificial

<220>
<223>  Primer forward

<400>  34
atgattgaaa aaaataattt caag                                              24


<210>  35
<211>  19
<212>  DNA
<213>  Artificial

<220>
<223>  Primer reverse

<400>  35
cctgctatct aaccacagt                                                    19


<210>  36
<211>  20
<212>  DNA
<213>  Artificial

<220>
<223>  Primer forward

<400>  36
atggcggtca ccatagacaa                                                   20


<210>  37
<211>  20
<212>  DNA
<213>  Artificial

<220>
<223>  Primer reverse

<400>  37
ggcttaaagc acagtttcgt                                                   20


<210>  38
<211>  21
<212>  DNA
<213>  Artificial

<220>
<223>  Primer forward

<400>  38
atgggcgaga tggcgtcgaa c                                                 21


<210>  39
<211>  21
<212>  DNA
<213>  Artificial

<220>
<223>  Primer reverse

<400>  39
tcaacattga gtttgtccta a                                                 21